# FASCICULE DE BREVET EUROPEEN

(11) **EP 1 383 732 B1**
(45) Date de publication et mention de la délivrance du brevet: **17.02.2010**
(21) Numéro de dépôt: 02740788.1
(22) Date de dépôt: 03.05.2002
(51) Int. Cl.: C07C 245/14, C07C 245/18, C07D 209/14, C07D 495/04, C07D 311/82, C12Q 1/68

(54) **REACTIFS DE MARQUAGE, PROCEDES DE SYNTHESE DE TELS REACTIFS ET PROCEDES DE DETECTION DE MOLECULES BIOLOGIQUES**
MARKIERUNGSREAGENTIEN, VERFAHREN ZUR SYNTHESE DIESER REAGENZIEN UND VERFAHREN ZUM NACHWEIS BIOLOGISCHER MOLEKÜLE
LABELLING REAGENTS, METHOD FOR SYNTHESIS OF SAID REAGENTS AND METHODS FOR DETECTING BIOLOGICAL MOLECULES

(30) Priorité: 04.05.2001 FR 0106040
(43) Date de publication de la demande: 28.01.2004
(73) Titulaire: BIO MERIEUX, 69280 Marcy l'Etoile (FR); UNIVERSITE JOSEPH FOURIER (GRENOBLE 1), F-38041 Grenoble Cédex (FR); CENTRE NATIONAL DE LA RECHERCHE SCIENTIFIQUE (CNRS), 75794 Paris Cedex 16 (FR)
(72) Inventeur: BOURGET, Cécile, F-74940 Annecy le Vieux (FR); LHOMME, Jean, F-38240 Meylan (FR); LAAYOUN, Ali, F-69780 Toussieu (FR); KOTERA, Mitsuharu, F-38240 Meylan (FR); TREVISIOL, Emmanuelle, F-81500 Montcarbier (FR); MENOU, Lionel, F-69230 Saint Genis Laval (FR); BERNAL MENDEZ, Eloy, F-69003 Lyon (FR)
(74) Mandataire: Guerre, Dominique
(86) Numéro de dépôt international: PCT/FR2002/001543
(87) Numéro de publication internationale: WO 2002/090319

(56) Documents cités:
- WO-A-90/08838
- WO-A-99/65926
- MASANOBU SHIGA ET AL.: "Fluorescence Detection of DNA Using A Novel Peroxidase Substrate, 4-(4-Hydroxyphenylcarbamoyl)butanoic Acid" ANALYTICAL SCIENCES, vol. 11, no. 4, 1995, pages 591-595, XP008005960 Tokyo, Japan
- MASANOBU SHIGA ET AL.: "Synthesis of a Novel Biotin Derivative That Bears a Diazo Group as the Reactive Site" ANALYTICAL SCIENCES, vol. 9, no. 4, 1993, pages 553-556, XP008005959 Tokyo, Japan
- MAKRIGIORGOS G M ET AL: "FLUORESCENT LABELLING OF ABASIC SITES: A NOVEL METHODOLOGY TO DETECT CLOSELY-SPACED DAMAGE SITES IN DNA" INTERNATIONAL JOURNAL OF RADIATION BIOLOGY, TAYLOR AND FRANCIS, LONDON, GB, vol. 74, no. 1, 1998, pages 99-109, XP002925462 ISSN: 0955-3002

## Description

La présente invention concerne de nouveaux réactifs de marquage de molécules biologiques, un procédé de synthèse desdits marqueurs ainsi que des applications pour le marquage de molécules biologiques en particulier dans le domaine du diagnostic par sondes nucléiques.

L'état de la technique montre que de nombreuses méthodes existent pour marquer des nucléotides, des oligonucléotides ou des acides nucléiques.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer un marquage d'un nucléotide, ou d'un analogue de nucléotide ou d'un acide nucléique, est enclin à effectuer cette fixation sur la base ou sur le sucre qui lui offrent plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16O94, DE-A-3.910.151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem Soc., 82, 1778-1785, 1960). De même dans la revue de O'Donnel et Mc Laughlin (« Reporter groups for the analysis of nucleic acid structure », p 216-243, dans « Bioorganic Chemistry : Nucleic Acids », Ed Hecht S.M., Oxford University Press, 1996) portant sur les méthodes d'introduction de sondes dans les fragments d'oligonucléotides, l'alkylation efficace du phosphodiester intemucléotidique est considérée comme étant impossible.

La demande de brevet WO-A-99/65926 décrit un procédé de marquage d'un acide ribonucléique (ARN) de synthèse ou naturel qui consiste à fragmenter l'ARN et à marquer au niveau du phosphate terminal. Ce document décrit un certain nombre de fonctions pouvant être utilisées pour le marquage en liaison avec la fragmentation comme les fonctions hydroxyle, amine, hydrazine, alcoxyamine, halogénure d'alkyle, halogénure d'alkyle de type benzylique et en particulier le dérivé 5-(bromométhyl)fluorescéine. Ces fonctions permettent de marquer les acides nucléiques, mais il faut associer une étape de fragmentation pour avoir un marquage efficace car ce marquage se produit sur le phosphate libéré lors de la fragmentation. De plus, il faut ajouter un excès important de réactif de marquage par rapport à l'ARN pour obtenir un marquage efficace ce qui induit des problèmes de bruit de fond générés par le marqueur en excès. Enfin, cette méthode ne fonctionne pas efficacement sur de l'ADN double brin.

Enfin, Masanobu Shiga et al. divulguent dans deux articles (« Fluorescence detection of DNA using a novel peroxidase substrate, 4-(4-hydroxyphenylcarbamoyl)butanoic acid », Analytical Siences, vol.11, no.4, 1995, pages 591-595 ; et « Synthesis of a novel biotin derivative that bears a diazo group as the reactive site », Analytical Sciences, vol.9, no.4, 1993, pages 553-556) l'utilisation de la diazobiotine comme agent de marquage d'ADN. Cependant, ce réactif de marquage présente le désavantage d'être peu stable dans le temps.

Il existe donc un besoin pour de nouveaux réactifs qui soient efficaces du point de vue du rendement de marquage, qui soient spécifiques au niveau de la position de marquage et en particulier qui n'affectent pas les propriétés d'hybridation des bases impliquées dans la formation de la double hélice, par l'intermédiaire des liaisons hydrogènes, qui soient utilisables à la fois pour l'ADN et l'ARN, et enfin qui permettent de marquer indifféremment i des nucléotides, des oligonucléotides, des acides nucléiques naturels ou préparés par amplification enzymatique.

La présente invention décrit de nouveaux marqueurs qui répondent aux conditions précitées et qui utilisent la fonction diazométhyle comme fonction réactive pour le marquage.

La fonction diazométhyle (de formule -C(N₂)-) a déjà été utilisée pour l'alkylation des groupements phosphates, mais un certain nombre de problèmes se posent. D'une part, les dérivés diazo en général sont instables eux-mêmes, ce qui pose des problèmes pour l'utilisation des ces réactifs de marquage dans un kit de marquage, et d'autre part, le produit de couplage est instable ce qui est rédhibitoire si le produit marqué a pour fonction de mettre en évidence la présence d'une molécule cible biologique dans un échantillon quelconque.

Enfin les dérivés portant la fonction diazométhyle sont insolubles dans l'eau, ce qui conduit à utiliser des conditions biphasiques pour le couplage avec des molécules biologiques, qui ne sont solubles et stables que dans l'eau ou des tampons aqueux, mais ces conditions ralentissent la vitesse de réaction et donc nuisent à l'efficacité du couplage.

Les nouveaux réactifs de marquage de l'invention résolvent aussi ces problèmes techniques.

Selon un premier mode de réalisation, la présente invention décrit un réactif de marquage stable à la température de formule (0) : 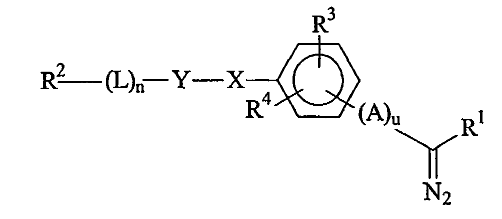 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle,
- A est un bras de liaison comportant au moins une double liaison covalente permettant la conjugaison de la fonction diazo avec le cycle aromatique et u est un nombre entier compris entre 0 et 2, préférentiellement de 0 ou 1, et
- -Y-X- représente -CONH-, -NHCO-, -CH₂O-, -CH₂S-, et
- le substituant portant la fonction diazo étant en position *ortho* ou *méta.*

Selon un deuxième mode de réalisation, la présente invention décrit un réactif de marquage stable à la température de formule (1) : 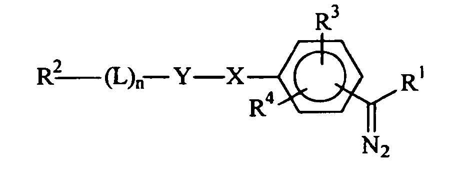 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle,
- -Y-X- représente -CONH-, -NHCO-, -CH₂O-, -CH₂S-, et
- le substituant portant la fonction diazo étant en position *ortho* ou *méta.*

Avantageusement, selon une première variante du deuxième mode de réalisation, le réactif stable à la température est de formule (2) : 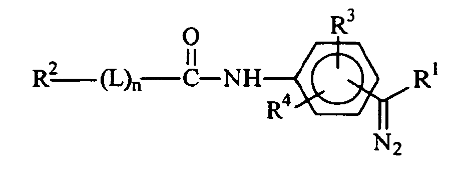 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1, et
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Avantageusement, selon une deuxième variante du deuxième mode de réalisation, le réactif stable à la température est de formule (3) : 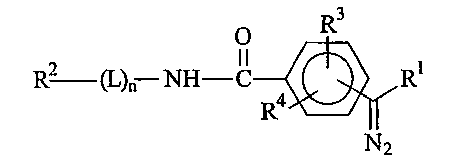 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1, et
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Avantageusement, selon une troisième variante du deuxième mode de réalisation, le réactif stable à la température est de formule (4) : 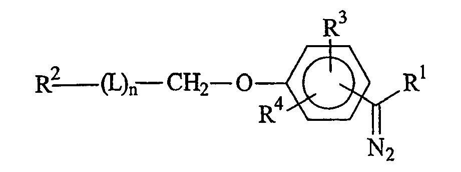 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Avantageusement, selon une première variante du premier mode de réalisation, le réactif stable à la température est de formule (21) : 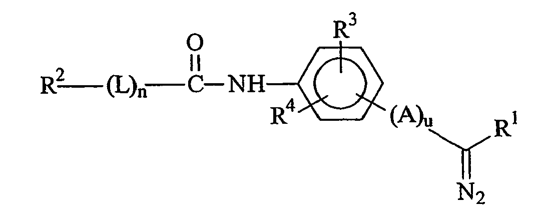 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- A est un bras de liaison comportant au moins une double liaison covalente permettant la conjugaison de la fonction diazo avec le cycle aromatique et u égal 1, et
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Avantageusement, selon une deuxième variante du premier mode de réalisation, le réactif stable à la température est de formule (22) : 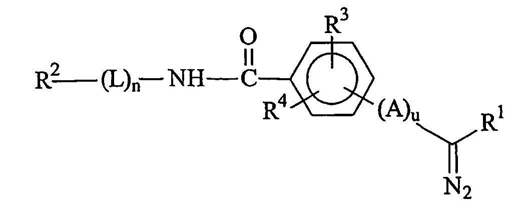 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- A est un bras de liaison comportant au moins une double liaison covalente permettant la conjugaison de la fonction diazo avec le cycle aromatique et u égal 1, et
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Avantageusement, selon une troisième variante du premier mode de réalisation, le réactif stable à la température est de formule (23) : 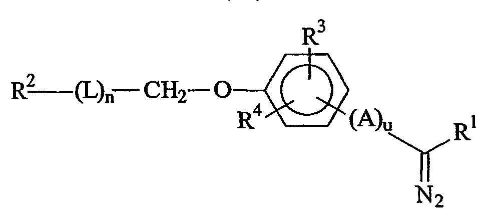 dans laquelle :
- R¹ représente H ou un groupe alkyle, aryle ou aryle substitué,
- R² représente un marqueur détectable ou au moins deux marqueurs détectables reliés entre eux par au moins une structure multimérique,
- L est un bras de liaison comportant un enchaînement linéaire d'au moins deux liaisons covalentes et n un nombre entier égal à 0 ou 1,
- A est un bras de liaison comportant au moins une double liaison covalente permettant la conjugaison de la fonction diazo avec le cycle aromatique et u égal 1, et
- R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Dans les formules ci-dessus (0) à (4) et (21) à (23), avantageusement R³ et R⁴ représentent indépendamment l'un de l'autre: H, NO₂, OCH₃, -CO-NH-(CH₂)₃-(O-CH₂-CH₂)₃-CH₂-NH-R², -CO-NH-(CH₂)₃-(O-CH₂-CH₂)₄-CH₂-NH-R².

Ainsi, un composé préféré selon la troisième variante du deuxième mode de réalisation (formule (4)) est de formule (4') : 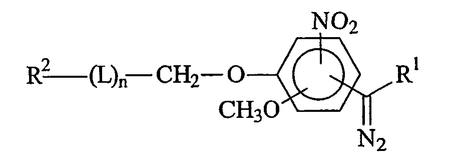 dans laquelle :
- R¹ représente H ou un groupe alkyle, aryle ou aryle substitué,
- R² représente un marqueur détectable ou au moins deux marqueurs détectables reliés entre eux par au moins une structure multimérique, et
- L est un bras de liaison comportant un enchaînement linéaire d'au moins deux liaisons covalentes et n un nombre entier égal à 0 ou 1.

De même, un composé préféré selon la première variante du deuxième mode de réalisation (formule (2)) est de formule (2') : 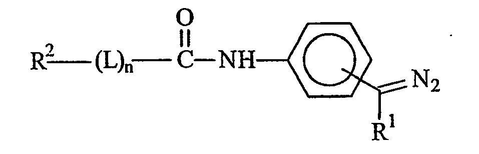 dans laquelle :
- R¹ représente H ou un groupe alkyle, aryle ou aryle substitué,
- R² représente un marqueur détectable ou au moins deux marqueurs détectables reliés entre eux par au moins une structure multimérique, et
- L est un bras de liaison comportant un enchaînement linéaire d'au moins deux liaisons covalentes et n un nombre entier égal à 0 ou 1.

Avantageusement dans la formule (2'), le substituant portant la fonction diazométhyle est en *ortho* ou en *méta*.

De même, un composé préféré selon la première variante du premier mode de réalisation (formule (21)) est de formule (24) : 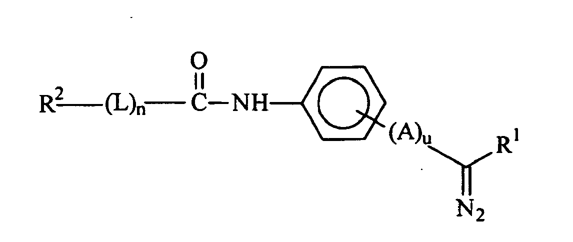 dans laquelle :
- R¹ représente H ou un groupe alkyle, aryle ou aryle substitué,
- R² représente un marqueur détectable ou au moins deux marqueurs détectables reliés entre eux par au moins une structure multimérique, et
- A est un bras de liaison comportant au moins une double liaison covalente permettant la conjugaison de la fonction diazo avec le cycle aromatique et u égal 1.

Par « structure multimérique », on entend un polymère formé d'unités répétées de synthons chimiques ou biologiques. Un exemple est cité dans l'exemple 30.2 de la description ci-après. De nombreuses variantes de telles structures utilisables dans la présente invention sont connues, comme par exemple :
- les polymères linéaires (EP-A-0.561.722, EP-A-0.669.991),
- les polymères ramifiés (WO-A-01/92361),
- les particules (EP-A-0 827 552),
- les dendrimères (US-A-4,507,466 ; US-A-4,568,737 ; US-A-6,083,708),
- les polynucléotides, et
- les polypeptides.

Par « marqueur détectable », on entend au moins un marqueur capable de générer directement ou indirectement un signal détectable. Une liste non limitative de ces marqueurs suit :
- les enzymes qui produisent un signal détectable par exemple par colorimétrie, fluorescence, luminescence, comme la péroxydase de raifort, la phosphatase alcaline, la β-galactosidase, la glucose-6-phosphate déshydrogénase,
- les chromophores comme les composés fluorescents, luminescents, colorants,
- les groupements à densité électronique détectable par microscopie électronique ou par leur propriété électrique comme la conductivité, l'ampérométrie, la voltamétrie, l'impédance,
- les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel,
- les molécules radioactives comme le ³²P, le ³⁵S ou le ¹²⁵I.

De préférence, le marqueur n'est pas un marqueur radioactif pour éviter les problèmes de sécurité liés à ces marqueurs.

Dans un mode de réalisation particulier de la présente invention le marqueur est détectable électrochimiquement, et en particulier le marqueur est un dérivé d'un complexe de fer, comme un ferrocène.

Des systèmes indirects peuvent être aussi utilisés, comme par exemple des ligands capables de réagir avec un anti-ligand. Les couples ligand/anti-ligand sont bien connus de l'homme du métier, ce qui est le cas par exemple des couples suivants : biotine/streptavidine, haptène/anticorps, antigène/anticorps, peptide/anticorps, sucre/lectine, polynucléotide/ complémentaire du polynucléotide. Dans ce cas, c'est le ligand qui porte la fonction réactive diazométhyle. L'anti-ligand peut être détectable directement par les marqueurs décrits au paragraphe précédent ou être lui-même détectable par un autre couple ligand/anti-ligand. Ce système d'empilement est illustré dans les exemples.

Un autre exemple de systèmes indirects utilise une liaison covalente spécifique entre le ligand et l'anti-ligand par exemple méthylcétone et alcoxyamine. Des exemples de ce système sont décrits dans les demandes de brevet WO-A-00/40590 et WO-A-98/05766. Ces systèmes de détection indirects peuvent conduire, dans certaines conditions, à une amplification du signal et l'on pourra se reporter aux demandes de brevet antérieures WO-A-00/07982, WO-A-01/92361 et WO-A-95/08000 pour des exemples d'amplification chimique en utilisant des polymères ou à la demande WO-A-01/44506 pour les systèmes d'amplification chimique par empilement.

Dans un mode particulier de l'amplification de signal, au moins deux marqueurs sont présents sur le réactif de marquage.

En particulier un réactif qui permet de mettre en oeuvre l'amplification de signal selon la présente invention possède une structure R²-(L)ₙ- de formule (5) ci dessous : 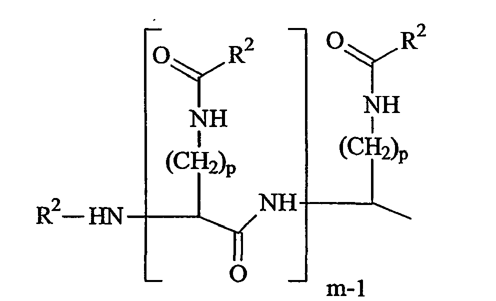 dans laquelle :
- R² représente un marqueur détectable,
- m est un nombre entier compris entre 1 et 100, de préférence compris entre 1 et 20, et
- p est un nombre entier compris entre 1 et 10, avantageusement 2 à 6, de préférence 4.

Cette structure de R²-(L)ₙ s'applique indifféremment aux formules (0) à (4) et (21) à (23) précédentes.

Un autre réactif de marquage préféré pour l'amplification de signal est le réactif de formule (6) : 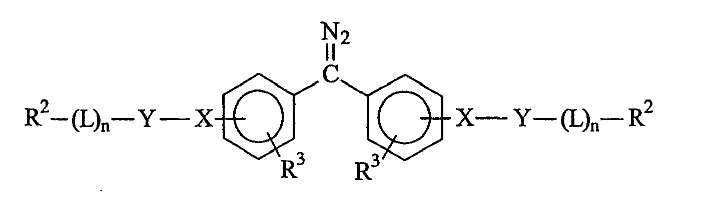 dans laquelle :
- R² représente un marqueur détectable,
- R³ représente H, NO₂, Cl, Br, F, I, OR, SR, NR₂, R, NHCOR, CONHR, COOR avec R = alkyle ou aryle,
- L est un bras de liaison comportant un enchaînement linéaire d'au moins 2 liaisons covalentes et n un nombre entier égal à 0 ou 1, et
- -Y-X- représente -CONH-, -NHCO-, -CH₂O-, -CH₂S-.

Avantageusement le réactif pour l'amplification de signal a la formule (7) 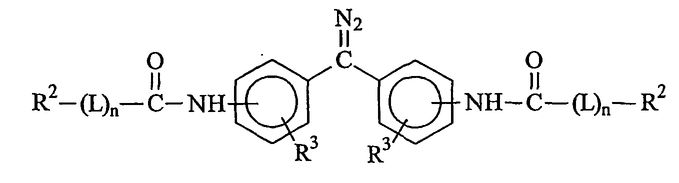 dans laquelle :
- R² représente un marqueur détectable ou au moins deux marqueurs détectables reliés entre eux par au moins une structure multimérique,
- R³ représente H, NO₂, Cl, Br, F, I, R² -(L)ₙ-Y-X-, OR, SR, NR₂, R, NHCOR, CONHR, COOR avec R = alkyle ou aryle, de préférence R³ représente H, NO₂, OCH₃, -CO-NH-(CH₂)₃-(O-CH₂-CH₂)₃-CH₂-NH-R² ou -CO-NH-(CH₂)₃-(O-CH₂-CH₂)₄-CH₂-NH-R², et
- L est un bras de liaison comportant un enchaînement linéaire d'au moins 2 liaisons covalentes et n un nombre entier égal à 0 ou 1.

Toujours un autre réactif de marquage préféré pour l'amplification de signal est le réactif de formule (25) : 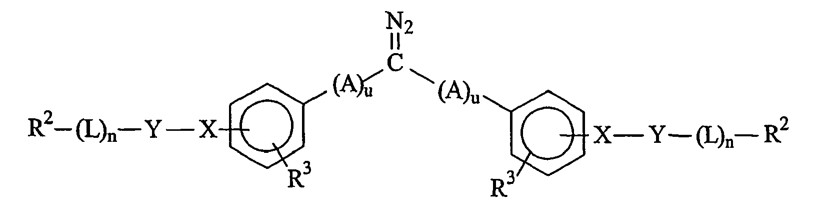 dans laquelle :
- R² représente un marqueur détectable ou au moins deux marqueurs détectables reliés entre eux par au moins une structure multimérique,
- R³ représente H, NO₂, Cl, Br, F, I, R² -(L)ₙ-Y-X-, OR, SR, NR₂, R, NHCOR, CONHR, COOR avec R = alkyle ou aryle,
- L est un bras de liaison comportant un enchaînement linéaire d'au moins deux liaisons covalentes et n un nombre entier égal à 0 ou 1,
- A est un bras de liaison comportant au moins une double liaison covalente permettant la conjugaison de la fonction diazo avec le cycle aromatique et u égal 1, et
- -Y-X- représente -CONH-, -NHCO-, -CH₂O-, -CH₂S-.

Dans un mode préféré de l'invention, le traceur est un composé fluorescent de faible encombrement stérique comme la fluorescéine, le dansyl, les chromophores du type IR (Li-COR Inc, Lincoln NE, USA), des dérivés cyanines comme le Cy5 et le Cy3 (Randolph J.B. and al, Nucleic Acids Res., 25(14), p2923-2929, 1997) et en particulier les dérivés du Cy5 ou bien le traceur est un haptène de faible encombrement stérique comme la biotine ou un dérivé de l'abiétane (voir la demande WO-A-00/07982). Par faible encombrement stérique, on entend un poids moléculaire inférieur à 1000 g/mole.

Dans le cas d'un fluorophore, il est préférable de travailler avec des fluorophores dont la longueur d'onde d'excitation est supérieure à 450 nm, de préférence supérieure à 600 nm.

Dans le cas où le traceur est un haptène qui ne produit pas de signal par lui-même comme par exemple la biotine, la détection est réalisé par la reconnaissance d'un anti-ligand marqué comme décrit plus haut. Dans le cas de la biotine, on utilise de préférence de la streptavidine ou un anticorps anti-biotine couplé à un composé fluorescent comme la fluorescéine, Cy5 ou la phycoérythrine. Dans le cas de l'abiétane, on utilise un anticorps monoclonal comme décrit dans la demande de brevet WO-A-00/07982.

En particulier les réactifs de marquage de l'invention sont solubles dans des solvants polaires comme le DMF, le DMSO, CH₃CN, THF, DMA (diméthylacétamide), NMP (N-méthylpyrrolidone), DME (diméthoxyéthane).

De préférence les réactifs de marquage sont solubles dans le DMSO ou l'eau.

Par solvant miscible à l'eau, on entend un solvant qui est miscible dans une proportion d'au moins 5% en volume avec de l'eau ou un tampon aqueux contenant des sels.

Avantageusement dans les formules précédentes, le bras L comprend un motif éthylène glycol ou polyéthylène glycol pour augmenter la solubilité du réactif dans l'eau.

A est un bras de liaison comportant au moins une double liaison de type éthylénique permettant la conjugaison de la fonction diazométhyle avec le cycle aromatique. Le bras de liaison A a pour fonction d'éloigner la fonction diazométhyle du cyle pour diminuer l'encombrement stérique tout en conservant la stabilité de la fonction diazométhyle. Par « conjugaison », on entend la délocalisation électronique du cycle aromatique le long de la chaîne carbonée du bras de liaison A. A titre d'exemple, le bras A peut avoir la structure suivante : 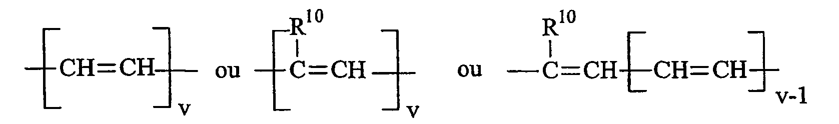 dans laquelle :
- v est un nombre entier compris entre 1 et 10, de préférence v est 1 ou 2, et
- R¹⁰ est H ou un groupement alkyle, de préférence R¹⁰ est H, méthyle ou éthyle.

Ces réactifs peuvent ainsi se fixer en phase homogène sur les molécules biologiques, la phase homogène étant constituée d'une solution sensiblement aqueuse c'est à dire contenant au moins 50% d'eau.

Par « molécule biologique », on entend un composé qui possède au moins un site de reconnaissance lui permettant de réagir avec une molécule cible d'intérêt biologique. A titre d'exemple on peut citer comme molécules biologiques les acides nucléiques, les antigènes, les anticorps, les polypeptides, les protéines, les haptènes.

Le terme « acide nucléique » signifie un enchaînement d'au moins deux désoxyribonucléotides ou ribonucléotides comprenant éventuellement au moins un nucléotide modifié, par exemple au moins un nucléotide comportant une base modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant l'hybridation. Ce polynucléotide peut aussi être modifié au niveau de la liaison internucléotidique comme par exemple les phosphorothioates, les H-phosphonates, les alkyl-phosphonates, au niveau du squelette comme par exemple les alpha-oligonucléotides ( FR 2 607 507) ou les PNA (M. Egholm et al., J. Am. Chem. Soc., 114, 1895-1897, 1992 ou les 2' O-alkyl ribose. L'acide nucléique peut être naturel ou synthétique, un oligonucléotide, un polynucléotide, un fragment d'acide nucléique, un ARN ribosomique, un ARN messager, un ARN de transfert, un acide nucléique obtenu par une technique d'amplification enzymatique telle que :
- PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
- LCR (Ligase Chain Reaction), exposée par exemple dans la demande de brevet EP-A-0.201.184,
- RCR (Repair Chain Reaction), décrite dans la demande de brevet WO-A-90/01069,
- 3SR (Self Sustained Sequence Replication) avec la demande de brevet WO-A-90/06995,
- NASBA (Nucleic Acid Sequence-Based Amplification) avec la demande de brevet WO-A-91/02818, et
- TMA (Transcription Mediated Amplification) avec le brevet US-A-5,399,491.

On parle alors d'amplicons pour désigner les acides nucléiques générés par une technique d'amplification enzymatique.

Chacune de ces modifications peut être prise en combinaison pour peu qu'au moins un phosphate soit présent dans l'acide nucléique.

Par « polypeptide », on entend un enchaînement d'au moins deux acides aminés.

Par « acides aminés », on entend :
- les acides aminés primaires qui codent pour les protéines,
- les acides aminés dérivés après action enzymatique, comme la trans-4-hydroxyproline,
- les acides aminés naturels, mais non présents dans les protéines comme la norvaline, la N-méthyl-L leucine, la staline (voir Hunt S. dans Chemistry and Biochemistry of the amino acids, Barett G.C., ed., Chapman and Hall, London, 1985), et
- les acides aminés protégés par des fonctions chimiques utilisables en synthèse sur support solide ou en phase liquide et les acides aminés non naturels.

Le terme « haptène » désigne des composés non immunogènes, c'est-à-dire incapables par eux-mêmes de promouvoir une réaction immunitaire par production d'anticorps, mais capables d'être reconnues par des anticorps obtenus par immunisation d'animaux dans des conditions connues, en particulier par immunisation avec un conjugué haptène-protéine. Ces composés ont généralement une masse moléculaire inférieure à 3000 Da, et le plus souvent inférieure à 2000 Da et peuvent être par exemple des peptides glycosylés, des métabolites, des vitamines, des hormones, des prostaglandines, des toxines ou divers médicaments, les nucléosides et nucléotides.

Le terme « anticorps » inclut les anticorps polyclonaux ou monoclonaux, les anticorps obtenus par recombinaison génétique, et des fragments d'anticorps tels que des fragments Fab ou F(ab')₂.

Le terme « antigène » désigne un composé susceptible de générer des anticorps.

Le terme « protéine » inclut les holoprotéines et les hétéroprotéines comme les nucléoprotéines, les lipoprotéines, les phosphoprotéines, les métalloprotéines et les glycoprotéines, aussi bien fibreuses que globulaires sous leur forme conformationnelle caractéristique.

Avantageusement la molécule biologique possède un groupe phosphate, c'est-à-dire ayant au moins un motif : 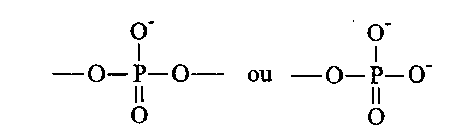 qui est soit présent naturellement dans la molécule biologique, soit peut être introduit par exemple par modification chimique ou enzymatique. Des exemples de modification chimique pour les protéines sont données dans « Chemistry of protein conjugation and cross linking », S.S. Wong, CRC Press, 1991.

De préférence, la molécule biologique est un acide nucléique.

Certains réactifs avantageux de l'invention sont :
a) de formule (8) : 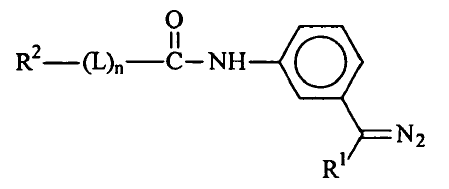 dans laquelle :
   - R¹ représente H ou un groupe alkyle ou aryle ou aryle substitué,
   - R² représente un marqueur détectable ou au moins deux marqueurs détectables reliés entre eux par au moins une structure multimérique,
   - L est un bras de liaison comportant un enchaînement linéaire d'au moins deux liaisons covalentes, et
   - n un nombre entier égal à 0 ou 1.
b) de formule (9) : 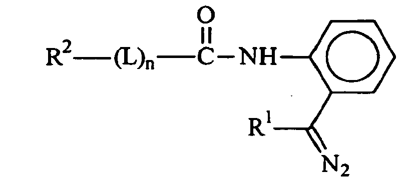 dans laquelle :
   - R¹ représente H ou un groupe alkyle ou aryle ou aryle substitué,
   - R² représente un marqueur détectable ou au moins deux marqueurs détectables reliés entre eux par au moins une structure multimérique,
   - L est un bras de liaison comportant un enchaînement linéaire d'au moins deux liaisons covalentes, et
   - n un nombre entier égal à 0 ou 1.

De préférence, le réactif de marquage a la :
a) formule (11) : 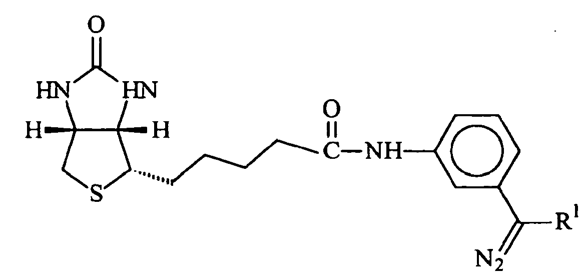 dans laquelle R¹ représente un groupe méthyle ou un phényle.
b) formule (12) : 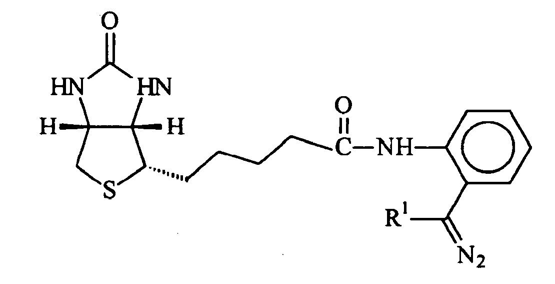 dans laquelle R¹ représente un groupe méthyle ou phényle.

D'autres réactifs préférés selon l'invention ont :
a) la formule (14) : 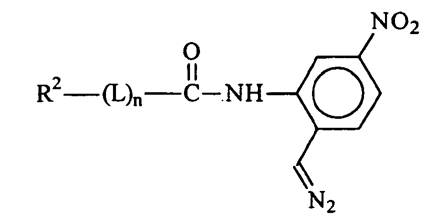 dans laquelle :
   - R² représente un marqueur détectable ou au moins deux marqueurs détectables reliés entre eux par au moins une structure multimérique,
   - L est un bras de liaison comportant un enchaînement linéaire d'au moins deux liaisons covalentes, et
   - n un nombre entier égal à 0 ou 1.
b) la formule (26) : 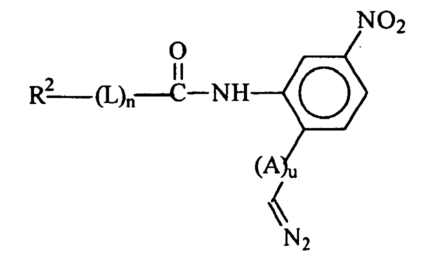 dans laquelle :
   - R² représente un marqueur détectable ou au moins deux marqueurs détectables reliés entre eux par au moins une structure multimérique,
   - A est un bras de liaison comportant au moins une double liaison covalente permettant la conjugaison de la fonction diazo avec le cycle aromatique et u égal 1, et
   L est un bras de liaison comportant un enchaînement linéaire d'au moins deux liaisons covalentes et n un nombre entier égal à 0 ou 1.
c) la formule (15) : 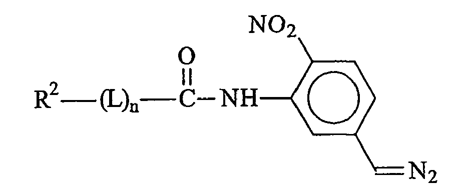 dans laquelle :
   - R² représente un marqueur détectable ou au moins deux marqueurs détectables reliés entre eux par au moins une structure multimérique,
   - L est un bras de liaison comportant un enchaînement linéaire d'au moins deux liaisons covalentes, et
   - n un nombre entier égal à 0 ou 1.
d) la formule (27) : 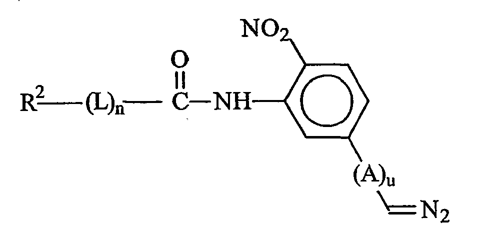 dans laquelle :
   - R² représente un marqueur détectable ou au moins deux marqueurs détectables reliés entre eux par au moins une structure multimérique,
   - A est un bras de liaison comportant au moins une double liaison covalente permettant la conjugaison de la fonction diazo avec le cycle aromatique et u égal 1, et
   - L est un bras de liaison comportant un enchaînement linéaire d'au moins deux liaisons covalentes et n un nombre entier égal à 0 ou 1.

Quelque soit la variante et le mode de réalisation du réactif, L peut comprendre un motif -(O-CH₂-CH₂)-, répété de 1 à 20 fois, préférentiellement de 1 à 10 fois, et encore plus préférentiellement de 2 à 5 fois.

C'est un autre objet de la présente invention que de décrire un procédé de synthèse d'un réactif de marquage ainsi que les réactifs de marquage, stables à la température, susceptibles d'être obtenus par ledit procédé comprenant les étapes suivantes :
a) on dispose d'un dérivé de formule (16 bis) : 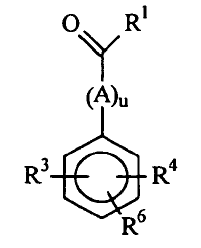 dans laquelle :
   - R¹ représente H ou un groupe alkyle ou aryle ou aryle substitué,
   - R³ et R⁴ représentent indépendamment l'un de l'autre : H, NO₂, Cl, Br, F, I, R⁶, OR, SR, NR₂, R, NHCOR, CONHR, COOR avec R = alkyle ou aryle,
   - R⁶ représente COOH ,COOM, NH₂, OH ou SH avec M = alkyle, en particulier méthyle ou éthyle et R⁶ étant en position *ortho* ou *méta,* et
   - A est un bras de liaison comportant au moins une double liaison covalente permettant la conjugaison de la fonction diazométhyle avec le cycle aromatique et u est un nombre entier égal à 0 ou 1,
b) on dispose d'un marqueur ou d'un précurseur de marqueur possédant une fonction réactive R⁷ complémentaire de R⁶,
c) on fait réagir ensemble la fonction complémentaire dudit marqueur ou précurseur de marqueur avec la fonction R⁶ du dérivé de formule (16 bis) en présence d'au moins un agent de couplage pour former une liaison covalente,
d) on fait réagir un dérivé de l'hydrazine ou l'hydrazine sur la fonction cétone ou aldéhyde pour former une hydrazone, et
e) on transforme l'hydrazone en fonction diazométhyle à l'aide d'un traitement approprié.

Si la fonction R⁶ est COOH ou COOM, la fonction complémentaire R⁷ est NH₂.

Si la fonction R⁶ est NH₂, la fonction complémentaire R⁷ est COOH.

Si la fonction R⁶ est OH, la fonction complémentaire R⁷ est choisie parmi : halogénure d'alkyle, sulfonate, tosylate.

Si la fonction R⁶ est SH, la fonction complémentaire R⁷ est choisie parmi : halogénure d'alkyle, maléimide.

Une variante du procédé de synthèse comprend les étapes suivantes :
a) on dispose d'un dérivé de formule (16 bis) : 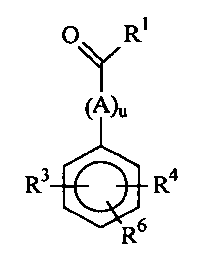 dans laquelle :
   - R¹ représente H ou un groupe alkyle ou aryle ou aryle substitué,
   - R³ et R⁴ représentent indépendamment l'un de l'autre : H, NO₂, CI, Br, F, I, R⁶, OR, SR, NR₂, R, NHCOR, CONHR, COOR avec R est alkyle ou aryle,
   - R⁶ représente COOH ,COOM, NH₂, OH ou SH avec M est alkyle, en particulier méthyle ou éthyle et R⁶ étant en position *ortho* ou *méta,* et
   - A est un bras de liaison comportant au moins une double liaison covalente permettant la conjugaison de la fonction diazométhyle avec le cycle aromatique et u est un nombre entier égal à 0 ou 1,
b) on dispose d'un bras de liaison L possédant au moins deux fonctions réactives R⁸ identiques ou différentes, la première fonction R⁸ complémentaire de R⁶ et la deuxième fonction R⁸ complémentaire de R⁷, et on dispose en outre d'un marqueur ou d'un précurseur de marqueur possédant une fonction réactive R⁷,
c) on fait réagir la première fonction réactive R⁸ du bras de liaison L avec le dérivé de formule (16 bis) en présence d'au moins un agent de couplage pour former une liaison covalente puis on fait réagir la deuxième fonction réactive R⁸ du bras de liaison L avec le marqueur ou le précurseur de marqueur en présence d'au moins un agent de couplage pour former une liaison covalente,
d) on fait réagir un dérivé de l'hydrazine ou l'hydrazine sur la fonction cétone ou aldéhyde pour former une hydrazone, et
e) on transforme l'hydrazone en fonction diazométhyle à l'aide d'un traitement approprié.

Dans ce cas particulier, le procédé comprend une étape supplémentaire où le bras de liaison L est additionné sur le composé (16 bis) avant de faire réagir le marqueur ou précurseur de marqueur. Dans ce cas, le bras de liaison L porte au moins une première fonction réactive R⁸ complémentaire de R⁶, pour permettre le couplage du bras L sur le composé (16 bis), et au moins une deuxième fonction R⁸, permettant le couplage du marqueur ou précurseur de marqueur sur le bras de liaison L, les deux fonctions R⁸ portées par le bras L pouvant être identiques ou différentes en fonction de la stratégie de couplage et des fonctions réactives R⁶ et R⁷, portées respectivement par le composé (16 bis) et le marqueur ou précurseur de marqueur.

Si la fonction R⁶ et/ou la fonction R⁷ est/sont COOH ou COOM, la première et/ou la deuxième fonction complémentaire R⁸ est/sont NH₂.

Si la fonction R⁶ et/ou la fonction R⁷ est/sont NH₂, la première ou la deuxième fonction complémentaire R⁸ est/sont COOH.

Si la fonction R⁶ et/ou la fonction R⁷ est/sont OH, la première et/ou la deuxième fonction complémentaire R⁸ est/sont choisie(s) indépendamment parmi : halogénure d'alkyle, sulfonate, tosylate.

Si la fonction R⁶ et/ou la fonction R⁷ est/sont SH, la première et/ou la deuxième fonction complémentaire R⁸ est/sont choisie(s) indépendamment parmi : halogénure d'alkyle, maléimide.

Dans le cas où le R⁶ est OH ou SH, l'agent de couplage est une base telle la potasse ou le carbonate de potassium ou une base organique.

Dans le cas où le R⁶ est COOH ou NH₂, l'agent de couplage est choisi par exemple parmi les agents de couplage utilisés en synthèse peptidique. On pourra se reporter à l'ouvrage « Peptide Chemistry, a practical textbook » de M. Bodansky, ed Springer Verlag, Berlin, 1988, chapitre V, pages 55-73.

Par « dérivé de l'hydrazine », on entend une molécule possédant la fonction NH₂-NH-. Le tosylhydrazine est un exemple d'un tel dérivé.

La transformation de l'hydrazone en diazométhyle est réalisée par les méthodes usuelles, en particulier l'oxydation par MnO₂.

D'autres méthodes sont utilisables comme décrites dans X. Creary, Organic Syntheses, Wiley : New York, Coll. Vol. VII, p438-443,1990; H. Zollinger, Diazo Chemistry II, VCH, Weinheim, p34-47, 1995; T. L. Holton and H. Shechter, J. Org. Chem., 60, 4725-4729, 1995.

Dans le cas de l'utilisation d'un dérivé tosylhydrazine, la méthode est décrite dans X. Creary, Organic Syntheses ; Wiley : New York, Coll. Vol. VII, p438-443, 1990.

Dans un mode particulier de l'un quelconque des procédés, ledit procédé comprend :
- une étape supplémentaire de protection de la fonction cétone ou aldéhyde (dans le cas où R¹ est H) du composé (16 bis), et
- une étape supplémentaire ultérieure de déprotection de ladite fonction cétone ou aldéhyde. Cette protection est réalisée par un groupement acétal par exemple. La déprotection est effectuée par un moyen approprié comme en milieu acide pour le groupement acétal. L'homme du métier détermine en fonction des composés à quelle étape de synthèse ces deux étapes de protection et de déprotection interviennent.

Selon un autre mode de la présente invention est décrit un procédé de synthèse d'un réactif de marquage ainsi que les réactifs de marquage, stables à la température, susceptibles d'être obtenus par ledit procédé comprenant les étapes suivantes :
a) on dispose d'un dérivé de formule (16) : 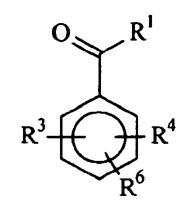 dans laquelle :
   - R¹ représente H ou un groupe alkyle ou aryle ou aryle substitué,
   - R³ et R⁴ représentent indépendamment l'un de l'autre : H, NO₂, Cl, Br, F, I, R⁶, OR, SR, NR₂, R, NHCOR, CONHR, COOR avec R = alkyle ou aryle, et
   - R⁶ représente COOH , NH₂, OH ou SH et R⁶ étant en position *ortho* ou *méta,*
b) on dispose d'un marqueur ou d'un précurseur de marqueur possédant une fonction réactive R⁷ complémentaire de R⁶,
c) on fait réagir ensemble la fonction complémentaire dudit marqueur ou précurseur de marqueur avec la fonction R⁶ du dérivé de formule (16) en présence d'au moins un agent de couplage pour former une liaison covalente,
d) on fait réagir l'hydrazine ou un de ses dérivés sur la fonction cétone ou aldéhyde pour former une hydrazone, et
e) on transforme l'hydrazone en fonction diazométhyle à l'aide d'un traitement approprié.

Si la fonction R⁶ est COOH, la fonction complémentaire R⁷ est NH₂.

Si la fonction R⁶ est NH₂, la fonction complémentaire R⁷ est COOH.

Si la fonction R⁶ est OH, la fonction complémentaire R⁷ est choisie parmi : halogénure d'alkyle, sulfonate, tosylate.

Si la fonction R⁶ est SH, la fonction complémentaire R⁷ est choisie parmi : halogénure d'alkyle, maléimide.

Dans le cas où le R⁶ est OH ou SH, l'agent de couplage est une base telle la potasse ou le carbonate de potassium.

Dans le cas où le R⁶ est COOH ou NH₂, l'agent de couplage est choisi par exemple parmi les agents de couplage utilisé en synthèse peptidique. On pourra se reporter à l'ouvrage « Peptide Chemistry, a practical textbook » de M. Bodansky, ed Springer Verlag, Berlin, 1988, chapitre V, pages 55-73.

Par « dérivé de l'hydrazine », on entend une molécule possédant la fonction NH₂-NH-. Le tosylhydrazine est un exemple d'un tel dérivé.

La transformation de l'hydrazone en diazométhyle est réalisée par les méthodes usuelles, en particulier l'oxydation par MnO₂.

D'autre méthodes sont utilisables comme décrites dans X. Creary, Organic Syntheses, Wiley : New York, Coll. Vol. VII, p438-443, 1990; H. Zollinger, Diazo Chemistry II, VCH, Weinheim, p34-47, 1995; T. L. Holton and H. Shechter, J. Org. Chem., 60,4725-4729, 1995. Dans le cas de l'utilisation d'un dérivé tosylhydrazine, la méthode est décrite dans X. Creary, Organic Syntheses ; Wiley : New York, Coll. Vol. VII, p438-443, 1990.

Un procédé préféré selon l'invention est un procédé où :
a) on dispose d'un dérivé de formule (17) : 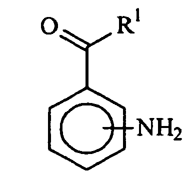 dans laquelle R¹ représente H ou un groupe alkyle ou aryle ou aryle substitué, et le groupe amino est en position *ortho* ou *méta,*
b) on dispose d'un marqueur ou d'un précurseur de marqueur possédant une fonction acide carboxylique,
c) on fait réagir ensemble la fonction carboxylique dudit marqueur ou précurseur de marqueur et la fonction amine primaire du dérivé de formule (17) en présence d'au moins un agent de couplage pour former une liaison amide,
d) on fait réagir de l'hydrazine sur la fonction cétone ou aldéhyde provenant du dérivé de formule (17) pour former une hydrazone, et
e) on oxyde ladite hydrazone en présence de MnO₂ pour former une fonction diazométhyle.

Dans tous les procédés décrits précédemment et avantageusement, le groupe alkyle est un groupe linéaire ou ramifié en C₁-C₄ et le groupe aryle est un groupe phényle éventuellement substitué.

De préférence R⁴ est un méthyle ou un phényle c'est à dire que le dérivé de formule (17) est l'acétophénone ou la benzophénone substitué par l'amine en position *ortho* ou *méta.*

L'agent de couplage est choisi parmi les agents de couplage notamment utilisé en synthèse peptidique comme décrit précédemment et par exemple iBuOCOCl en présence d'une base comme la N-méthylmorpholine.

Par « précurseur de marqueur », on entend un composé ayant au moins une fonction réactive éventuellement protégée différente de la fonction diazométhyle et compatible avec ladite fonction qui permet la fixation d'un marqueur ultérieurement, c'est à dire après l'une quelconque des étapes du procédé et en particulier après l'étape d'oxydation par MnO₂. En particulier, la précurseur de marqueur peut comprendre le bras de liaison L. Un exemple de stratégie utilisant un précurseur de marqueur est donné ci-dessous dans le cas de l'amplification de signal mais d'autres variantes sont possibles en utilisant les différents groupements protecteurs qui sont bien connus de l'homme du métier.

Dans le cas de l'amplification de signal, le procédé de synthèse est similaire. Le précurseur du marqueur a la formule (18) ci-dessous. 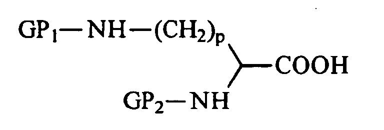 dans laquelle GP₁ et GP₂ représentent deux groupements protecteurs de fonction amine identiques ou différents et p est un nombre entier compris entre 1 et 10, avantageusement 2 et 6 de préférence 4. Avantageusement, GP₁ et GP₂ sont différents pour pouvoir additionner plusieurs motifs comme cela est expliqué ci-dessous.

Des exemples de groupement protecteurs GP1 ou GP2 utilisables dans la présente invention sont données dans T.W. Greene and P.G.M. Wuts, Protective Groups in Organic Synthesis, 2eme édition, John Wiley and Sons, New York, 1991, préférentiellement ceux couramment utilisés en synthèse peptidique comme Boc (tertiobutyloxycarbonyle), Fmoc (9-fluorénylméthylèneoxycarbonyle), Cbz (carboxybenzyle) ou Alloc (allyloxycarbonyle).

En particulier GP1 et GP2 sont respectivement les groupements protecteurs Boc et Fmoc.

La réaction entre ce précurseur qui possède une fonction carboxylique et le dérivé de formule (17) a lieu en présence d'un agent de couplage pour former la liaison amide. Après déprotection dans des conditions usuelles d'un des deux groupements protecteurs, par exemple Fmoc avec une base telle que la pipéridine, la fonction amine libérée est utilisée pour coupler une autre molécule de formule (18). Ce processus est répété autant de fois que nécessaire pour obtenir une multitude de fonctions NH₂ protégé par un groupement protecteur par exemple une fonction Boc. Le motif est additionné entre une (1) et cent (100) fois, de préférence entre une (1) et vingt (20) fois.

On fait réagir de l'hydrazine sur la fonction cétone provenant du dérivé phénylcétone pour former une hydrazone puis on oxyde en présence de MnO₂ pour former un résidu diazométhyle. Puis après déprotection de la fonction amine portant le groupement Boc, un traceur, par exemple une biotine activée par un groupement N-hydroxysuccinimide, est couplé sur les fonctions amines pour conduire à un réactif dont le motif R²-(L)ₙ- est celui de la formule (5).

C'est un autre objet de 1a présente invention que de décrire un procédé, ainsi que les produits obtenus par ce procédé, pour le marquage d'une molécule biologique, en particulier un acide nucléique, comprenant la mise en contact en solution, dans une solution homogène sensiblement aqueuse, d'une molécule biologique et d'un réactif de marquage selon l'invention.

Par « solution sensiblement aqueuse », on entend une solution contenant au moins 50% d'eau. Cette solution contient de préférence des sels comme une solution tampon.

Par « solution homogène », on entend une solution monophasique telle qu'une solution eau/DMSO par opposition à une solution biphasique telle qu'une solution eau/chloroforme.

Les conditions particulières pour les réactions de marquage varient en fonction des molécules biologiques et du marqueur. En ce qui concerne les acides nucléiques, un pH compris entre 5 et 8 permet un marquage efficace. En particulier, un pH compris entre 5,5 et 7,0 est préféré pour l'ensemble des réactifs de l'invention. Avec le réactif de formule (11), la gamme de pH est plus large pour le marquage. Une bonne efficacité de marquage est obtenue pour un pH compris entre 3 et 8 pour ce réactif.

En particulier, le procédé de marquage et de fragmentation d'un acide nucléique simple ou double brin comprend les étapes suivantes dans un ordre quelconque :
- fragmenter l'acide nucléique,
- attacher un marqueur sur au moins un des fragments par l'intermédiaire d'un réactif de marquage choisi parmi les composés de formule (0) selon la présente invention,
ledit réactif se couplant de manière covalente et majoritaire sur au moins un phosphate dudit fragment.

La liaison entre le réactif de marquage et l'acide nucléique est covalente mais il a été précédemment décrit que des interactions non covalentes pouvaient être utilisées notamment dans les systèmes d'empilement, ou dans le cas où le marqueur est détectable indirectement. Le terme « attacher » couvre donc ces différentes possibilités.

De préférence, le réactif de marquage est choisi parmi les composés de formule (1) selon la présente invention.

Les autres nouveaux réactifs selon l'invention qui sont décrits par les formules (1) à (27) ainsi que les réactifs susceptibles d'être obtenus par les procédés de synthèse selon l'invention sont aussi des réactifs préférés selon le procédé de fragmentation et de marquage décrit ci-dessus.

Ce procédé de marquage et de fragmentation est particulièrement utile dans le cas où l'acide nucléique marqué doit s'hybrider avec une multitude d'acides nucléiques, notamment oligonucléotides, fixés sur le support solide à une position prédéterminée pour former une puce à ADN. Par "puce à ADN", on entend un support solide de dimension réduite où sont fixés une multitude de sondes de capture à des positions prédéterminées. En effet, la densité des acides nucléiques fixés sur le support solide impose des contraintes stériques importantes lors de l'hybridation et la fragmentation permet d'améliorer cette étape d'hybridation. Des exemples de ces puces à ADN sont donnés par exemple dans les publications de G. Ramsay, Nature Biotechnology, 16, p40-44, 1998; F. Ginot, Human Mutation, 10, p1-10, 1997 ; J. Cheng et al, Molecular diagnosis, 1(3), p183-200, 1996; T. Livache et al, Nucleic Acids Research, 22(15), p2915-2921, 1994 ; J. Cheng et al, Nature Biotechnology, 16, p541-546, 1998.

La fragmentation et le marquage s'effectuent en une étape ou en deux étapes et le marquage peut s'effectuer indifféremment avant, après ou simultanément avec la fragmentation.

De préférence, le marquage et la fragmentation s'effectuent simultanément c'est-à-dire que les réactifs nécessaires à ces deux étapes sont mis ensemble en solution homogène sensiblement aqueuse avec l'acide nucléique par exemple. C'est notamment le cas pour la fragmentation chimique ou enzymatique. Dans le cas de la fragmentation mécanique par un moyen physique, marquage et fragmentation s'effectuant simultanément signifie que le moyen physique est appliqué à une solution homogène sensiblement aqueuse contenant au moins les acides nucléiques et le réactif de marquage.

La fragmentation de l'acide nucléique s'effectue par voie enzymatique, chimique ou physique.

La fragmentation par voie enzymatique de l'acide nucléique est réalisée par exemple par des nucléases.

La fragmentation par voie physique de l'acide nucléique est réalisée par exemple par sonication ou par radiation.

La fragmentation par voie chimique, si l'acide nucléique est un ARN, est réalisée par les méthodes usuelles (voir par exemple Chem. Rev, 98, 961-990, 1998 de Oivanen M. et al.).

Les complexes de métaux, tels que décrits dans la revue de G. Pratviel et al, Adv. Org. Chem., 45, p251-312, 1998 ou la revue G. Pratviel et al., Angew. Chem. Int. Ed. Engl., 34, p746-769, 1995, sont utilisables pour la fragmentation de l'ADN ou l'ARN.

Dans un premier mode de réalisation, la fragmentation chimique d'ARN est réalisée par des cations métalliques associés ou non à un catalyseur chimique. Dans ce cas, les cations métalliques sont des ions Mg²⁺, Sr²⁺, Ba²⁺, Pb²⁺, Zn²⁺, Cd²⁺, Mn²⁺, Fe²⁺, Co²⁺, Ni²⁺, Ru³⁺, Ce³⁺, Eu³⁺, Tb³⁺, Tm³⁺, Yb³⁺ ou Lu³⁺, le catalyseur chimique est constitué par de l'imidazole, un analogue substitué, par exemple le N-méthyl-imidazole, ou toute molécule chimique ayant une affinité pour l'ARN et portant un noyau imidazole ou un analogue substitué. Les conditions de fragmentation à l'aide de métaux sont bien décrites dans la demande de brevet WO-A-99/65926. Avantageusement, les métaux sont Mg²⁺, Mn²⁺, Zn²⁺, Tb³⁺ ou Ce³⁺, de préférence Mg²⁺, Mn²⁺, Zn²⁺.

Des conditions efficaces de fragmentation sont obtenues avec une concentration en cation métallique comme Mn⁺⁺ entre 2 et 100 mM, une concentration en imidazole entre 2 et 100mM.

Des conditions spécialement efficaces sont obtenues avec une concentration en cation comme Mn⁺⁺ comprise entre 3 et 15 mM, et une concentration en imidazole comprise entre 20 et 50 mM, en particulier 30 mM.

Le pH de la réaction doit être compris entre 5 et 8. Avantageusement, le pH est compris entre 5,5 et 6,5 de préférence le pH est de 6. Le pH de 6 représente donc un compromis très intéressant pour réaliser la combinaison marquage et fragmentation avec de l'ARN (voir discussion précédente sur le marquage).

Dans un deuxième mode de réalisation, la fragmentation chimique de l'ARN est réalisée par action d'une polyamine, comme la spermine, la putrescéine ou la cadavérine. Des concentrations de 5 à 100 mM permettent la fragmentation. Celle-ci est totale à partir de 10 mM de polyamine.

Dans un troisième mode de réalisation, la fragmentation chimique de l'ARN est réalisée par action d'une nucléase artificielle (voir G. Pratviel et al., Adv. Inorg. Chem.,45, p251-312, 1998; D. S. Sigman et al. Chem. Rev., 93, p2295-2316, 1993), comme la 1,10-phénanthroline associée à un cation métallique comme le fer, le cuivre ou le zinc. Ces cations proviennent respectivement de FeSO₄ ou CuCl₂ ou ZnCl₂ en solution. Des concentrations entre 2 et 50 mM de 1,10-phénanthroline sont utilisées pour la fragmentation d'ARN en particulier entre 4 et 10 mM.

La fragmentation par voie chimique de l'ADN est réalisée en mettant en présence l'acide nucléique avec un moyen chimique de création de site abasique. La formation d'un site abasique résulte de la coupure de la liaison N-glycosidique qui lie le sucre 2-désoxyribose à la base nucléique. Il s'agit d'un dépurination pour la perte d'une purine (guanine, adénine) ou d'une dépyrimidination pour les pyrimidiques (cytosine, thymine).

Cette dépurination est spontanée dans des conditions physiologiques (pH 7,4 à 37°C) mais la vitesse de la réaction est très faible de l'ordre de 3.10⁻¹¹ dépurination par seconde, c'est-à-dire inutilisable pour une fragmentation efficace. Pour augmenter la vitesse de réaction, on utilise des agents alkylants qui fragilisent la liaison N-glycosidique ou des enzymes comme des ADN glycosylases, en particulier l'uracile ADN glycosilase.

Le site abasique obtenu par dépurination ou dépyrimidation est très instable. La fragmentation au niveau de ce site est obtenue à température ambiante en milieu basique. En milieu acide, la température élevée accélère également cette fragmentation. L'utilisation de molécules capables d'initier le phénomène de β-élimination accélère aussi la fragmentation.

Un mode préféré de réalisation de la fragmentation est obtenu par l'utilisation d'un pH acide c'est-à-dire un pH inférieur à 5. Avantageusement le pH est de 3.

Un tampon formiate de sodium à pH 3 permet de fragmenter de manière efficace selon l'invention. Ce tampon est compatible avec les conditions de marquage en une étape comme cela sera démontré dans les exemples. Encore plus avantageusement, un milieu acide (HCl, carbonate, H₂SO₄) est utilisé.

Dans un mode particulier de la présente invention et dans le but d'augmenter encore la fragmentation, l'acide désoxyribonucléique contient au moins une base modifiée susceptible de générer un site abasique plus facilement.

Diverses bases modifiées sont utilisables comme les N7-alkyl purines, les N3-alkyl purines, les O6-alkyl purines, les 8-bromopurines, les 8-thiopurines, les 8-alkylthiopurines, les 8 azidopurines ou les 8-alkylsulfonylpurines.

Dans le cas où l'acide nucléique à marquer est généré par une technique d'amplification enzymatique comme la PCR, l'utilisation d'une 8-bromopurine permet d'avoir une incorporation efficace pendant l'amplification, ce qui facilite d'autant le procédé de fragmentation et de marquage selon l'invention, tout en conservant une sensibilité excellente pour l'étape d'amplification enzymatique.

La présente invention décrit une molécule biologique marquée et en particulier un acide nucléique marqué, susceptible d'être obtenu(e) par l'un quelconque des procédés selon l'invention.

La présente invention concerne aussi un kit de détection d'une molécule biologique, en particulier un acide nucléique cible comprenant un réactif de marquage selon l'invention. En fonction des applications du kit, d'autres éléments comme par exemple, des moyens de lyse (micro-organismes et/ou cellules) et/ou des moyens de concentration (comme de la silice ou des particules magnétiques) et/ou des moyens d'amplification enzymatique sont incorporés dans le kit.

L'invention concerne l'utilisation d'une molécule biologique marquée, en particulier un acide nucléique marqué tel que défini ci-dessus, comme sonde de détection d'une molécule biologique cible, et en particulier d'un acide nucléique cible.

L'invention concerne aussi l'utilisation d'un acide nucléique tel que défini ci-dessus, comme cible marquée pouvant se fixer sur une sonde de capture.

Pour permettre la détection et/ou la quantification et/ou la purification de la molécule biologique cible, la molécule biologique marquée est capable de former un complexe avec la molécule biologique cible. A titre d'exemple, pour la mise en évidence d'une molécule cible de type acide nucléique, l'acide nucléique marqué est suffisamment complémentaire de la cible pour s'hybrider spécifiquement en fonction des conditions de réaction, et notamment de la température ou de la salinité du milieu réactionnel.

Le procédé de détection est applicable pour le séquençage, le profil d'expression des ARN messagers ou le criblage de mutations à des fins de recherche ainsi que le criblage de drogues dans l'industrie pharmaceutique, le diagnostic de maladies infectieuses ou génétiques, le contrôle alimentaire ou industriel.

La tendance en matière de diagnostic et notamment pour les maladies infectieuses (SIDA ou Tuberculose par exemple) est de baisser le niveau de sensibilité, jusqu'à la détection d'une molécule unique dans un échantillon qui peut représenter plusieurs millilitres dans le cas d'un prélèvement liquide type sang ou urine ou liquide céphalo-rachidien. Ce niveau de sensibilité ne peut être obtenu que si toutes les étapes depuis le prélèvement de l'échantillon jusqu'au rendu de résultat sont optimisées. Les différents moyens de l'invention permettent cette optimisation sans difficulté car les réactifs, méthodes et procédés de l'invention sont applicables de manière très large à différentes molécules biologiques. En particulier dans le cas où une étape d'amplification enzymatique est nécessaire pour obtenir la sensibilité nécessaire (infection virale ou bactérienne comme VIH, VHC ou Tuberculose), un procédé de marquage et/ou de fragmentation, comme décrit dans la présente invention, permet de ne pas affecter la sensibilité de la technique d'amplification, soit parce qu'il n'est pas nécessaire de remplacer les désoxyribonucléotides ou les ribonucléotides utilisés dans la technique d'amplification enzymatique, soit parce ce que les ribonucléotides ou désoxyribonucléotides incorporés n'altèrent pas la sensibilité.

La chimie de greffage décrite dans la présente invention possède des caractéristiques telles, du point de vue réactivité et spécificité, que d'autres applications sont décrites ci-après :
- Dans un premier mode de réalisation, cette chimie de greffage est appliquée à la fixation covalente d'acides nucléiques sur un support solide.
- Dans une première variante du procédé, un précurseur de la fonction diazométhyle, tel une cétone ou hydrazine comme décrit précédemment, est introduit pendant la synthèse chimique et la fonction diazométhyle est introduite sur les acides nucléiques dans une deuxième étape.
- Dans une deuxième variante préférée du procédé, les fonctions diazométhyles sont introduites sur le support solide et les acides nucléiques sont fixés sur le support solide par l'intermédiaire des phosphates des acides nucléiques et en particulier des phosphates terminaux (5' ou 3').

L'introduction de phosphate à l'extrémité 3' ou 5' des acides nucléiques est bien connue (voir « Protocols for Oligonucleotides and Analogs, Synthesis and Properties » édité par S. Agrawal, Humana Press, Totowa, New Jersey).

Dans un mode de réalisation particulier d'un tel support solide, un réactif de marquage portant un ligand, en particulier un haptène comme la biotine ou l'abiétane, est fixé sur le support solide sur lequel est fixé de manière covalente ou par adsorption un anti-ligand, comme la streptavidine ou un anticorps par exemple. Ces supports solides sont bien connus dans l'état de la technique et sont même commercialement disponibles (plaque de microtitration-streptavidine ou latex-streptavidine par exemple). La fonction du marqueur n'est plus dans ce cas de permettre la détection mais de permettre la fixation du réactif de marquage sur le support solide. La fonction diazométhyle est alors disponible pour réagir sur des acides nucléiques. Les dérivés de formule (11), (12) sont des exemples de réactifs utilisables pour la fabrication d'un tel support solide. La technique des anticorps monoclonaux permet de préparer des anticorps contre un grand nombre de marqueur comme la fluorescéine ou un dérivé du Cy5. L'homme du métier peut mettre en oeuvre un support solide avec les réactifs de marquage de la présente invention sans difficulté excessive par ce mode de préparation indirect du support solide dans lequel une réaction ligand/anti-ligand est utilisée pour fixer la fonction diazométhyle sur le support solide.

Un deuxième mode de réalisation du support solide concerne les supports particulaires comme les latex. Différents modes de polymérisation peuvent être utilisés pour préparer les particules portant une fonction diazométhyle à partir d'un monomère fonctionnel polymérisable portant soit une fonction diazométhyle soit préférentiellement une fonction précurseur de la fonction diazométhyle comme un aldéhyde ou une cétone et notamment :
- Polymérisation à réacteur fermé appelée « batch » : les monomères sont introduits dans le réacteur avant le début de réaction avec les autres ingrédients et sans ajout ultérieur. En raison de la différence de réactivité des monomères, ce procédé conduit souvent à l'apparition d'une dérive de composition. Celle-ci se manifeste par l'obtention de macromolécules ayant des compositions qui varient considérablement en fonction de la conversion. Cette méthode est peu efficace pour l'incorporation en surface car une partie importante du monomère fonctionnel risque d'être perdue soit à l'intérieur des particules, soit sous forme de polymère hydrosoluble. Lorsque la copolymérisation est effectuée en « batch » avec des monomères de nature polaire, on obtient des particules plus petites, en grand nombre, mais avec une conversion limitée. Ce comportement est lié à l'importante solubilité dans l'eau de ces monomères, et il est attribué à la prépondérance du mécanisme de nucléation homogène.
- Polymérisation en semi-continu : une partie au moins des monomères est introduite dans le réacteur sur une période comprise entre le début de la réaction et la fin de celle-ci. Ce rajout peut être effectué à une vitesse fixe ou bien suivant un profil donné. Le but est de contrôler l'addition du mélange de monomères de façon à obtenir un copolymère de composition contrôlée (contrôle de la composition de l'interface); c'est ainsi qu'on se place souvent dans des conditions d'addition telles que la vitesse de polymérisation soit plus grande que celle d'addition.
- Polymérisation par addition différée appelée « shot » : une fois que la réaction de polymérisation est en cours, le monomère fonctionnel seul, ou en présence du monomère de base, est introduit dans le système d'une façon contrôlée. Le succès de l'opération dépend donc du degré de connaissance préalable de la cinétique de copolymérisation. C'est une méthode efficace pour favoriser l'incorporation superficielle. La sélection des conditions expérimentales (degré de conversion au moment de l'addition, composition et concentration du mélange des monomères) permet d'optimiser les rendements de surface.
- Polymérisation sur semence : elle consiste à introduire le monomère fonctionnel dans le système contenant un latex déjà constitué et parfaitement caractérisé. Le monomère fonctionnel peut être additionné seul ou en mélange avec le monomère de base de la semence, en une étape ou en semi-continu.

Les techniques de polymérisation sur semence, polymérisation par addition différée, polymérisation en semi-continu sont préférées car elles conduisent à un maximum d'incorporation du dérivé portant le précurseur de la fonction diazométhyle en surface. Des exemples de particules portant des fonctions aldéhydes sont donnés par exemple dans B. Charleux et al, Die Makromolecular Chem., 193, p 187 et p. 205, 1992 ou dans le brevet EP-B-0.350.407.

C'est un autre objet de la présente invention que de décrire un intermédiaire de fixation stable à la température de formule (30) : 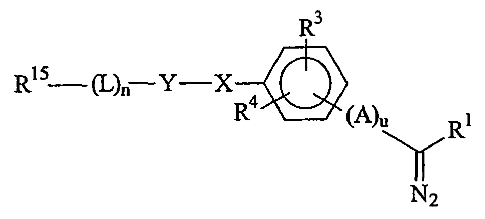 dans laquelle :
- R¹ représente H ou un groupe alkyle, aryle ou aryle substitué,
- R¹⁵ représente une fonction réactive nucléophile ou électrophile, en particulier COOH, NH₂, SH, OH, O-NH₂, alkylcétone, aldéhyde, isocyanate, isothiocyanate, maléimide, halogénure d'alkyle, ester activé, tosylate
- L est un bras de liaison comportant un enchaînement linéaire d'au moins deux liaisons covalentes et n un nombre entier égal à 0 ou 1,
- R³ et R⁴ représentent indépendamment l'un de l'autre : H, NO₂, Cl, Br, F, I, R¹⁵ -(L)ₙ-Y-X-, OR, SR, NR₂, R, NHCOR, CONHR, COOR avec R = alkyle ou aryle,
- A est un bras de liaison comportant au moins une double liaison covalente permettant la conjugaison de la fonction diazo avec le cycle aromatique et u est un nombre entier compris entre 0 et 2, préférentiellement de 0 ou 1,
- -Y-X- représente -CONH-, -NHCO-, -CH₂O-, -CH₂S-, et
- le substituant portant la fonction diazo étant en position *ortho* ou *méta.*

Des intermédiaires de fixation préférés selon l'invention sont de formule (30bis) : 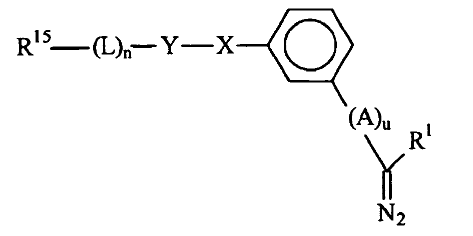 dans laquelle :
- R¹ représente H ou un groupe alkyle, aryle ou aryle substitué,
- R¹⁵ représente une fonction réactive nucléophile ou électrophile, en particulier COOH, NH₂, SH, OH, O-NH₂, alkylcétone, aldéhyde, isocyanate, isothiocyanate, maléimide, halogénure d'alkyle, ester de N-hydroxysuccinimide, tosylate.
- L est un bras de liaison comportant un enchaînement linéaire d'au moins deux liaisons covalentes et n un nombre entier égal à 0 ou 1,
- A est un bras de liaison comportant au moins une double liaison covalente permettant la conjugaison de la fonction diazo avec le cycle aromatique et u est un nombre entier compris entre 0 et 2, préférentiellement de 0 ou 1, et
- -Y-X- représente -CONH-, -NHCO-, -CH₂O-, -CH₂S-.

Un troisième mode de réalisation du support solide consiste à disposer d'un support solide comprenant une première fonction réactive nucléophile ou électrophile, comme par exemple NH₂, SH, OH, O-NH₂, alkylcétone, aldéhyde, isocyanate, isothiocyanate, maléimide, halogénure d'alkyle, ester de N-hydroxysuccinimide, tosylate, puis à faire réagir un intermédiaire de fixation, de formule (30) ou (30 bis) décrites ci-dessus, dont la fonction R¹⁵ est complémentaire de la première fonction réactive du support solide. Cette réaction entre le support solide et l'intermédiaire de fixation s'effectue en présence, éventuellement, d'un agent de couplage pour former une liaison covalente.

Un tel support solide comprenant au moins une fonction diazométhyle, selon les différents modes de réalisation décrits ci-dessus, en particulier un support solide sur lequel est fixé indirectement un réactif de marquage de l'invention, est aussi un objet de la présente invention ainsi que le support solide comprenant des acides nucléiques fixés sur le support solide par l'intermédiaire des fonctions diazométhyles.

Une première application d'un tel support solide est la fabrication de puces à ADN. Des méthodes existent pour répartir des acides nucléiques sur le support solide en des positions discrètes et prédéterminées.

Le brevet US-A-6,110,426 propose une méthode pour réaliser ces puces à ADN à l'aide d'un capillaire que l'on met en contact sur une surface solide pour délivrer un volume contrôlé de liquide. Un contact effectif a lieu entre l'extrémité du capillaire et le support solide pour que la goutte se dépose par capillarité. De même, le brevet US-A-6,083,763 décrit un ensemble de capillaires coulissant dans un dispositif de façon à compenser les différences de hauteur de chacun d'eux. Ils sont amenés au contact d'une surface plane pour le dépôt par capillarité d'oligonucléotides spécifiques.

Le brevet US-A-6,083,762 propose une système de répartition de gouttes comprenant un microdispenseur couplé à un transducteur piézo-électrique pour éjecter des volumes de goutte inférieurs au nanolitre sur une surface solide. Un résultat semblable est obtenu en appliquant une source chaude sur la paroi d'un capillaire pour former une bulle qui éjecte un volume défini de solution (voir T. Okamoto et al., Nature Biotechnology, 18, p438-441, 2000).

La fonction diazométhyle permet ainsi de greffer de manière covalente les acides nucléiques sur le support. Le greffage est simple, la liaison est stable, par rapport à l'adsorption notamment, et la sélectivité de la réaction par rapport au phosphate terminal permet de réaliser un couplage orienté de l'acide nucléique sur le support solide, ce qui facilite d'autant les étapes d'hybridation ultérieures en diminuant l'encombrement stérique.

Une deuxième application d'un support solide selon l'invention est la purification des acides nucléiques.

Dans le cas de la purification, cette purification est soit directe (le support solide porteur de fonctions diazométhyles réagit avec les acides nucléiques à purifier) soit indirecte (des acides nucléiques de capture sont fixés sur le support solide). Ces acides nucléiques de capture sont suffisamment complémentaires de la cible à capturer pour s'hybrider avec le degré de spécificité souhaité et c'est le complexe « acides nucléiques de capture/support solide » qui permet la purification des acides nucléiques cibles.

Le support solide est de préférence sous forme dispersée pour l'utilisation en purification comme des particules de latex, par exemple des particules magnétiques.

Par « étape de purification », on entend notamment la séparation entre les acides nucléiques des micro-organismes et les constituants cellulaires relargués dans l'étape de lyse qui précéde la purification des acides nucléiques. Ces étapes de lyse sont bien connues à titre d'exemple indicatif, on peut utiliser les méthodes de lyse telles que décrite dans les demandes de brevet :
- WO-A-00/60049 sur la lyse par sonication,
- WO-A-00/05338 sur la lyse mixte magnétique et mécanique,
- WO-A-99/53304 sur la lyse électrique, et
- WO-A-99/15621 sur la lyse mécanique.

L'homme du métier pourra utiliser d'autres méthodes de lyse bien connues telles que les chocs thermiques ou osmotiques ou les traitements par des agents chaotropiques, tels que les sels de guanidium (brevet US-A-5,234,809).

Cette étape permet généralement de concentrer les acides nucléiques. A titre d'exemple, on peut utiliser des particules magnétiques (voir à ce sujet les brevets US-A-4,672,040 et US-A-5,750,338), et ainsi purifier les acides nucléiques, qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO-A-97/45202 et WO-A-99/35500.

Le terme "support solide" tel qu'utilisé ici inclut tous les matériaux sur lesquels peut être fixé un acide nucléique. Des matériaux de synthèse, ou des matériaux naturels, éventuellement modifiés chimiquement, peuvent être utilisés comme support solide, notamment les polysaccharides, tels que les matériaux à base de cellulose, par exemple du papier, des dérivés de cellulose tels que l'acétate de cellulose et la nitrocellulose, ou le dextran ; des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels, etc. Le support solide peut être sous la forme d'une plaque de microtitration, d'une membrane, d'une particule ou d'une plaque sensiblement plane de verre ou silicium ou dérivés.

L'invention concerne enfin un procédé de capture d'acides nucléiques comprenant les étapes suivantes :
- on dispose d'un support solide sur lequel est fixé directement ou indirectement au moins une molécule de formule (0) selon la présente invention,
- on met en contact un échantillon biologique susceptible de contenir des acides nucléiques libres, et
- on lave le support solide où la (ou les) molécule(s) sont fixée(s) de manière covalente au moins à un acide nucléique.

Des informations complémentaires peuvent être trouvées dans une autre demande de brevet de la Demanderesse, déposée le 4 mai 2001 et publié sous le numéro FR-A-2824335, ainsi que de son extension internationale déposée le même jour que la présente invention.

Les figures et exemples ci-joints représentent des modes particuliers de réalisation et ne peuvent pas être considérés comme limitant la portée de la présente invention.

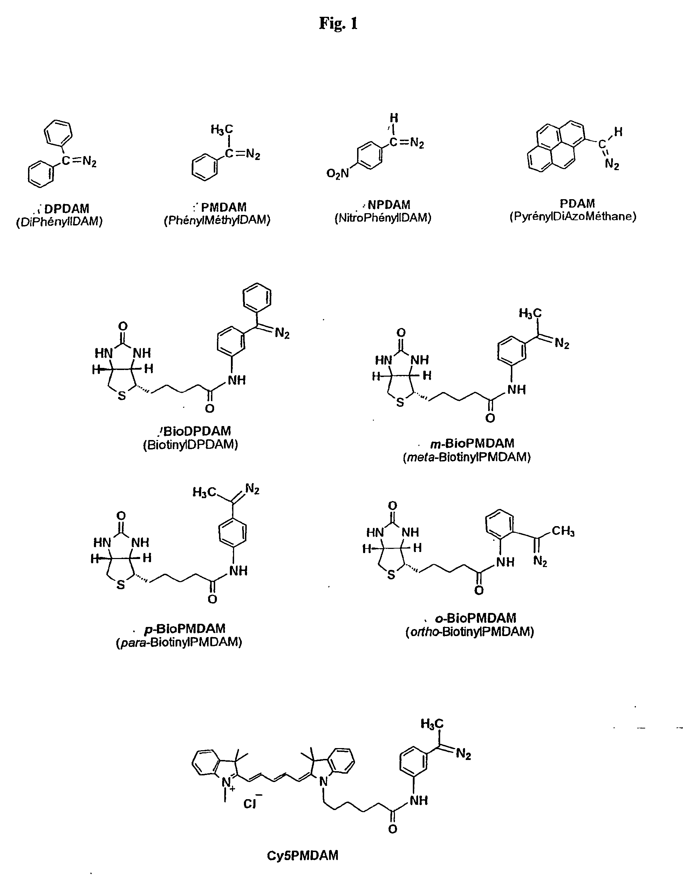

La figure 1 représente les formules développées de différents réactifs utilisés dans la présente invention ainsi que l'abréviation les désignant.(o- signifie *ortho, m- méta* et p- *para)*
Les figures 2A à 2I représentent les profils en fonction du temps analysés par électrophorèse capillaire du couplage covalent de différents réactifs portant une fonction diazométhyle sur l'uridine 3' monophosphate (3'-UMP) selon l'exemple 6.1 :
   - PDAM en figure 2A,
   - DPDAM à 2 millimoles par litre (mM) en figure 2B,
   - DPDAM à 20 mM en figure 2C,
   - PMDAM en figure 2D,
   - NPDAM en figure 2E,
   - BioDPDAM en figure 2F,
   - *méta*-BioPMDAM en figure 2G,
   - *para*-BioPMDAM en figure 2H, et
   - *ortho*-BioPMDAM en figure 2I.
Les figure 3A à 3D représentent les profils en fonction du temps analysés par électrophorèse capillaire de la réaction du *méta*-BioPMDAM sur quatre (4) nucléotides 3'-monophosphate selon l'exemple 6.2 :
   - 3'-CMP en série ribonucléotide selon la figure 3A,
   - 3'-AMP en série ribonucléotide selon la figure 3B,
   - 3'-GMP en série ribonucléotide selon la figure 3C, et
   - 3'-TMP en série désoxyribonucléotide selon la figure 3D.
La figure 4 représente les profils en fonction du temps analysés par électrophorèse capillaire de la réaction du *méta*-BioPMDAM sur un dinucléotide 5'-ApUp selon l'exemple 6.3.
Les figures 5A à 5D représentent le spectre RMN du proton dans D₂O des différents conjugués entre le réactif *méta*-BioPMDAM et quatre (4) ribonucléotides 3'-monophosphate selon l'exemple 6.4 :
   - 3'-GMP en figure 5A,
   - 3'-AMP en figure 5B,
   - 3' CMP en figure 5C, et
   - 3' UMP en figure 5D.

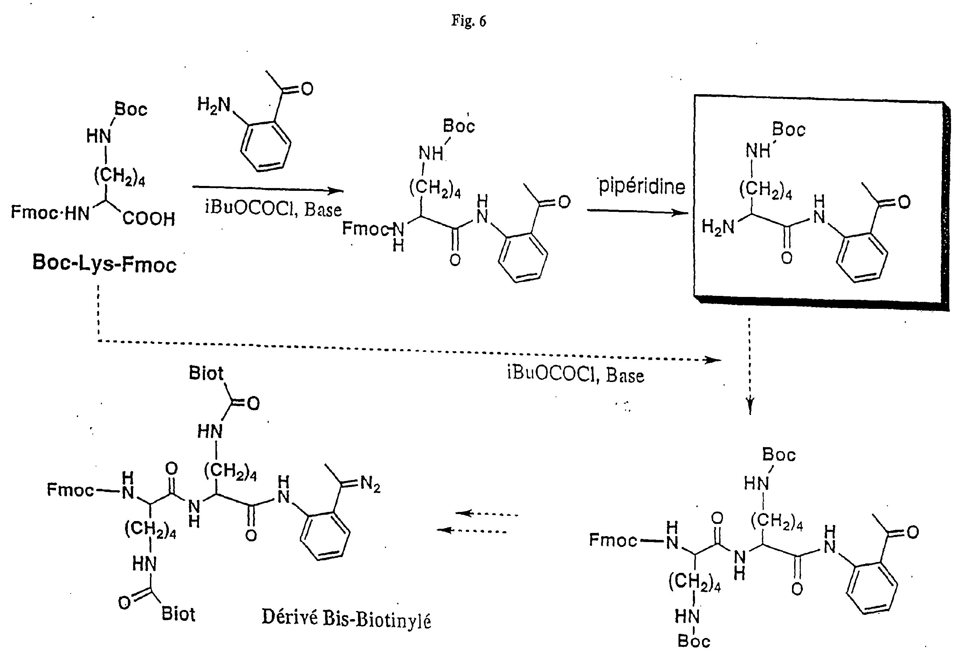

La figure 6 représente un schéma de synthèse d'un réactif de marquage portant deux (2) biotines pour l'amplification chimique du signal.

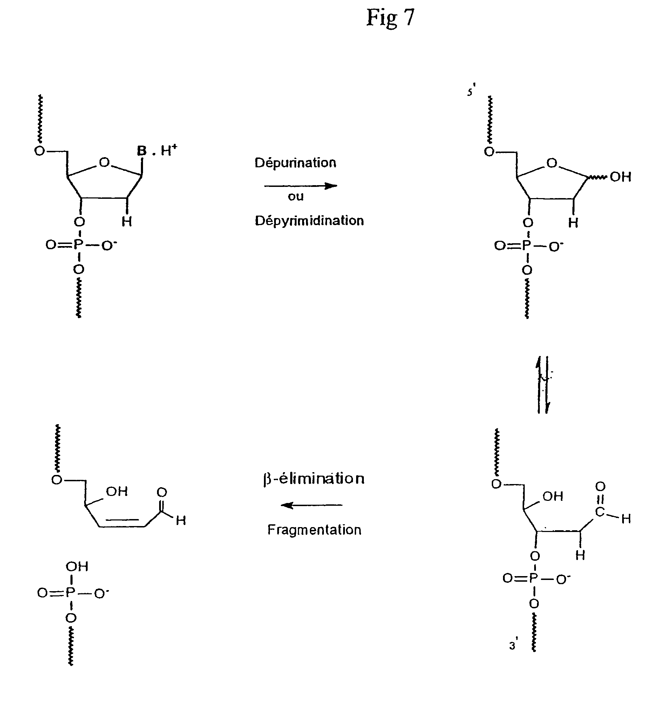

La figure 7 représente le mécanisme de fragmentation en milieu acide par la formation de site abasique.
La figure 8 montre selon l'exemple 16.1 la cinétique de dégradation à pH acide pour différents nucléosides modifiés (8-bromo-2'-désoxyadénosine (8-BrdA) et la 5-bromo-2'-désoxyoxycytidine (5-BrdC)) ainsi que les quatre (4) nucléosides naturels (dA, dC, dG et dT). Les résultats sont représentés sous forme de pourcentage d'hydrolyse du nucléoside de départ (en ordonnée) par rapport au temps de réaction en minutes (en abscisse).

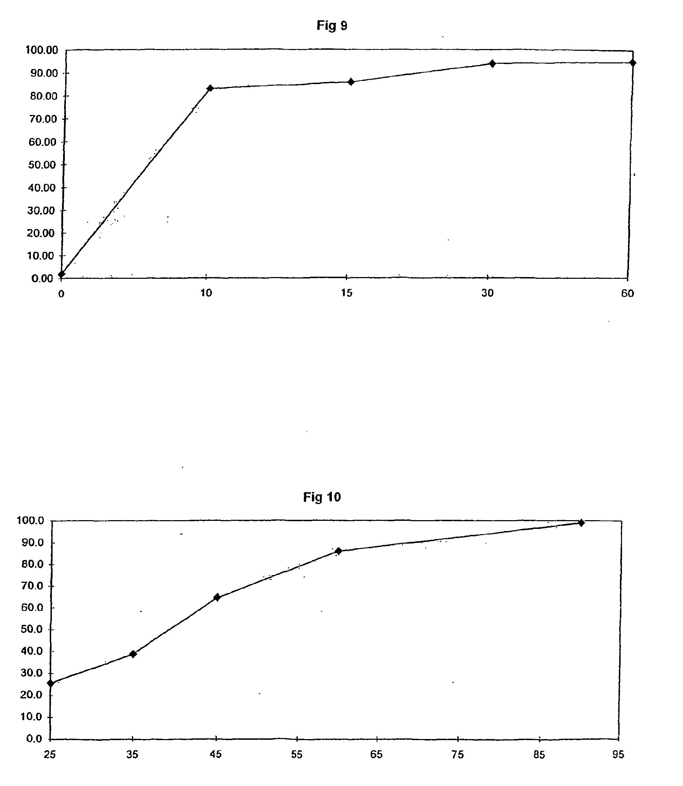

La figure 9 représente selon l'exemple 19.2 la cinétique de marquage en fonction du temps à une température de 60°C avec le réactif PDAM sur un oligodésoxynucléotide (ODN) synthétique 5'-phosphate. Les résultats sont représentés sous forme de pourcentage de marquage par rapport au temps de réaction exprimé en minutes (min) (en abscisse).
La figure 10 représente selon l'exemple 19.3 le pourcentage de marquage en fonction de la température de réaction. Les résultats sont présentés sur la figure 10 avec en ordonnée le pourcentage de marquage et en abscisse la température de réaction en °C.

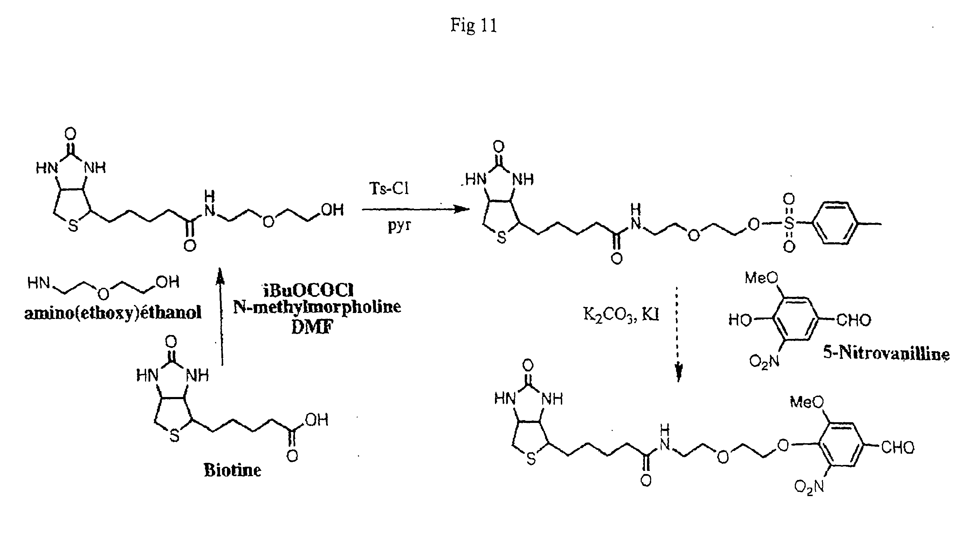

La figure 11 représente une voie de synthèse pour un réactif selon la formule (4') en utilisant le réactif commercial 5-Nitrovanilline. La fonction aldéhyde permet d'obtenir la fonction diazométhyle par formation de l'hydrazone puis oxydation de l'hydrazone par MnO₂.

### Exemple 1 : Synthèse de réactifs avec la biotine :

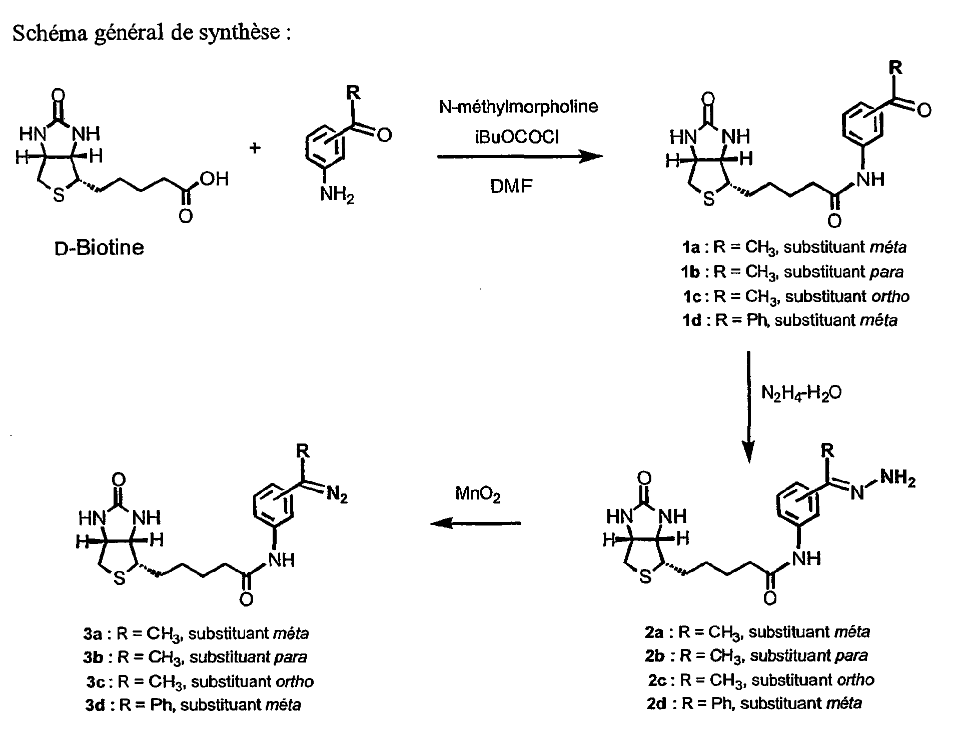

### Exemple 1.1 : Synthèse du méta-BioPMDAM :

### • Composé biotine méta-acétophénone 1a :

On solubilise 1a D-biotine (1,0 gramme (g), 4,1 millimoles (mmol)) dans 45 millilitres (mL) de DMF anhydre à chaud. On refroidit à 0°C sous argon, puis on ajoute successivement la *N-*méthylmorpholine (590 microlitres (µL), 5,33 mmol) et le chloroformiate d'isobutyle (840 µL, 6,60 mmol). On laisse sous agitation pendant 30 minutes (min), puis on ajoute la 3-aminoacétophénone (824 mg, 6,10 mmol) et la *N*-méthylmorpholine (480 µL, 4,35 mmol), dans 10 mL de DMF. La solution est maintenue sous agitation à 0°C pendant 2 heures (h), puis on évapore à sec. On reprend le résidu dans 3 mL de MeOH, puis on ajoute 50 mL d'eau.

Le précipité obtenu est filtré, lavé avec de l'eau, du CH₂Cl₂ et de l'éther pour donner 1,2 g (80 %) de produit **1a** brut. Une recristallisation dans le couple MeOH-H₂O donne **1a** (1,01 g, 70 %) sous forme d'une poudre blanche.

F 145°C. - IR (KBr) : 3280, 2931, 2857, 1691, 1590,1540, 1487, 1434, 1298, 1266 cm⁻¹. - RMN ¹H (300 MHz, DMSO-d₆) δ = 1,3-1,7 (m, 6 H) ; 2,33 (t, *J* = 8 Hz, 2 H) ; 2,55 (s, 3 H) ; 2,58 ; (d, *J* = 12 Hz, 1 H) ; 2,83 (dd, *J* = 12 et 5 Hz, 1 H) ; 3,13 (m, 1 H) ; 4,15 (m, 1 H) ; 4,31 (m, 1 H) ; 6,34 (s, 1 H) ; 6,41 (s, 1 H) ; 7,44 (t, *J* = 8 Hz, 1 H) ; 7,64 (d, *J* = 8 Hz, 1 H) ; 7,85 (d, *J* = 8 Hz, 1 H) ; 8,17 (s, 1 H) ; 10,05 (s, 1 H). - MS (FAB/glycérol), *m*/*z*: 362 [M+H]⁺.

### µ Composé méta-hydrazone 2a :

Une solution de **1a** (500 mg, 1,38 mmol) et d'hydrazine monohydrate (200 µL, 4,15 mmol) dans de l'éthanol absolu (8 mL) est chauffée à reflux pendant 2 h. Après refroidissement à température ambiante, le précipité blanc est filtré, lavé avec de l'eau, puis avec de l'éther et séché. On obtient ainsi 385 mg (74 %) de produit **2a** sous forme d'une poudre blanche.

F 185°C. - IR (KBr): 3298, 2931, 2857, 1698, 1665, 1626, 1541, 1494, 1470, 1446, 1330, 1265 cm⁻¹. - RMN ¹H (300 MHz, DMSO-d₆) δ = 1,3-1,7 (m, 6 H) ; 1,98 (s, 3 H) ; 2,26 (t, *J* = 8 Hz, 2 H) ; 2,56 ; (d, *J =* 12 Hz, 1 H) ; 2,81 (dd, *J =* 12 et 5 Hz, 1 H) ; 3,11 (m, 1 H) ; 4,13 (m, 1 H) ; 4,29 (m, 1 H) ; 6,39 (s, 3 H) ; 6,42 (s, 1 H) ; 7,22 (m, 2 H) ; 7,50 (d, *J* = 8 Hz, 1 H) ; 7,84 (s, 1 H) ; 9,82 (s, 1 H). - MS (FAB/glycérol), *m*/*z*: 376 [M+H]⁺.

### • Composé méta-diazométhane 3a :

On solubilise **2a** (180 mg, 0,48 mmol) dans 2 mL de DMF. On ajoute alors MnO₂ (340 mg, 3,9 mmol). Après 30 minutes d'agitation à température ambiante, le mélange est filtré à travers un entonnoir fritté contenant de la célite (épaisseur : 0,5 cm) et des tamis moléculaires en poudre 3 Å (0,5cm). Le mélange réactionnel est concentré jusqu'à un volume d'environ 0,5 mL, puis 5 mL d'éther sont ajoutés. Le précipité résultant est filtré, lavé à l'éther puis séché. Le composé **3a** (170 mg, 95 %) est obtenu sous forme d'une poudre rose.

F 160°C. - IR (KBr) : 3278, 2935, 2859, 2038, 1704, 1666, 1605, 1577, 1536, 1458, 1430, 1263 cm⁻¹. - RMN ¹H (300 MHz) δ = 1,3-1,7 (m, 6 H) ; 2,11 (s, 3 H); 2,28 (t, *J* = 8 Hz, 2 H); 2,57 ; (d, *J* = 12 Hz, 1 H) ; 2,81 (dd, *J* =12 et 5 Hz, 1 H) ; 3,11 (m, 1 H) ; 4,13 (m, 1 H) ; 4,29 (m, 1 H) ; 6,33 (s, 1 H) ; 6,41 (s, 1 H) ; 6,60 (m, 1 H) ; 7,25 (m, 3 H) ; 9,84 (s, 1 H). ).

### Exemple 1.2 : Synthèse du para-BioPMDAM :

### • Composé biotine para-acétophénone 1b :

On solubilise la D-biotine (1 g, 4,1 mmol) dans 45 mL de DMF anhydre à chaud. On refroidit à 0°C sous argon puis on ajoute successivement la *N*-méthylmorpholine (590 µL, 5,33 mmol) et chloroformiate d'isobutyle (840 µl, 6,60 mmol). On laisse sous agitation pendant 30 min, puis on ajoute la 4-aminoacétophénone (824 mg, 6,10 mmol). La solution est maintenue sous agitation à 0°C pendant 2 h puis on évapore à sec. On reprend le résidu dans 50 mL d'eau. Le précipité obtenu est filtré, lavé avec de l'eau puis avec 50mL de MeOH à chaud. Le précipité blanc est dissous dans du DMF en chauffant puis la solution obtenue est filtrée et lavée au MeOH. Le filtrat est récupéré et évaporé à sec pour donner 888 mg de **1b** (2,46 mmol, 60 %).

F 260°C. - IR (KBr) : 3260, 2930, 2358, 1706, 1673, 1610, 1526, 1401, 1380, 1322, 1257, 1150 cm⁻¹. - RMN ¹H (200 MHz, DMSO-d₆) δ = 8,82 (s, 1H, NH-CO) ; 7,57 (d, 2H, *J* = 9 Hz, Ar-H) ; 6,83 (d, 2H, *J* = 9 Hz, Ar-H); 6,40 (s large, 1H, NH-CO-NH) ; 6,32 (s large, 1H, NH-CO-NH); 4,28 (m, 1H, CH₂-CH-NH) ; 4,12 (m, 1H, CH-CH-NH) ; 3,11 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S); 2,35 (t, 2H, *J* = 8 Hz, CH₂-CO); 2,10 (s, 3H, CH₃); 1,60-1,34 (m, 6H, (CH₂)₃).

### • Composé para-hydrazone 2b :

Le composé **1b** (870 mg, 2,4 mmol) est dissous à chaud dans l'éthanol (99 %, 8 mL) puis l'hydrazine monohydrate (995 µL, 19,5 mmol) est ajoutée. La solution est chauffée à reflux pendant 3 h. Le précipité blanc obtenu est filtré, lavé avec de l'eau glacée. On obtient ainsi 820 mg (90 %) de produit **2b** sous forme d'une poudre blanche.

F 305°C. - IR (KBr) : 3281, 3183, 2930, 2857, 1698, 1658, 1593, 1521, 1459, 1401, 1325, 1263,1187 cm⁻¹. - RMN ¹H (200 MHz, DMSO-d₆) δ = 9,68 (s, 1H, NH-CO) ; 7,52 (s, 4H, *J* = 9 Hz, Ar-H) ; 6,43 (s large, 1H, NH-CO-NH) ; 6,35 (s large, 1H, NH-CO-NH) ; 6,21 (s, 2H, NH₂) 4,29 (m, 1H, CH₂-CH-NH) ; 4,12 (m, 1H, CH-CH-NH) ; 3,12 (m, 1H, CH-S) ; 2,81 et 2,56 (système ABX, 2H, ²*J*_{AB}= 5 Hz, ³*J*_{AX} = 12 Hz, ³*J*_{BX} = 0 Hz, CH₂-S) ; 2,32 (t, 2H, *J* = 8 Hz, CH₂-CO) ; 1,97 (s, 3H, CH₃) ; 1,63-1,36 (m, 6H, (CH₂)₃).

### • Composé para-diazométhane 3b :

On solubilise **2b** (200 mg, 0,53 mmol) dans 10 mL de DMF. On ajoute alors 800 mg de MnO₂. Après 10 minutes d'agitation, le mélange est filtré à travers une couche mixte Célite (0,5 cm) - tamis moléculaire (0,5 cm en poudre). Le mélange réactionnel est évaporé à sec puis lavé à l'éther et séché. Le composé **3b** (190 mg, 96 %) est obtenu sous forme d'une poudre rose.

F 180°C (dec). - IR (KBr) : 3257, 2930, 2857, 2032, 1698, 1597, 1524, 1510, 1455, 1404, 1307, 1259, 1180 cm⁻¹. - RMN ¹H (200 MHz, DMSO-d₆) δ = 10,18 (s, 1H, NH-CO) ; 7,88 (d, 2H, *J* = 6 Hz, Ar-H) ; 7,7 (d, 2H, *J* = 6 Hz, Ar-H) ; 6,41 (s large, 1H, NH-CO-NH) ; 6,34 (s large, 1H, NH-CO-NH) ; 4,28 (m, 1H, CH₂-CH-NH) ; 4,12 (m, 1H, CH-CH-NH) ; 3,11 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S) ; 2,35 (t, 2H, *J* = 8 Hz, CH₂-CO) ; 2,10 (s, 3H, CH₃) ; 1,60-1,34 (m, 6H, (CH₂)₃).

### Exemple 1.3 : Synthèse de l'ortho-BioPMDAM :

### • Composé biotine ortho-acétophénone 1c :

On solubilise la D-biotine (1 g, 4,1 mmol) dans 45 mL de DMF anhydre à chaud. On refroidit à 0°C sous argon puis on ajoute successivement la *N*-méthylmorpholine (590 µL, 5,33 mmol) et chloroformiate d'isobutyle (840 µL, 6,60 mmol). On laisse sous agitation pendant 30 min, puis on ajoute la 2-aminoacétophénone (824 mg, 6,10 mmol). La solution est maintenue sous agitation à température ambiante pendant 3 h 30 puis on évapore à sec. On reprend le résidu dans 50 mL d'eau. Le précipité obtenu est filtré, lavé avec de l'eau puis avec 50mL de MeOH à chaud. Le précipité obtenu est filtré, lavé avec de l'eau. Une recristallisation est faite en dissolvant le produit dans du MeOH à chaud et en reprécipitant par addition d'eau. Le précipité est filtré, lavé à l'eau, puis à l'éther pour donner 1,1 g (2,95 mmol, 72 %) de produit **1c** brut.

F 150°C. - IR (KBr) : 3248, 2930, 2857, 2359, 1691, 1669, 1651, 1582, 1528, 1448, 1354, 1310, 1245, 1161 cm-¹. - RMN ¹H (200 MHz, DMSO-d₆) δ = 11,24 (s, 1H, NH-CO) ; 8,33 (d, 1H, *J* = 8,5 Hz, Ar-H) ; 7,97 (d, 2H, *J* = 8 Hz, Ar-H) ; 7,57 (t, 1H, *J* = 7 Hz, Ar-H) ; 7,18 (t, 1H, *J* = 7 Hz, Ar-H) ; 6,44 (s large, 1H, NH-CO-NH) ; 6,35 (s large, 1H, NH-CO-NH) ; 4,30 (m, 1H, CH₂-CH-NH) ; 4,14 (m, 1H, CH-CH-NH) ; 3,12 (m, 1H, CH-S) ; 2,80 et 2,55 (système ABX, 2H, ²*J*_{AB}= 5 Hz, ³*J*_{AX} = 12 Hz, ³*J*_{BX} = 0 Hz, CH₂-S) ; 2,61 (s, 3H, CH₃) ; 2,37 (t, 2H, *J* = 8 Hz, CH₂-CO) ; 1,62-1,38 (m, 6H, (CH₂)₃).

### • Composé ortho-hydrazone 2c :

Le composé **1c** (500 mg, 1,38 mmol) est dissous à chaud dans l'éthanol (99 %, 8 mL) puis l'hydrazine monohydrate (572 µL, 11,1 mmol) est ajoutée. La solution est chauffée à reflux pendant 50 minutes. La solution est évaporée à sec. Le précipité blanc obtenu est filtré, lavé avec de l'eau puis séché à l'éther. On obtient ainsi 416 mg (11,1 mmol, 80 %) de produit **2c** sous forme d'une poudre blanche.

F 161°C. - IR (KBr) : 3412, 3240, 2930, 2857, 2351, 1706, 1677, 1604, 1590, 1531, 1463, 1444, 1372, 1303, 1270, 1169 cm⁻¹.- RMN ¹H (200 MHz, DMSO-d₆) δ =11,97 (s, 1H, NH-CO) ; 8,35 (d, 1H, *J* = 8 Hz, Ar-H) ; 7,45 (d, 1H, *J* = 7 Hz, Ar-H) ; 7,19 (t, 1H, *J* = 7,5 Hz, Ar-H) ; 7,04 (t, 1H, *J* = 7 Hz, Ar-H) ; 6,61 (s, 2H, NH₂) ; 6,42 (s large, 1H, NH-CO-NH) ; 6,35 (s large, 1H, NH-CO-NH) ; 4,32 (m, 1H, CH₂-CH-NH) ; 4,14 (m, 1H, CH-CH-NH) ; 3,12 (m, 1H, CH-S) ; 2,81 et 2,56 (système ABX, 2H, ²*J*_{AB}= 5 Hz, ³*J*_{AX} = 12 Hz, ³*J*_{BX} = 0 Hz, CH₂-S) ; 2,31 (t, 2H, *J* = 8 Hz, CH₂-CO) ; 2,09 (s, 3H, CH₃) ; 1,63-1,36 (m, 6H, (CH₂)₃).

### • Composé ortho-diazométhane 3c :

On solubilise **2c** (200 mg, 0,53 mmol) dans 10 mL de DMF. On ajoute alors 800 mg de MnO₂. Après 15 minutes d'agitation, le mélange est filtré à travers une couche mixte Célite(0,5cm)-tamis moléculaire(0,5cm en poudre). Le mélange réactionnel est évaporé à sec puis lavé à l'éther et séché. Le composé **3c** (130 mg, 65 %) est obtenu sous forme d'une poudre rose.

F 110°C. - IR (KBr) : 3248, 2930, 2857, 2367, 2342, 2038, 1699, 1521,1456 cm⁻¹. - RMN ¹H (200 MHz, DMSO-d₆) δ = 9,37 (s, 1H, NH-CO) ; 7,26-7,00 (m, 4H, Ar-H) ; 6,43 (s large, 1H, NH-CO-NH) ; 6,35 (s large, 1H, NH-CO-NH) ; 4,30 (m, 1H, CH₂-CH-NH) ; 4,15 (m, 1H, CH-CH-NH) ; 3,12 (m, 1H, CH-S) ; 2,82 et 2,54 (système ABX, 2H, ²*J*_{AB}= 5 Hz, ³*J*_{AX} = 12 Hz, ³*J*_{BX} = 0 Hz, CH₂-S) ; 2,24 (t, 2H, *J* = 8 Hz, CH₂-CO) ; 2,12 (s, 3H, CH₃) ; 1,63-1,37 (m, 6H, (CH₂)₃).

### Exemple 1.4 : Synthèse du méta-BioDPDAM :

### • Composé méta-benzophénone 1d :

On solubilise la D-biotine (500 mg, 2,05 mmol) dans 23 mL de DMF anhydre à chaud. On refroidit à 0°C sous argon, puis on ajoute successivement la *N*-méthylmorpholine (295 µL, 2,67 mmol) et le chloroformiate d'isobutyle (420 µL, 3,28 mmol). On laisse sous agitation pendant 30 min, puis on ajoute la 3-aminobenzophénone (605 mg, 3,07 mmol) et la *N-*méthylmorpholine (240 µL, 2,17 mmol) dans 7 mL de DMF. La solution est maintenue sous agitation à 0°C pendant 2 h, puis on évapore à sec. On reprend le résidu dans 1 mL de MeOH, puis on ajoute 25 mL d'eau. Le précipité obtenu est filtré, lavé avec de l'eau, puis avec de l'éther pour donner 810 mg (93 %) de produit **1d** brut. Une recristallisation dans le couple MeOH-H₂O donne **1d**(630 mg, 72 %) sous forme d'une poudre blanche.

RMN ¹H (200MHz, DMSO-d₆) δ =10,10 (s, 1H, NH-CO) ; 8-7,39 (m, 9H, Ar-H) ; 6,43 (s large, 1H, NH-CO-NH) ; 6,35 (s large, 1H, NH-CO-NH) ; 4,27 (m, 1H, CH₂-CH-NH) ; 4,13 (m, 1H, CH-CH-NH) ; 3,12 (m, 1H, CH-S) ; 2,84 et 2,55 (système ABX, 2H, ²*J*_{AB}= 5 Hz, ³*J*_{AX} = 12 Hz, ³*J*_{BX} = 0 Hz, CH₂-S) ; 2, 31 (t, 2H, *J* = 8 Hz, CH₂-CO) ; 1,59-1,36 (m, 6H, (CH₂)₃).

### • Composé méta-hydrazone 2d :

On solubilise **1d** (350 mg, 0,83 mmol) dans 5,5 mL d'éthanol absolu puis on ajoute l'hydrazine monohydrate (140 µL, 2,48 mmol). La solution est chauffée à reflux pendant une nuit. Après évaporation, le produit est repris dans 1 mL d'éthanol et de l'eau. Le précipité blanc est recristallisé : on le dissout dans un minimum d'éthanol à chaud et l'on ajoute de l'eau jusqu'à apparition d'un léger trouble. Après refroidissement, le précipité obtenu est lavé à l'eau puis séché à l'éther. On obtient ainsi 264 mg (73 %) de produit **2d** sous forme d'une poudre blanche.

RMN ¹H (200 MHz, DMSO-d₆) δ = 9,99 (s, 1H, NH-CO) ; 9,80 (s, 2H, NH₂) ; 7,54-6,88 (m, 9H, Ar-H) ; 6,26 (s large, 1H, NH-CO-NH) ; 6,21 (s large, 1H, NH-CO-NH) ; 4,28 (m, 1H, CH₂-CH-NH) ; 4,13 (m, 1H, CH-CH-NH) ; 3,12 (m, 1H, CH-S) ; 2,78 et 2,59 (système ABX, 2H, ²*J*_{AB}= 5 Hz, ³*J*_{AX} = 12 Hz, ³*J*_{BX} = 0 Hz, CH₂-S) ; 2,27 (t, 2H, *J* = 8 Hz, CH₂-CO) ; 1,57-1,36 (m, 6H, (CH₂)₃).

### • Composé méta-diazodiphényl 3d :

On solubilise **3d** (500 mg, 0,53 mmol) dans 1 mL de THF. On ajoute alors 80 mg de MnO₂ activé. Après 5 minutes d'agitation à température ambiante, le mélange est filtré à travers une couche mixte Célite (0,5 cm) -tamis moléculaire (0,5 cm en poudre). Le mélange réactionnel est évaporé à sec. Le composé **3d** (47 mg, 100 %) est obtenu sous forme d'une huile violette.

RMN ¹H (200 MHz, DMSO-d₆) δ = 9,95 (s, 1H, NH-CO) ; 7,60-6,9 (m, 9H, Ar-H) ; 6,42 (s large, 1H, NH-CO-NH) ; 6,35 (s large, 1H, NH-CO-NH) ; 4,28 (m, 1H, CH₂-CH-NH) ; 4,14 (m, 1H, CH-CH-NH) ; 3,12 (m, 1H, CH-S) ; 2,83 et 2,59 (système ABX, 2H, ²*J*_{AB}= 5 Hz, ³*J*_{AX} = 12 Hz, ³*J*_{BX} = 0 Hz, CH₂-S) ; 2,27 (t, 2H, *J* = 8 Hz, CH₂-CO) ; 1,58-1,35 (m, 6H, (CH₂)₃).

### Exemple 2 : Réactif de marquage avec le Cy5 : Cy5-PMDAM :

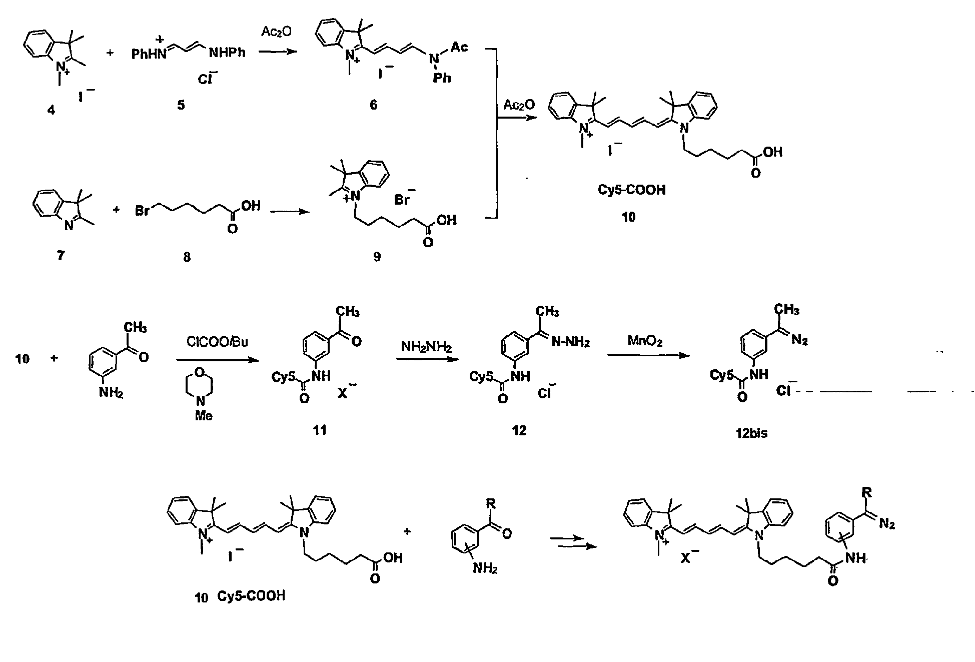

- Composé Iodure de 2-[4-(*N*-acétyl-*N*-phénylamino)buta-1,3-diényl]-1,2,3,3-tétraméthyl[3H] indolium **6** :

Le mélange du monochlorhydrate de malonaldehyde-bis(phénylimine) **5** (18,3 g; 70,0 mmole), de NaOAc (9,0 g ; 110 mmole) et de l'iodure de 1,2,3,3- tétraméthyl[3H]indolium **4** (4,25 g ; 14,1 mmole) dans de l'anhydride acétique (75 mL) est chauffé à 110°C pendant 20 min. Après refroidissement, on ajoute de l'éther (350 mL) et le solide brun précipité est filtré, lavé avec de l'éther (3 × 100 mL). Le solide est redissout dans 150 mL de CH₂Cl₂, filtré (élimination des sels minéraux) puis évaporé pour donner un solide brun (6,0 g, 90 %). RMN ¹H (CDCl₃) : δ = 8,64 (d ; 1H ; *J* = 12 Hz ; 1-H) ; 8,14 (t ; 1H ; *J* = 16 ; 12 Hz ; 3-H) ; 7,63-7,19 (m ; 9H) ; 6,90 (d ; 1H ; *J* = 15 Hz ; 4-H) ; 5,82 (t ; 1H ; *J* = 12 ; 13 Hz ; 2-H) ; 4,06 (s ; 3H ; NCH₃) ; (2,16 (s ; 3H ; -COCH₃) ; 1,74 (s ; 6H ; CH₃).

### • Composé Bromure de 1-(5-carboxypentyl)-2,3,3-triméthyl[3H]indolium 9 :

On mélange le 2,3,3-triméthylindole **7** (10,0 g ; 62,8 mmole) et l'acide 6-bromohexanoïque **8** (12,3 g ; 62,8 mmole) sans solvant et chauffe à 110°C pendant 12 h sous argon. Le mélange réactionnel pâteux rouge-violet est lavé avec de l'acétate d'éthyle (2 × 60 mL, on triture la pâte avec la spatule et on décante le surnageant), puis avec de l'acétone (50 mL, la pâte solidifie). Le solide rose est filtré puis séché sous vide (16,0 g ; 73 %).

### • Composé Cy5COOH 10:

Le mélange de l'iodure **6** (6,0 g ; 12,7 mmole), du bromure **9** (4,5 g ; 12,7 mmole) et de NaOAc (2,6 g ; 32 mmole) dans de l'anhydride acétique (35 mL) est chauffé à 110 °C pendant 20 min. Après refroidissement, on ajoute de l'éther (150 mL) et le précipité est filtré et lavé avec de l'éther (3 × 50 mL). Le solide est dissous dans 100 mL de CH₂Cl₂, filtré et purifié par chromatographie sur colonne de SiO₂ (éluant : MeOH 5-10 %/CH₂Cl₂). On obtient 3,4 g (44 %).

RMN ¹H (CDCl₃) : δ = 8,03 (t ; 2H ; *J* = 10 ; 11 Hz, 2-H, 4-H) ; 7,38-6,91 (m ; 9H ; Ar-H, 3-H) ; 6,41 (d ; 1H ; *J* = 14 Hz ; 1-H) ; 6,31 (d ; 1H ; *J* = 13 Hz ; 5-H) ; 4,07 (t ; 2H, *J* = 7 ; 7 Hz ; α-CH₂) ; 3,68 (s ; 3H ; NCH₃) ; 2,47 (t ; 2H, *J* = 7 ; 7 Hz ; ε-CH₂) ; 1,71 (m ; 18H ; CH₃, β,γ et δ-CH₂).

### • Composé de couplage de la 3-aminoacétophénone avec le Cy5COOH 10 (produit 11) :

À une solution de Cy5COOH **10** (1,19 g; 1,9 mmole) dans 12 mL de CH₂Cl₂, on ajoute *N-*méthylmorpholine (360 µL ; 3,2 mmole). La solution est refroidie avec un bain de glace, puis on ajoute le chloroformiate d'isobutyle (480 µL ; 3,7 mmole). Après 5 minutes d'agitation, on additionne la 3-aminoacétophénone (488 mg ; 3,6 mmole). On agite le mélange à température ambiante pendant 3 h. En ajoutant 250 mL d'éther, on obtient un solide pâteux. Après agitation, on laisse reposer le solide au fond de ballon et on décante le surnageant. On ajoute de nouveau 50 mL d'éther et on triture avec une spatule pour obtenir un solide. Celui-ci est filtré, lavé avec de l'eau, de l'éther, puis séché sous vide. Le produit (iodure) est ensuite dissous dans de l'éthanol, passé sur colonne d'amberlite IRA900 (Cl⁻ ; 15 g). La solution éthanolique recueillie est évaporée à sec puis passée sur colonne de SiO₂. On obtient 0,93 g (77 %) de solide bleu.

### • Composé Cy5-hydrazone 12 :

On ajoute à une solution de l'acétophénone **11** (0,93 g ; 1,46 mmole) dans 5 mL d'éthanol absolu l'hydrazine monohydraté (180 µL ; 3,1 mmole) que l'on agite à température ambiante pendant 7 h. On ajoute 50 mL d'éther et le précipité est filtré et lavé avec de l'éther. On dissout le produit brut dans 50 mL de CH₂Cl₂, la solution est filtrée puis concentrée à 10 mL. Le produit est précipité par ajout de 100 mL d'éther, filtré, lavé à l'éther et séché sous vide. On obtient 540 mg de produit 12 (57 %).

### • Composé Cy5PMDAM 12 bis:

A une solution de 100 mg d'hydrazone **12** dans 2 mL de DMF, on ajoute 300 mg de MnO₂ et l'on agite vigoureusement pendant 10 min. On filtre la suspension à travers une couche de célite et lave avec DMF (3x500 µL). On ajoute 50 mL d'éther et l'huile déposée est triturée avec une spatule et le surnageant est décanté. On répète l'opération de lavage trois fois avec 25 mL d'éther et le solide ainsi obtenu est filtré, séché. On obtient 65 mg (65 %) de produit **12** bis. La pureté du produit est d'environ 80-85 % (RMN ¹H).

### Exemple 3 : Autres réactifs synthétisés :

### Exemple 3.1 : Synthèse de para-nitrophényldiazométhane (NPDAM) :

Le 4-nitrobenzaldéhyde hydrazone est commercialement disponible (référence 28,118-2, Aldrich, France).

On travaille donc sur 600 mg (3,64 mmole) de ce produit que l'on dissout dans 9 mL de THF. La solution est laissée sous agitation pendant 5 minutes puis 1,26 g (4 équivalent, 14,56 mmole) de MnO₂ sont additionnés avec précaution. Le mélange est laissé sous agitation pendant 10 minutes, puis filtré. Le filtrat récupéré est évaporé à sec. Après lavage au pentane, le composé *para*-nitrophényldiazométhane est obtenu sous forme d'une poudre orange vif avec un rendement de 79 % (468 mg, 2,87 mmole).

F 80-82°C. - RMN ¹H (300 MHz, DMSO-d₆) δ = 8,11 (d, 2H, *J* = 9 Hz, Ar-H₃) ; 7,18 (d, 2H, *J* = 9 Hz, Ar-H₂); 6,06 (s, 1H, CH₁-N₂).

### Exemple 3.2 : Synthèse du phénylméthyldiazométhane (PMDAM) :

Hydrazone de l'acétophénone : On dilue l'acétophénone (2,0 g, 16 mmole) dans 16 mL d'éthanol absolu puis on ajoute l'hydrazine (2,3 mL, 48 mmole). On porte au reflux. Après 2 h, on évapore le solvant et on reprend le résidu dans de l'éther (150 mL). On lave avec de l'eau (100mL). Après séchage sur Na₂SO₄, on évapore l'éther. On obtient l'huile jaune pâle (1,5 g, 11 mmole, 69 %).

Phénylméthyldiazométhane (PMDAM) : On dissout l'hydrazone (150 mg, 1,1 mmole) dans 3 mL de THF. On ajoute le MnO₂ (480 mg, 5,5 mmole). On agite pendant 30 min à température ambiante. Le milieu se colore en rouge. On filtre et on évapore le solvant. On obtient l'huile rouge (145 mg, 100 %). Ce réactif est utilisé sans purification.

### Exemple 3.3 : Synthèse du diphényldiazométhane (DPDAM) :

La Benzophénone hydrazone est commerciale (référence B 960-2 Aldrich, France).

On travaille donc sur 196 mg (1,0 mmole) que l'on dissout dans 5 mL de THF. 435 mg (5 éq, 5,0 mmole) de MnO₂ sont additionnés, le mélange est laissé sous agitation pendant 10 minutes, puis filtré. Le filtrat récupéré est évaporé à sec. On obtient 193 mg (0,99 mmole). Ce réactif est utilisé sans purification.

### Exemple 3.4 : Synthèse du NVDAM :

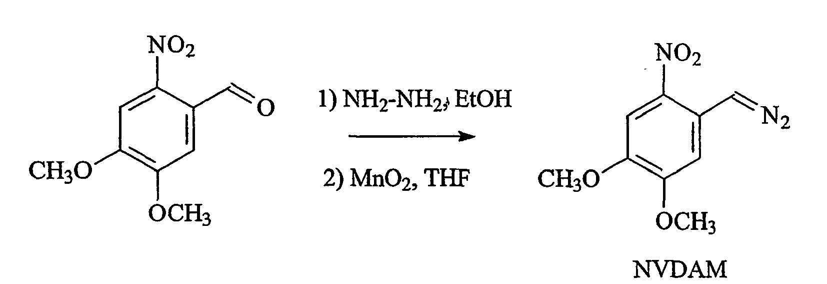

La synthèse est réalisée selon le protocole décrit précédemment à partir de 6-nitrovératraldéhyde (Aldrich, référence 27,960-9).

### Exemple 4 : Synthèse du dérivé biotinylé à partir du NPDAM :

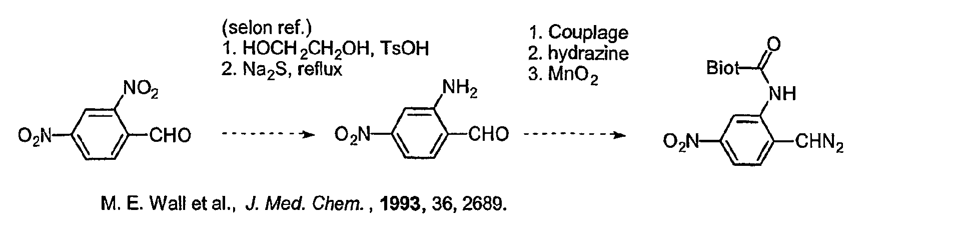

Le dérivé 2-amino-4-nitro benzaldéhyde est préparé selon la méthode de ME Wall et al référencé ci-dessus.

La préparation du diazométhane NPDAM est identique à celle décrite dans l'exemple 3.1 ci-dessus.

### Exemple 5 : Préparation des acides nucléiques ADN et ARN :

### Exemple 5.1 : Préparation des amplicons ADN :

Les amplicons ADN sont générés par PCR à partir de cibles d'ADN génomique *Mycobacterium tuberculosis* 16S (10⁺⁴ copies comme cibles de départ) en utilisant le kit Fast Start de Roche, 0,2 mM de chaque désoxyribonucléotide (d-ATP, d-CTP, d-GTP, d-TTP), 0,3 µM d'amorces et 0,4 µL d'enzyme.

Les paramètres de la PCR sont les suivants :
- 95°C : 4min puis 35 cycles (95°C : 30 sec ; 55°C : 30 sec ; 72°C : 30 sec) puis 4°C.

Les amplicons sont analysés qualitativement par électrophorèse sur gel d'agarose (1,5%, TBE 0,5X). Le volume déposé est de 5 µL et la migration s'effectue durant 20min à 100 Volts (V). La visualisation des produits PCR est réalisée sous lampe UV après coloration au bromure d'éthidium.

Les conditions pour la culture, l'extraction des Mycobactéries ainsi que les amorces d'amplification sont données dans la demande de brevet WO-A-99/65926.

### Exemple 5.2 : Préparation des ARN transcrits :

Les transcriptions sont réalisées à partir de cible PCR (fragment de l'ARN 16S de *Mycobacterium tuberculosis*) en utilisant le kit MEGAscript d'Ambion : 7,5 mM de chaque nucléotide (ATP, CTP, GTP et UTP) et 2 µL d'enzyme (ARN polymérase). Le temps d'incubation est de 3 heures (h) à 37 °C. Les amorces d'amplification de la PCR portent un promoteur de polymérase T3 ou T7, comme décrit dans la demande WO-A-99/65926 ou dans l'article J. Clin Microbiol. 37(1), p 49-55, 1999, ce qui permet de réaliser la transcription.

Les transcrits sont analysés par électrophorèse sur gel d'agarose (1,5%; TBE 0,5X). Le volume déposé est de 5 µL et la migration s'effectue durant 20min à 100V. La visualisation des transcrits est réalisée sous lampe UV après coloration au bromure d'éthidium.

Des résultats identiques du point de vue de l'invention peuvent être obtenus en utilisant d'autres techniques d'amplification comme la NASBA ou TMA, qui génèrent directement des amplicons ARN.

### Exemple 6 : Réactivité des réactifs de marquage sur des nucléotides modèles :

La synthèse des réactifs de marquage est décrite dans les exemples 1 à 4. Le PDAM décrit dans la présente invention est commercialement disponible (référence P1405, Molecular Probes, Eugene, OR).

### Exemple 6.1 : Marquage des monomères UMP 3'-phosphate :

La réactivité des réactifs de marquage portant une fonction diazométhyle a été étudiée pour contrôler la spécificité de la réaction.

Un protocole a été mis au point qui consiste à étudier cette réactivité par électrophorèse capillaire sur un composé modèle le 3'-UMP (Uridine 3'monophosphate, référence U1126 Sigma) dans les conditions standards suivantes :
3'-UMP 0,04 mM ; H₃BO₃ 2,0 mM ; Marqueur 2,0 mM [ajouté avec un solvant organique approprié (THF, AcOEt ou DMSO)] ; Solvants H₂O - CH₃CN -Solvant organique (rapport : 1/3/1).

On répartit cette solution en dix fractions de 250 µL que l'on chauffe à 60 °C. Après une durée définie, chaque fraction est traitée avec 250 µL de dichlorométhane. Après agitation, on élimine la phase organique (phase inférieure). On répète cette opération encore deux fois. Après centrifugation (5 min, 5000 tours par minutes (rpm)), la phase aqueuse est analysée par électrophorèse capillaire. Les conditions de l'électrophorèse capillaire (EC) sont les suivantes : l'analyse EC a été effectuée par l'appareil Beckman P/ACE 5000. Un capillaire de silice fondue non traité (75 µm × 50 cm) a été utilisé. Le voltage appliqué est de 30 kV (polarité normale) et la température du capillaire a été maintenue à 23°C. Les électrophorégrammes ont été enregistrés à 254 nm. La solution tampon borate (0,1 M, pH 8,3) a été préparée à partir de l'acide borique en ajustant le pH avec une solution NaOH et filtrée à travers un filtre 0,2 µm. Les échantillons ont été injectés sous pression (0,5 psi, 5 secondes). Chaque analyse est précédée de la régénération du capillaire par passages successifs d'une solution de NaOH (0,1 M, 2 min), eau (2 min) et de tampon borate (2 min) sous pression (20 psi).

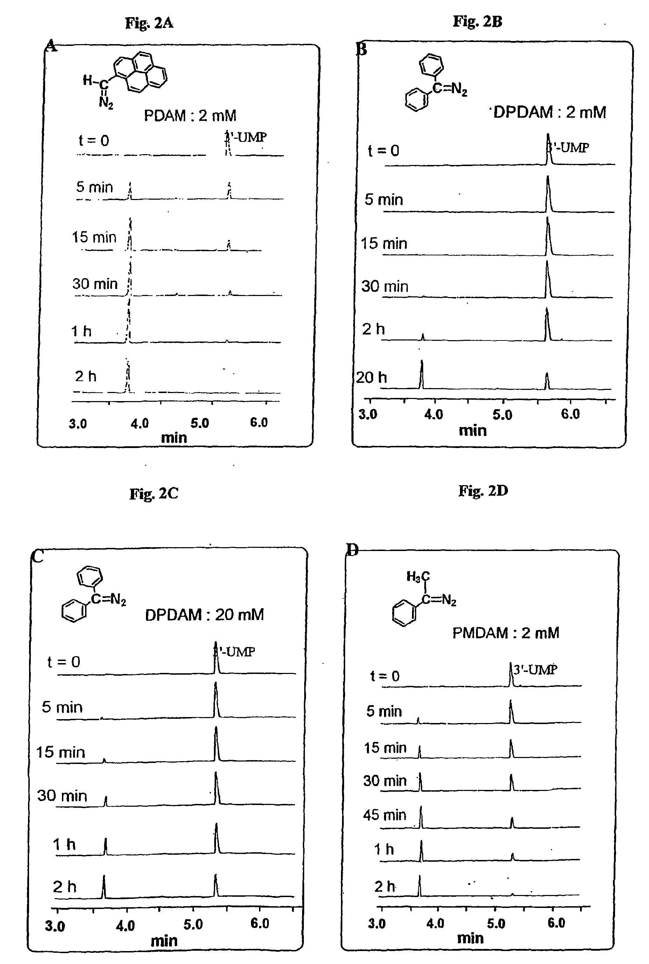

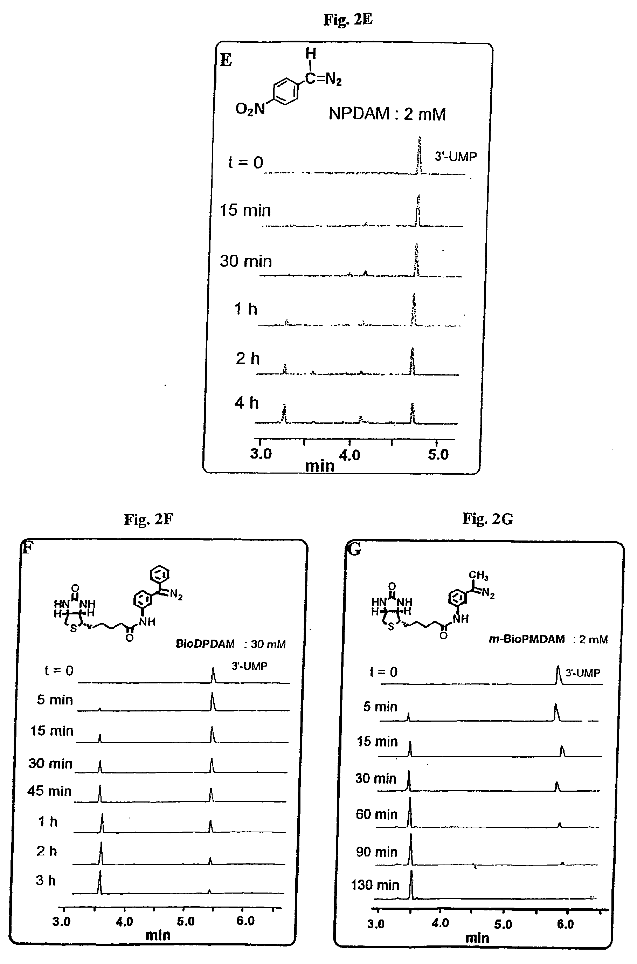

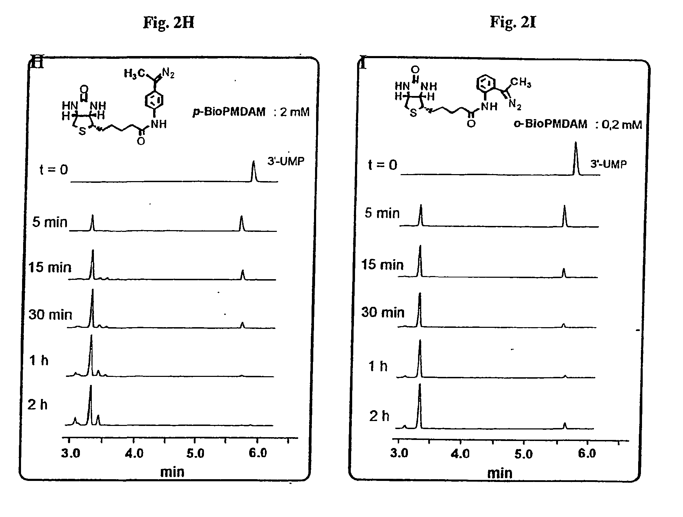

La réaction est effectuée en faisant varier le temps de réaction entre 0 et 4 heures, comme indiqué sur chaque figure, et les résultats sont présentés pour chaque réactif testé sur les figures 2A à 2I avec la concentration en réactif utilisé.

Le temps de réaction est indiqué sur chaque électrophorégramme.

Avec tous les réactifs testés, on obtient la formation exclusive d'un produit monoalkylé, ce qui prouve la spécificité de la réaction.

La réactivité, c'est-à-dire le temps de demi réaction du réactif sur le 3'-UMP, peut être ainsi calculée, par comparaison de la hauteur des pics, et les résultats sont présentés dans le tableau 1 ci-dessous (conditions standards décrites ci-dessus) :

**Tableau 1 : Réactivité (temps de demi réaction) de réactifs**

| | | | | |
|---|---|---|---|---|
| Formules chimiques et noms | 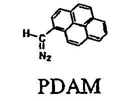 | 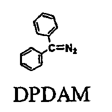 | 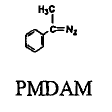 | 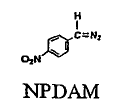 |
| Réactivité | 5 min | 20 h | 30 min | 4 h |
| | | | | |
| Formules chimiques et noms | 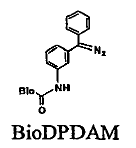 | 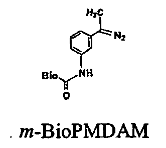 | 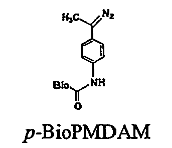 | 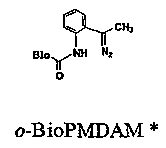 |
| Réactivité | 10 h | 15 min | 5 min | 1 min |

| | | | | |
|---|---|---|---|---|
| * Avec le *o*-BioPMDAM, la réactivité est estimée car dans les conditions de concentration de 2 mM en réactif de marquage, la réaction est complète en moins de 5 min. La figure 2I utilise donc une concentration de 0,2 mM. | | | | |

On peut néanmoins noter que, la réaction étant très spécifique et ne conduisant pas à des sous produits pour tous les réactifs testés, il est possible d'augmenter la concentration du réactif de marquage sans conséquence du point de vue de la sélectivité sur le marquage.

Ainsi pour le réactif DPDAM, si l'on augmente la concentration à 20 mM (voir figure 2C), la réactivité (temps de demi réaction) est de 2 heures. Le même résultat est obtenu avec le BioDPDAM (figure 2F) où la réactivité est de 45 min à une concentration de 30 mM.

### Exemple 6.2 : Test de différents nucléotides 3'-monophosphate :

De manière à éviter toute erreur d'interprétation, une étude complémentaire sur le marqueur *méta*-BioPMDAM, pris à titre d'exemple significatif, a été effectuée avec les autres nucléotides 3'-monophosphate.

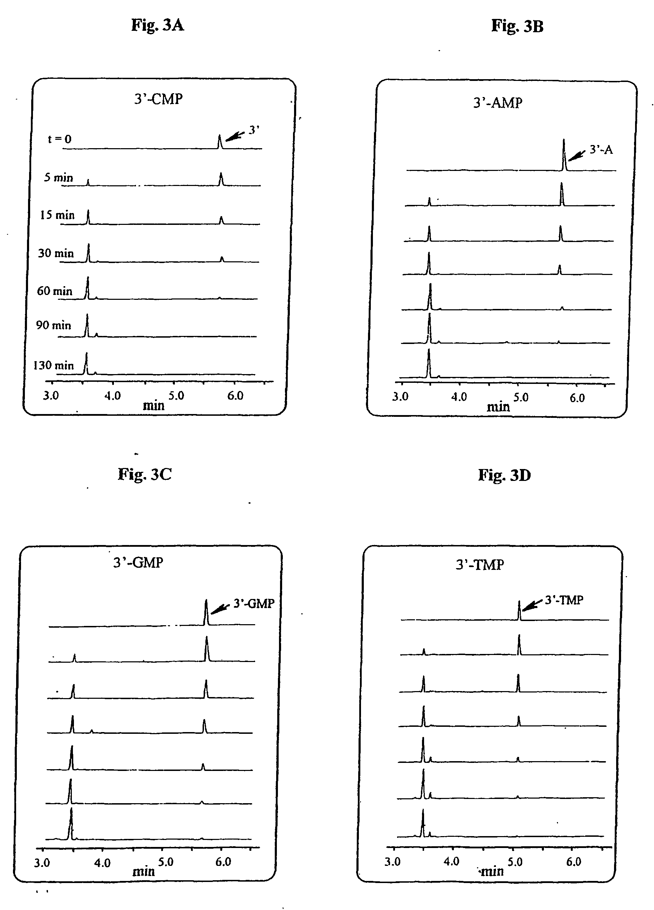

Les nucléotides testés sont les suivants : 3'-AMP (référence 85,194-9, Aldrich), 3'-GMP (référence 151214, ICN), 3'-CMP (référence C1133, Sigma), 3'-TMP (série désoxyribo) (référence T1008, Sigma). Les électrophorégrammes obtenus avec les différents nucléotides sont représentés sur les figures 3A à 3D. Les temps de réaction indiqués sur la figure 3A sont identiques pour les figures 3B à 3D.

Quel que soit le nucléotide de départ (série ribo ou désoxyribo), nous observons la formation exclusive et complète du produit alkylé en 130 min à 60°C. Il est important de noter que dans le cas de la guanine (base la plus réactive avec les réactifs alkylants usuels), seul le produit alkylé au phosphate est observé prouvant la très grande sélectivité de la réaction.

Cette étude permet également de vérifier que la vitesse de la réaction ne dépend pas de la nature de nucléotide en tant que substrat.

### Exemple 6.3 : Etude d'un dinucléotide :

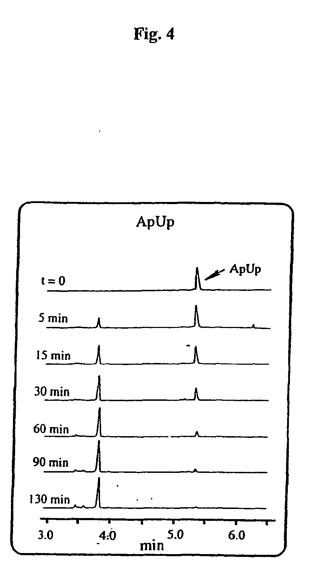

L'alkylation du dinucléotide ApUp (référence A4298, Sigma) a été effectuée avec le *méta-*BioPMDAM afin de vérifier la sélectivité de la réaction vis-à-vis du phosphate terminal par rapport au phosphate internucléotidique. Le suivi de la réaction par électrophorèse est représenté sur la figure 4. Les conditions sont les conditions standards de l'exemple 6.1 déjà décrit.

La formation exclusive d'un seul produit est observée montrant une bonne sélectivité du réactif *méta*-BioPMDAM vis-à-vis du phosphate terminal par rapport au phosphate internucléotidique.

### Exmple 6.4 : Caractérisation des adduits avec les 4 nucléotides 3'-monophosphates :

Pour s'assurer que les produits obtenus provenaient bien d'une alkylation au phosphate, la synthèse des adduits des monophosphates 3'-UMP, 3'-CMP, 3'-GMP et 3'-AMP avec le réactif *méta*-BioPMDAM a été effectuée. La réaction d'alkylation est réalisée à l'échelle préparative comme indiqué ci-dessous. Les adduits, obtenus avec des rendements de l'ordre de 70 % sont purifiés puis étudiés par RMN du proton et du phosphore.

### Protocole de préparation :

On dissout le 3'-UMP (sous forme de sel disodique ; 9,3 mg ; 21,1 µmol) dans 2 mL d'une solution aqueuse 0,1 M de H₃BO₃, puis on ajoute successivement 2 mL de CH₃CN, 6 mL de MeOH puis le réactif *méta*-BioPMDAM (75 mg ; 0,20 mmol). La réaction est réalisée pendant 2,5 h à température ambiante. Elle est suivie par électrophorèse capillaire. On ajoute 3 mL d'eau, puis l'excès du réactif est éliminé par extraction avec CH₂Cl₂. La phase aqueuse est évaporée. Le résidu est dissous dans une petite quantité d'eau et purifié par passage sur une colonne de gel de silice en phase inverse (Lichroprep RP-18, Merck ; élution MeOH/H₂O (20/80)). On obtient 10 mg (69 %) de l'adduit du 3'-UMP.

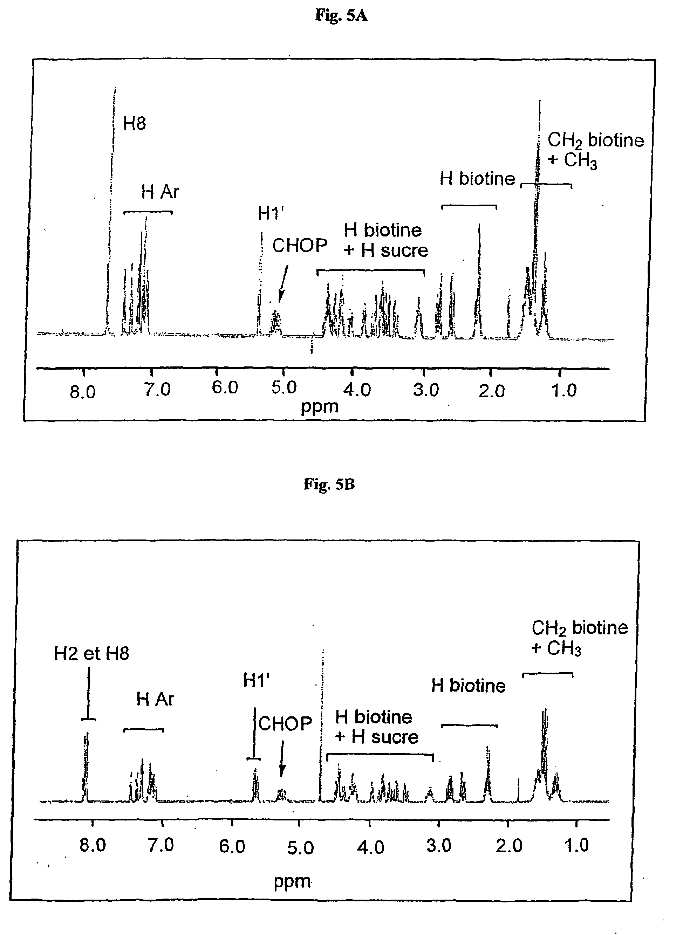

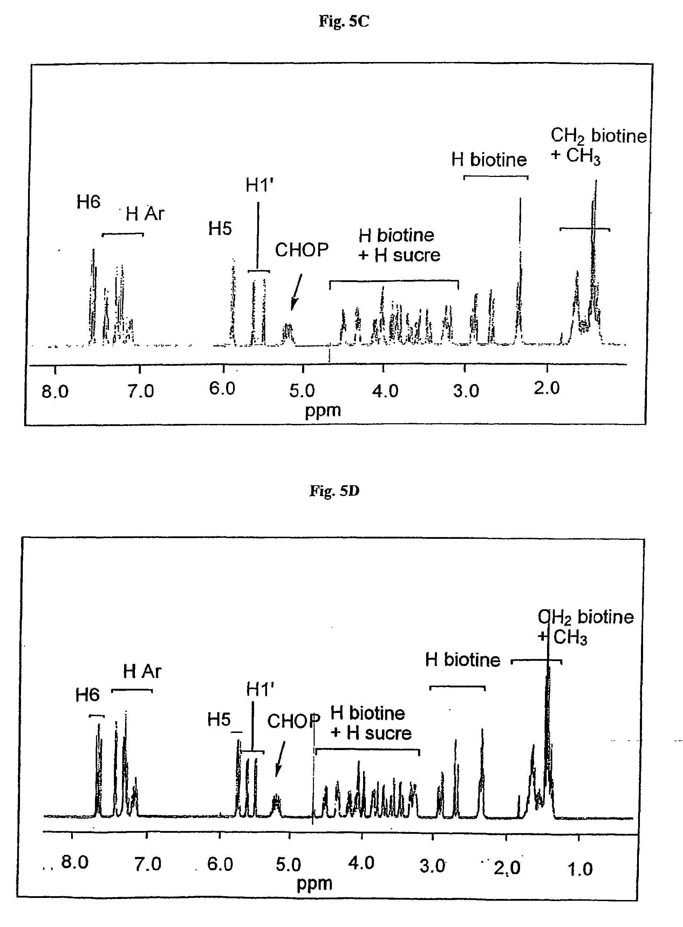

Les spectres de RMN du proton obtenus pour les adduits du 3'-NMP (N= G, U, C, A) sont présentés sur les figures 5A à 5D. L'identification des adduits a été réalisé par des expériences de RMN à deux dimensions ¹H/¹H (COSY). Deux diastéréoisomères pour chacun de ces adduits dans un rapport 1/1 sont présents.

Un seul pic est présent pour la RMN du phosphore vers 0 ppm (300 MHz, D2O).

Ces expériences démontrent que la réaction est bien spécifique, qu'un seul adduit est observé et que le marquage a bien lieu sur le phosphore. Il n'y a pas de réaction secondaire d'alkylation sur les bases. Les produits de marquage sont donc particulièrement adaptés pour une étape d'hybridation.

### Exemple 7 : Etude de stabilité:

### Exemple 7.1 : Stabilité des réactifs de marquage à la température :

Tous les dérivés du diazométhane, décrit dans le tableau de l'exemple 6.1 ci-dessus, sont conservés à l'état solide dans le congélateur à -20 °C pendant au moins trois (3) mois et aucune perte de réactivité n'est observée.

La stabilité à température ambiante sur la paillasse a été déterminée par RMN ¹H pour les deux réactifs NPDAM et *méta*-BioPMDAM. Nous n'avons observé aucune décomposition en laissant le NPDAM pendant un mois sur la paillasse sans précaution particulière. Nous observons environ 50% de décomposition en laissant le *méta*-BioPMDAM sur la paillasse pendant vingt-cinq (25) jours.

La stabilité à la température du réactif de marquage est une caractéristique essentielle. En effet, la destination finale d'un tel réactif pour une application industrielle est un kit de marquage. Un réactif qui n'est pas stable au moins pendant quinze (15) jours à -20°C, de préférence un (1) mois est invendable. Même s'il existe des moyens de stockage et d'envoi jusqu'à -110°C, il existe une relation entre la stabilité à -110°C et à -20°C et c'est pourquoi la valeur de quinze (15) jours à -20°C, de préférence un (1) mois à -20°C est un minimum industriel. Au delà de -110°C, les laboratoires ne disposent pas des équipements nécessaires (congélateur) pour stocker ces réactifs du point de vue de l'utilisateur et du fabricant, et il n'y a pas de moyen simple type carboglace pour les expédier du point de vue du fabricant.

En ce qui concerne la stabilité à température ambiante, une stabilité de quelques heures, de préférence de un (1) jour, est suffisante pour permettre à l'utilisateur d'effectuer le marquage.

### Exemple 7.2 : Hydrolyse de différents conjugués Nucléotide 3'-UMP-marqueurs :

Nous avons testé la stabilité des conjugués [3'-UMP-marqueur] en milieu basique. Le mécanisme d'hydrolyse peut être représenté de la manière suivante :

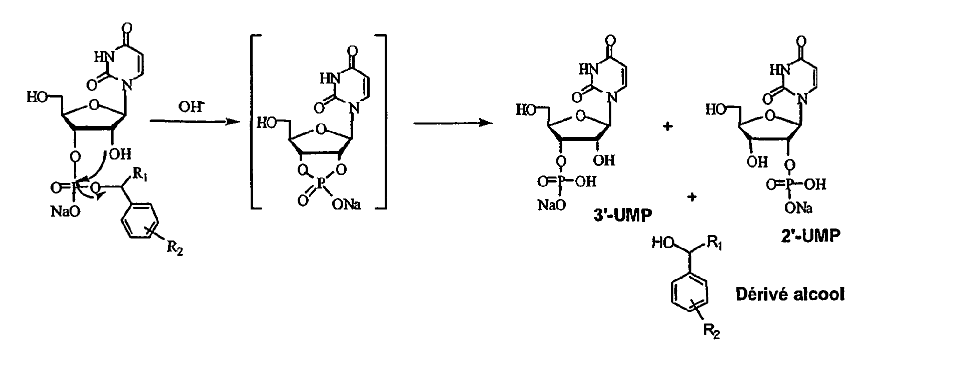

Les différents conjugués utilisés ont été synthétisés selon le protocole suivi pour l'alkylation du monophosphate 3'-UMP (série ribonucléotide) par le *méta*-BioPMDAM.

Les différents réactifs de marquage testés avec le 3'UMP sont :
- *méta*-BioPMDAM,
- *ortho*-BioPMDAM,
- *para*-BioPMDAM,
- DPDAM, et
- PDAM.

### Mode Opératoire

On prépare une solution à 20 mM du réactif de marquage dans le DMSO.

Pour la réaction, on mélange :
- 20 µL de cette solution,
- 40 µL de NaOH 1N,
- 10 µL de produit de référence à 40 mM, et
- 130 µL de H₂O.

Cette solution est chauffée à 60°C et des prélèvements à temps précis sont effectués.

Pour les prélèvements, on opère de la façon suivante :
- 10 µL de la solution chauffée,
- 40 µL de H₃BO₃ 0,5M, et
- 150 µL de H₂O.

On injecte alors 15 µL de cette solution finale par électrophorèse capillaire, ceci pour chaque prélèvement.

La vitesse de la réaction d'hydrolyse a été déterminée par électrophorèse capillaire en étudiant la diminution de la quantité de conjugué en fonction du temps (mesure de la surface du pic comparée à celle d'un étalon interne : acide 3,5-diaminobenzoïque ou acide naphtoïque).

De cette façon, nous avons pu comparer la stabilité des différents conjugués.

Les résultats sont présentés dans le tableau 2 ci-dessous :

**Tableau 2 : Réactivité (temps de demi réaction) de réactifs conjugués à des nucléotides 3'-UMP-marqueurs**

| Réactif de marquage couplé au 3'UMP | Temps de demi vie du conjugué (min) |
|---|---|
| *méta*-BioPMDAM. | 197 |
| *ortho*-BioPMDAM. | 30 |
| *para*-BioPMDAM. | 8 |
| DPDAM | 54 |
| PDAM | 16 |

Ainsi, nous avons trouvé que le conjugué avec le *méta*-BioPMDAM présentait un temps de demi-vie de 197min, soit un temps vingt-cinq (25) fois supérieur à celui du dérivé *para-*BioPMDAM, sept (7) fois supérieur à celui du dérivé DPDAM et quatre (4) fois supérieur à celui du dérivé *ortho*-BioPMDAM.

Les dérivés *ortho* et *méta* permettent de mieux stabiliser le conjugué ce qui permet une meilleure détection dans le cas d'un test diagnostic.

### Exemple 8 : Marquage des ARN transcrits par le dérivé méta-bioPMDAM (3a) :

### Marquage :

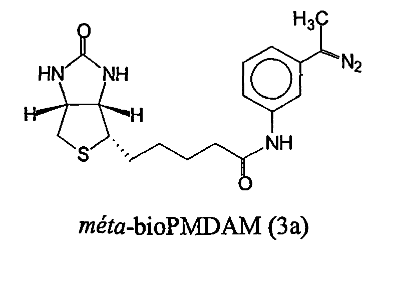

Le dérivé *méta*-bioPMDAM (3a) a été obtenu selon le schéma réactionnel décrit dans l'exemple 1.1. Les cibles ARN ont été préparées par transcription post-PCR selon la procédure décrite dans l'exemple 5.2.

A 50 µL d'une solution contenant des ARN transcrits, 9 µL d'eau pure (Sigma), 9 µL d'imidazole à 0,1M et 9 µL de MnCl₂ à 1M ont été ajoutés. La concentration de l'imidazole et du MnCl₂ est de 6mM et 60mM respectivement. Avant l'incubation, de la solution contenant la cible ARN et le tampon nécessaire pour la fragmentation, 3 µL de *méta*-bioPMDAM (3a) à 100mM dans le DMSO ont été ajoutés. La concentration finale en marqueur *méta-*bioPMDAM est de 2 mM. Après avoir complété la solution à 150 µL avec de l'eau pure, le mélange réactionnel est homogénéisé est incubé 30min à 60°C.

Après 30min d'incubation et addition de l'EDTA (100mM finale), l'excès de marqueur est éliminé par purification sur colonnes QIAVAC™ (Qiagen, Hilden, Allemagne) en utilisant les tampons et le protocole du fournisseur.

### Hybridation :

Après l'étape de marquage pendant la fragmentation, les fragments obtenus sont hybridés sur les puces à ADN conçues pour l'analyse de la région 213-415 de la séquence M20940 « Genbank » de l'ARN 16S de *Mycobacterium tuberculosis.* Cette puce à ADN est décrite dans A. Troesch et al, J. Clin Microbiol., 37(1), p49-55, 1999.

Les étapes d'hybridation ont été réalisées sur les stations fluidiques (Affymetrix, santa Clara, CA) en utilisant le protocole d'hybridation et les tampons décrits dans A. Troesch et al, J. Clin Microbiol., 37(1), p49-55, 1999. Une étape supplémentaire est nécessaire pour révéler la biotine (détection indirecte).

Les fragments biotinylés hybridés sur les sondes de capture à la surface de la puce à ADN sont révélés par l'introduction d'une streptavidine marquée par la phycoérythrine (excitation : 488 nanomètres (nm)) et par un Cy5 (excitation : 650 nm) en utilisant les conditions suivantes :300 µL d'eau pure ; 300 µL de tampon Tris 100 mM pH 7 / NaCL 1M / tween 0,05% / Antimousse 0,005% ; 6 µL de BSA (50 mg/mL) ; 6 µL de streptavidine marquée (200 µg / mL). Les références des constituants utilisés sont :
- Streptavidine-Phycoérythrine : référence R0438, Dako, Danemark.
- Streptavidine-CY5 : référence C0050 Dako Danemark.
- Antimousse référence M5-575, Ultra Additives Inc.
- Tween référence P-7949, Sigma.

### Lecture de la puce à ADN :

La lecture de la fluorescence émise à la surface de la puce à ADN, après marquage et hybridation, ainsi que la génération des données en termes d'intensité du signal et du pourcentage d'homologie sont réalisés par les systèmes de lecture et le logiciel fournis par Affymetrix (GeneChip® Instrument System et GeneChip® Information System, Santa Clara CA).

Le système de lecture fournit des intensités en signal et bruit de fond exprimées en rfu (« relative fluorescence unit »). Le pourcentage d'homologie est donné par rapport à une séquence référence qui, dans ce cas, est la séquence de *Mycobacterium tuberculosis.*

Les résultats en termes d'intensité moyenne du signal (I), du bruit de fond (B) et du pourcentage d'homologie (%) sont donnés dans le tableau 3 ci-dessous.

De manière générale, on considère qu'un pourcentage d'homologie supérieur à 90% est un résultat satisfaisant, bien que l'on cherche en général un résultat supérieur à 95%. Au-dessus de 95%, les valeurs ne sont plus indiquées car elles ne sont pas significatives dans le cas de la puce à ADN Mycobactéries. Une intensité élevée avec un bruit de fond faible est le deuxième résultat recherché dans les exemples qui suivent. Dans tous les résultats, le bruit de fond B est déduit de l'intensité moyenne I.

**Tableau 3 : Lecture de la fluorescence émise à la surface de la puce à ADN après marquage et hybridation**

| **Marqueur** | **Homologie** | **I (rfu)** | **B (rfu)** | **I/B** |
|---|---|---|---|---|
| Phycoérythrine | >95% | 2358 | 397 | 6,9 |
| Cy5 | >95% | 4688 | 72 | 66,3 |

Avec les deux marqueurs, le pourcentage d'homologie est supérieur à 95 %. Les intensités des signaux en rfu sont supérieures à celles obtenues par les techniques de marquage standards. Ces résultats montrent que la réaction de marquage pendant la fragmentation des ARN transcrits utilisant le dérivé *méta*-bioPMDAM permet de générer suffisamment de fragments marqués et détectables sur la puce à ADN. La fonction diazométhyle permet donc l'introduction du marqueur biotine dans la séquence d'ARN transcrit. Cette chimie ne modifie pas les propriétés d'interaction biotine-streptavidine. En effet, que ce soit avec le fluorophore phycoérythrine ou le Cy5, les niveaux d'intensités ainsi que le rapport signal sur bruit de fond (I/B) sont bons. Cette technique permet d'utiliser un fluorophore différent en fonction des applications voire de détecter à deux longueurs d'onde différentes (voir par exemple Nature Genetics volume 14, pages 441-447, 1996).

### Exemple 9 : Optimisation des conditions de marquage par le dérivé méta-bioPMDAM :

### Exemple 9.1 : Optimisation de la concentration en sel de fragmentation (MnCl₂) :

Le marquage des ARN transcrits a été réalisé selon le protocole décrit dans l'exemple 8 en utilisant différentes concentrations en MnCl₂.

L'hybridation et la lecture ont également été réalisées selon les procédures décrites dans l'exemple 8.

Les résultats de marquage montrent que les intensités élevées sont obtenues avec des concentrations faibles en MnCl₂.

| | | |
|---|---|---|
| MnCl₂ : 0 mM, | Intensité I : 3857 rfu, | Homologie : 93,5%, |
| MnCl₂ : 5 mM, | Intensité I : 3031 rfu, | Homologie : 93,5%, et |
| MnCl₂ : 10mM | Intensité I : 2471 rfu, | Homologie : 94,1%. |

L'analyse de la fragmentation des transcrits ARN à différentes concentration en MnCl₂ montrent que la fragmentation est toujours efficace à 5 mM en métal et en incubant à 60°C. Ceci montre que de très bons résultats de marquage peuvent être obtenus en utilisant les réactifs de marquage dans les conditions optimales de marquage et de fragmentation.

### Exemple 9.2 : Optimisation de la concentration en imidazole :

Quatre (4) tubes contenant 50 µL d'une solution d'ARN transcrits (Mycobactéries 16S) sont mis en présence de 4,5 µL ou 18 µL ou 36 µL d'imidazole (1 M dans l'eau pure) respectivement de manière à obtenir 30, 120 et 240 mM en concentration finale. Trois (3) µL de *méta*-bioPMDAM (100 mM dans le DMSO) sont ensuite ajoutés et le volume total est ajusté à 150 µL avec de l'eau pure.

Les solutions dans les quatre tubes sont mélangées au vortex et mis à incuber 10 minutes à 60°C.

### Purification

L'élimination de l'excès de marqueur a été effectuée par purification des quatre solutions de réactions de marquage sur des colonnes QIAVAC™.

Les étapes d'hybridation et de lecture sont celles de l'exemple 8 (détection streptavidine phycoérythrine).

Les résultats en termes d'intensité du signal sont les suivants :

| | |
|---|---|
| Imidazole 30 mM | intensité 3600 rfu, |
| Imidazole 120 mM | intensité 1600 rfu, et |
| Imidazole 240 mM | intensité 1400 rfu. |

Le meilleur résultat en terme d'intensité de marquage et obtenu avec 30 mM en imidazole. Il est probable que les concentrations élevées (120 et 240 mM) engendrent des fragmentations excessives et par conséquence, génèrent des fragments plus courts.

Ceci montre aussi que la chimie de marquage basée sur la fonction diazométhyle peut être optimisée et adaptée aux conditions de marquage et à la cible à marquer.

### Exemple 9.3 : Effet de la concentration en marqueur méta-bioPMDAM :

Six (6) tubes contenant 50 µL d'une solution d'ARN transcrits (exemple 5.2) sont mis en présence de 4,5 µL d'imidazole (1 M dans de l'eau pure) et de respectivement un volume de 0,38 ; 0,75 ; 1,5 ; 3 ; 4,5 et 7,5 µL de *méta*-bioPMDAM (100 mM dans du DMSO). Le volume total est ajusté à 150 µL avec de l'eau pure.

Les solutions sont mélangées par vortex et mises à incuber 10 minutes à 60°C.

Les conditions de purification, d'hybridation et de lecture sont conformes à l'exemple 8.

Les résultats sont donnés dans le tableau 4 ci-dessous :

**Tableau 4 : Tableau récapitulatif des résultats de marquage en fonction des concentrations en marqueur méta-bioPMDAM. Révélation sur puce ADN avec de la streptavidine-phycoérythrine (PE) et la streptavidine-Cy5 (Cy5).**

| **Concentration en *méta*-bioPMDAM** | **Marqueur** | **Homologie (%)** | **I(rfu)** | **B(rfu)** | **I/B** |
|---|---|---|---|---|---|
| 0,25mM | PE | 72,4 | 193 | 786 | 0,2 |
| | Cy5 | 60,5 | 3263 | 487 | 6,7 |
| 0,5mM | PE | 88,6 | 454 | 785 | 0,6 |
| | Cy5 | 77,3 | 7925 | 589 | 13,5 |
| 1,0mM | PE | 91,4 | 891 | 809 | 1,1 |
| | Cy5 | 84,3 | 11194 | 662 | 16,9 |
| 2,0mM | PE | 94,6 | 2361 | 787 | 3,0 |
| | Cy5 | 91,4 | 15375 | 417 | 36,8 |
| 3,0mM | PE | 95,1 | 3944 | 797 | 5,0 |
| | Cy5 | 93,5 | 23248 | 222 | 104,8 |
| 5,0mM | PE | 93,5 | 6112 | 820 | 7,5 |
| | Cy5 | 92,4 | 34794 | 479 | 72,6 |

A partir de 2 mM de *méta*-bioPMDAM, les pourcentages d'homologies et les intensités de marquage sont bons. Ceci est vrai dans le cas de la révélation de la biotine avec la streptavidine portant la phycoérythrine et aussi dans le cas de la streptavidine liée au Cy5. Au delà de cette concentration, le signal est augmenté et le pourcentage d'homologie n'est pas affecté.

### Exemple 9.4 : Suppression de l'étape de purification post-marquage :

Dans le but de s'affranchir de l'étape de purification, différents volumes de la réaction de marquage sont hybridés sur puce à ADN sans purification préalable.

A 50 µL d'une solution d'ARN transcrits (exemple 5.2), le tampon de marquage pendant la fragmentation est ajouté, le mélange est ensuite incubé 30min à 60°C. Les conditions de marquage pendant la fragmentation sont : 30 mM imidazole (4,5 µL d'une solution d'imidazole à 1 M dans l'eau pure), 10 mM MnCl₂ (1,5 µL d'une solution de chlorure de manganèse à 1 M dans l'eau pure) et 2 mM de *méta*-BioPMDAM (3 µL d'une solution 100 mM dans le DMSO anhydre).

Après incubation, différents volumes de la solution de marquage ont été hybridés sur la puce à ADN sans aucune purification préalable. Le protocole d'hybridation utilisé est décrit ci-dessus. Les fragments biotinylés hybridés sur les sondes de capture à la surface de la puce à ADN sont révélés par l'introduction d'une streptavidine marquée à la phycoérythrine (ex : 488 nm) en utilisant les conditions de l'exemple 8.

La détection du marqueur phycoérythrine et l'analyse des résultats ont été aussi réalisés selon le protocole recommandé par Affymetrix et qui est décrit à l'exemple 8.

Les résultats sont donnés dans le tableau 5 ci-dessous.

**Tableau 5 : Suppression de l'étape de purification post-marquage**

| **Volume hybridé sur la puce à ADN (**µ**l)** | **Homologie (%)** | **I (rfu)** | **B(rfu)** | **I/B** |
|---|---|---|---|---|
| 20 | 94,1 | 932 | 801 | 1,2 |
| Réf*. | >95 | 728 | 752 | 1,0 |

| | | | | |
|---|---|---|---|---|
| Réf*.: purification post-marquage sur colonnes du volume total de la réaction de marquage. | | | | |

Ces résultats montrent qu'avec 2 mM en *méta*-BioPMDAM, il est possible de supprimer la purification si on n'hybride pas la totalité du volume réactionnel. Avec 20 µL hybridés sur la puce à ADN, les résultats en termes de pourcentage d'homologie et d'intensité du signal sont comparables à ceux obtenus avec le protocole standard utilisant une étape de purification.

Les mêmes résultats, présentés dans le tableau 6, sont obtenus avec des protocoles de marquage sans métal (MnCl₂). Les signaux sont plus importants.

**Tableau 6 : Suppression de l'étape de purification post-marquage sans MnCl₂**

| **Volume hybridé sur la puce à ADN (**µ**L)** | **Homologie (%)** | **I (rfu)** | **B(rfu)** | **I/B** |
|---|---|---|---|---|
| 20 | 93,5 | 2057 | 875 | 2,3 |

Ces résultats montrent aussi que l'utilisation d'autres agents de fragmentation que les métaux peut être une solution pour éviter les précipités et le bruit de fond important.

Dans cet exemple 9.4, les ARN transcrits utilisés sont dégradés, ce qui explique la faiblesse des signaux, mais la référence utilisée permet la comparaison.

### Exemple 10 : Autres agents de fragmentation utilisables pour le marquage d'ARN :

### Exemple 10.1 : Utilisation d'une chaîne polyamine :

Pour s'affranchir des problèmes de diminution du rendement de marquage en présence de sels, nous avons utilisé une polyamine « biogénique », la spermine comme agent de fragmentation. En effet, cette chaîne polyamine est connue pour son interaction avec les phosphates des acides nucléiques.

Cinquante (50) µL d'ARN transcrits (exemple 5.2) sont incubés 30 minutes à 60 °C avec différentes concentrations de spermine (référence 13275-0010, Acros, FR, solution mère 0,5 M pH 7,5) et 2 mM *méta*-BioPMDAM (3 µL d'une solution de BioPMDAM 100 mM dans le DMSO anhydre) dans 150 µL final.

La purification, l'hybridation et la détection sur puce à ADN ont été réalisées selon le protocole décrit dans l'exemple 8 (détection streptavidine-phycoérythrine).

Les résultats de marquage sont présentés dans le tableau 7 :

**Tableau 7 : Utilisation de la spermine comme agent de fragmentation**

| **Spermine (mM)** | **Homologie (%)** | **I (rfu)** | **B(rfu)** | **I/B** |
|---|---|---|---|---|
| 6 | 93,0 | 444 | 537 | 0,8 |
| 5 | 94,1 | 842 | 552 | 3,3 |
| 4 | 94,6 | 1884 | 461 | 4,1 |
| 1 | 94,6 | 2925 | 449 | 6,5 |

Les résultats de marquage pendant la fragmentation, pourcentage d'homologie et intensité du signal, avec la spermine comme agent de fragmentation sont satisfaisants. Les chaînes polyamines peuvent donc être utilisées pour fragmenter les cibles ARN lors de leur marquage par les réactifs de marquage portant une fonction diazométhyle.

### Exemple 10.2 : Utilisation de dérivés phénanthrolines :

*Essai 1 :* A 5 µL de transcrits ARN sont ajoutés 20 µL de phénanthroline-FeSO₄ (référence P1929, Sigma, 25 mM) et 2 µL de *méta*-BioPMDAM (100 mM dans du DMSO). Le volume est ajusté à 100 µL. Le tube est incubé 60min à 95°C.

*Essai* 2 : A 5 µL de transcrits ARN sont ajoutés 3 µL d'imidazole pH 9,5 (1 M dans de l'eau pure), 0,5 µL de MnCl₂ et 2 µL de *méta*-BioPMDAM (100 mM dans DMSO). Le volume est ajusté à 100 µL. Le tube est incubé 10min à 60°C.

Dans les deux cas, les échantillons sont hybridés, détectés et analysés sur puce à ADN.

Les fragments biotinylés hybridés sur les sondes de capture à la surface de la puce à ADN sont révélés par l'introduction d'une streptavidine couplée à un fluorophore, la phycoérythrine (excitation : 488 nm), comme précédemment.

**Tableau 8 : Utilisation de dérivées phénanthrolines**

| **Conditions** | **Homologie (%)** | **I (rfu)** | **B (rfu)** | **I/B** |
|---|---|---|---|---|
| 5 mM phénanthroline FeSO₄, 2 mM *méta-*bioPMDAM pendant 60 min à 95°C | >95 | 21821 | 633 | 34,5 |
| 30 mM Imidazole pH9,5, 10 mM MnCl₂, 2 mM *méta*-bioPMDAM durant 10 min à 60°C | >95 | 10719 | 540 | 19,9 |

Les résultats obtenus, dans le tableau 8, avec la phénanthroline comme agent de fragmentation sont supérieurs à ceux obtenus avec le protocole standard utilisant le métal et l'imidazole. D'autres dérivés phénanthrolines compléxés avec d'autres métaux que le Fe⁺⁺ peuvent être utilisés, par exemple Cu⁺⁺, Zn⁺⁺, etc.

### Exemple 11 : Marquage et fragmentation en deux étapes d'ARN avec le méta-bioPMDAM :

Cinquante (50) µL d'une solution d'ARN transcrits obtenus selon le protocole décrit dans l'exemple 5.2 sont incubés 30 minutes à 60 °C en présence du tampon de fragmentation : imidazole 30mM et MnCl₂ 10mM. Le dérivé *méta*-bioPMDAM est alors ajouté à une concentration finale de 2 mM et incubé 5 minutes supplémentaires à 60°C.

Avant hybridation, les fragments d'ARN marqués sont purifiés sur colonne selon le protocole décrit précédemment (exemple 8).

**Tableau 9 : Marquage et fragmentation en deux étapes d'ARN avec le méta-bioPMDAM**

| **Protocole utilisé** | **Homologie (%)** | **I (rfu)** | **B(rfu)** | **I/B** |
|---|---|---|---|---|
| 1. Fragmentation puis marquage | 81,1 | 44245 | 791 | 56,0 |
| 2. Marquage pendant la fragmentation | 91,9 | 20548 | 588 | 35,0 |

Ici la comparaison, selon le tableau 9, doit être réalisée en tenant compte des intensités et du ratio I/B car les signaux obtenus dans le cas du protocole 1 (fragmentation puis marquage) sont trop forts. On observe une saturation du lecteur, ce qui conduit à des pourcentages d'homologie (81 %) plus faibles en comparaison avec 91.9% obtenus avec le protocole de référence.

Néanmoins, cet exemple montre que les étapes de fragmentation et de marquage peuvent être séparées.

### Exemple 12 : Amplification du signal pour le marquage d'ARN avec le réactif méta-bioPMDAM :

Le but de cette approche et d'amplifier le signal de fluorescence en introduisant plusieurs fluorophores par molécule de *méta*-bioPMDAM.

### Réaction de marquage

A 5 µL de transcrits (myco 16S), 89 µL d'eau pure (sigma), 30 µL d'imidazole (1 M dans de l'eau pure), 1 µL de MnCl₂ (1 M dans de l'eau pure) et 2 µL de *méta*-bioPMDAM (100 mM dans DMSO) sont ajoutés.

Les tubes sont agités par vortex puis incubés 10 minutes à 60°C.

La purification sur colonne et l'hybridation sur puce à ADN sont réalisées selon le protocole décrit dans l'exemple 8.

### Sans amplification du signal

Les fragments biotinylés sont révélés par l'interaction avec de la streptavidine (marquée à la phycoérythrine : PE) avec le réactif de marquage *méta*-bioPMDAM. Le protocole d'addition de la streptavidine sur la puce à ADN est celui décrit dans l'exemple 8.

### Avec amplification du signal

1^{ère} étape : Fixation de streptavidine (300 µL d'eau pure, 300 µL de tampon Tris 100 mM pH 7 / NaCl 1 M / tween 0,05% / Antimousse 0,005% ; 6 µL de BSA à 50 mg / mL ; 4 µL de streptavidine à 1,5 mg / mL).

2^{ème} étape : Fixation d'anticorps anti-streptavidine biotinylé (300 µL d'eau pure, 300 µL de tampon Tris 100 mM pH 7 / NaCl 1 M / tween 0,05% / Antimousse 0,005% ; 6 µL de BSA à 50 mg / mL ; 3 µL d'anticorps anti-streptavidine biotinylé à 1 mg / mL).

3^{ème} étape : Fixation de streptavidine marquée la phycoérythrine (300 µL d'eau pure, 300 µL de tampon Tris 100 mM pH 7 / NaCl 1 M / tween 0,05% / Antimousse 0,005% ; 6 µL de BSA à 50 mg / mL ; 6 µL streptavidine marquée la phycoérythrine à 300 µg / mL).

On utilise l'anti-streptavidine biotinylé suivante dans les étapes ci-dessus : référence 216-065-084, Jackson Immuno Research.

La lecture des signaux sur la puce à ADN est effectuée selon le protocole décrit dans l'exemple 8. Les résultats en termes de pourcentage d'homologie et d'intensité de marquage sont donnés dans le tableau 10 ci-dessous :

**Tableau 10 : Amplification du signal pour le marquage d'ARN avec le réactif méta-bioPMDAM**

| **Marquage post-hybridation** | **Homologie (%)** | **I (rfu)** | **B(rfu)** | **I/B** |
|---|---|---|---|---|
| Sans amplification du signal | >95 | 2400 | 581 | 4,1 |
| Avec amplification du signal | >95 | 13798 | 860 | 16,0 |

L'amplification du signal améliore considérablement la sensibilité de détection.

### Exemple 13 : Marquage d'ARN par les dérivés ortho-bioPMDAM (3c) :

Le réactif a été préparé selon le protocole décrit dans l'exemple 1.3 et a été évalué dans l'approche de marquage pendant la fragmentation des ARN transcrits.

Les ARN transcrits ont été obtenus selon le protocole décrit dans l'exemple 5.2.

Le protocole de marquage, d'hybridation, d'introduction de la streptavidine marquée à la phycoérythrine et le protocole de purification sont ceux décrits dans l'exemple 8.

Les résultats sont donnés dans le tableau 11 ci-dessous :

**Tableau 11 : Marquage d'ARN par le dérivé ortho-bioPMDAM**

| **Molécule utilisée** | **Homologie (%)** | **I (rfu)** | **B(rfu)** | **I/B** |
|---|---|---|---|---|
| *ortho*-bioPMDAM | >95 | 3657 | 532 | 6,9 |

Ces résultats montrent que la substitution en *ortho* donne aussi des résultats intéressants en terme d'intensité de marquage et de pourcentage d'homologie.

### Exemple 14 : Marquage des ARN transcrits par un dérivé BioDPDAM (3d) :

Le dérivé BioDPDAM (3d) a été préparé selon le protocole décrit dans l'exemple 1.4.

Les ARN transcrits ont été obtenus selon le protocole décrit dans l'exemple 5.2.

Le procédé marquage et fragmentation est effectué en une étape en comparaison avec le réactif *méta*-BioPMDAM dans les conditions décrites dans le tableau 12 ci-dessous (volume de réaction 100 µL).

Le protocole d'hybridation, d'introduction de la streptavidine marquée à la phycoérythrine et le protocole de purification sont ceux décrits dans l'exemple **8**.

**Tableau 12 : Marquage d'ARN transcrits par le BioDPDAM (3d)**

| **Conditions de marquage** | **Homologie (%)** | **I (rfu)** | **B(rfu)** | **I/B** |
|---|---|---|---|---|
| **Protocole de référence** : 30 mM Im pH 9,5 ; 5 mM MnCl₂ ; 2 mM *méta*-bioPMDAM (3a) ; 10 min à 60°C | 89,7 | 35519 | 528 | 67,2 |
| 30 mM Im pH 9,5 ; 5 mM MnCl₂ ; 2 mM BioDPDAM (3d) ; 10 min à 60°C | >95 | 3252 | 446 | 7,3 |
| 30 mM Im pH 9,5 ; 5 mM MnCl₂ ; 2 mM BioDPDAM (3d) ; 15 min à 60°C | >95 | 4482 | 435 | 10,3 |
| 30 mM Im pH 9,5 ; 5 mM -MnCl₂ ; 2 mM BioDPDAM (3d) ; 30 min à 60°C | >95 | 6429 | 428 | 15,0 |

| | | | | |
|---|---|---|---|---|
| Im =Imidazole | | | | |

Le dérivé 3d donne de bons résultats de marquage des ARN sur le phosphate. Ce résultat montre que des substitutions, telles que le groupement phényle, peuvent être utilisées pour moduler la réactivité et la stabilité des marqueurs portant la fonction diazométhyle.

### Exemple 15 : Marquage d'ARN par le dérivé Cy5-PMDAM, (12bis) :

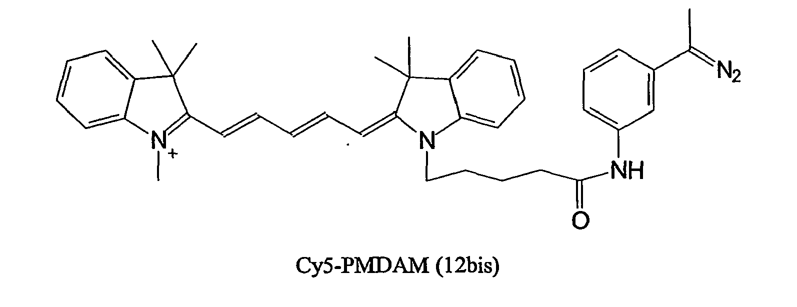

Le marqueur Cy5-PMDAM (12bis) a été préparé selon le protocole décrit dans l'exemple 2.

Cinq (5) µL de transcrit ARN (exemple 5.2) sont marqués par le Cy5-PMDAM dans différentes conditions de fragmentation et de marquage. Les conditions générales sont les suivantes :
- volume final de réaction 100 µL,
- 30 mM d'imidazole pH 8 (3 µL d'une solution mère à 1 M),
- 5 mM de MnCl₂ (6 µL d'une solution mère à 0,1 M), et
- 3 mM du Cy5-PMDAM (3 µL d'une solution mère à 100 mM dans du DMSO anhydre).

Dans un premier essai, le marquage pendant la fragmentation est réalisé en une seule étape. Dans un deuxième essai, le marquage et la fragmentation ont été réalisés en deux étapes : la fragmentation est réalisée avant le marquage.

Après chaque incubation ayant contenue du MnCl₂, le sel est neutralisé par addition de 20 µL d'une solution à 0,5 M d'EDTA.

L'incubation a été réalisée à 60 °C pendant 30min pour le marquage et la fragmentation en une étape. Pour le deuxième essai, où le marquage et la fragmentation ont été réalisés en deux étapes, l'incubation, 15min d'incubation, a été utilisée pour chaque étape.

Les fragments marqués ont été purifiés, hybridés et détectés selon le protocole décrit dans l'exemple 8. La seule différence réside dans le fait que lors de l'étape d'hybridation, l'addition de la streptavidine marquée n'est plus nécessaire, car le marqueur est détecté de manière directe par un lecteur approprié (GMS 418 Array Scanner, Affymetrix, Santa Clara, CA).

Les résultats de marquage sont donnés dans le tableau 13 ci-dessous :

**Tableau 13 : Marquage d'ARN transcrits par le dérivé de Cy5-PMDAM**

| **Protocole de marquage** | **Homologie (%)** | **I (rfu)** | **B(rfu)** | **I/B** |
|---|---|---|---|---|
| Marquage pendant la fragmentation | >95 | 6612 | 352 | 17,3 |
| Fragmentation puis marquage | 94,1 | 8378 | 158 | 53,1 |

Le marquage sur le phosphate des ARN transcrits par un réactif de marquage (12bis) donne des résultats très satisfaisants en termes d'intensité de marquage et du pourcentage d'homologie.

Il est aussi important de noter que le rapport signal sur bruit de fond est très satisfaisant dans le cas de ce marqueur. En effet, les longueurs d'onde d'excitation et d'émission des dérivés cyanines sont éloignées de celles des molécules biologiques.

Ces résultats montrent aussi que la fonction diazométhyle permet l'introduction d'autres marqueurs que la biotine au niveau des groupements phosphates des acides nucléiques.

### Exemple 16 : Etude de la fragmentation de l'ADN :

### Exemple 16.1 : Hydrolyse de différents nucléosides en milieu acide :

Le but de cette étude est de démontrer la différence en terme de stabilité à pH acide entre les nucléosides naturels, les nucléosides modifiés ainsi que les nucléosides de type puriniques et pyrimidiniques. Cette étude permet également de mieux contrôler la fragmentation de l'ADN en tenant compte de sa composition en bases.

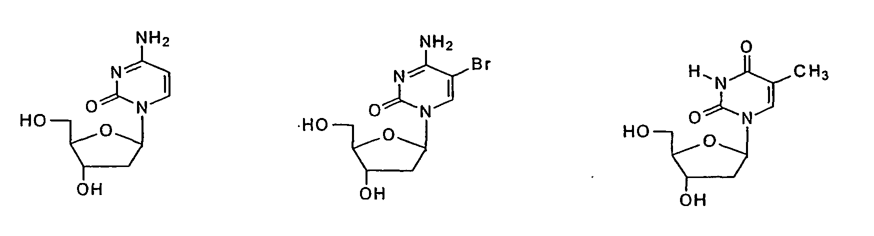

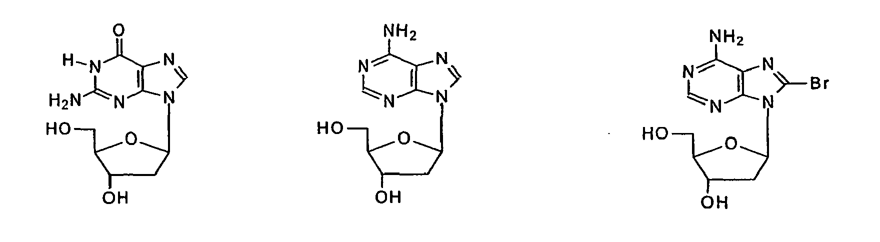

Deux nucléosides modifiés, la 8-bromo-2'-désoxyadénosine (8-BrdA) et la 5-bromo-2'-désoxycytidine (5-BrdC) ainsi que les quatre (4) nucléosides naturels (dA, dC, dG et dT) ont été utilisés dans cette étude.

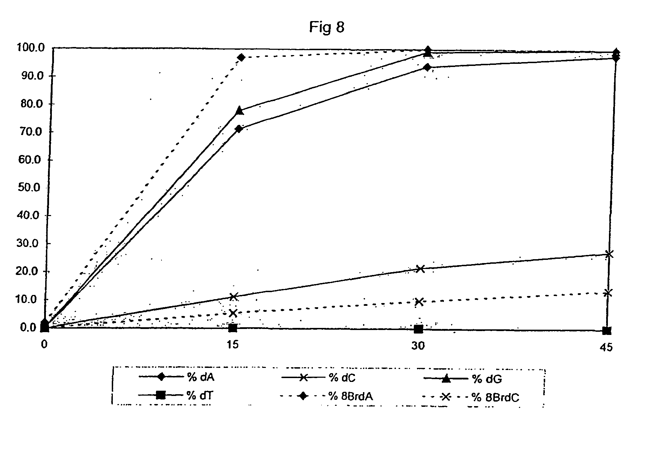

50 nanomoles (nmol) de chaque nucléoside sont incubées dans 50 mM de formiate de sodium pH 3 à 95°C. Les temps d'incubation varient de 0 à 45 min. Après séchage sous vide et reprise par 20 µl d'eau pure, les échantillons (10 nmol) sont alors analysés par HPLC en phase inverse. Les résultats sont donnés sous forme de pourcentage d'hydrolyse du nucléoside de départ (en ordonnée) par rapport au temps d'incubation en minutes (en abscisse) voir figure 8.

Les courbes de la figure 8 montrent que la modification de l'adénine en position 8 par un atome de brome rend ce nucléoside moins stable que le nucléoside naturel. D'autre part, les résultats montrent que, dans les conditions utilisées, la dépurination est beaucoup plus importante que la dépyrimidination.

Cette étude montre que la fragmentation de l'ADN par la dépurination ou la dépyrimidination peut être contrôlée en optimisant les conditions d'hydrolyse, ou en incorporant soit des bases modifiées moins stables que les bases naturelles soit des bases pouvant être modifiées et hydrolysées après leur incorporation.

### Exemple 16.2 : Fragmentation de l'ADN double brin incorporant ou non un nucléotide modifié :

Trois amplifications PCR ont été réalisées en parallèle à partir de cible ADN génomique *Mycobacterium tuberculosis* 16S (10⁺⁴ copies de départ) en utilisant le kit *Fast Start* de Roche, 0,2 mM de chaque désoxyribonucléotide (d-ATP, d-CTP, d-GTP, d-TTP), 0,3 µM d'amorces et 0,4 µL d'enzyme.

Les paramètres de la PCR sont ceux de l'exemple 5.

Dans le premier cas, le protocole est utilisé tel quel : cas de la PCR dite naturelle.

Dans le deuxième cas, le protocole est modifié pour obtenir une PCR à 30 % de 8Br-dATP. Ceci est réalisé en introduisant 0,2 mM de d-CTP, d-GTP et d-TTP. On introduit également 0,14 mM de d-ATP et 0,06 mM de 8-BrdATP. (La 8-BrdATP est commerciale (référence N-2005-1, TriLink Biotechnologies, San Diego CA).

Dans le troisième cas, le protocole est modifié pour obtenir une PCR à 50 % de 8-BrdATP. Ceci est réalisé en introduisant 0,2 mM de d-CTP, d-GTP et d-TTP. On introduit également 0,1 mM de d-ATP et 0,1 mM de 8-BrdATP.

L'étude de la fragmentation seule de ces amplicons a été réalisée dans les conditions décrites ci-dessus : 50 mM de formiate de sodium pH 3 à 95°C.

L'analyse a été effectuée sur gel dénaturant de polyacrylamide (8 % polyacrylamide, 7 M urée, 1 X TBE) utilisant coloration au bromure d'éthidium.

Après 15 min d'incubation à 95 °C dans 50 mM de formiate de sodium pH 3, aucune différence n'est visible entre les trois (3) cibles. Dans tous les cas, nous avons observé une fragmentation totale des amplicons PCR.

La dépurination des amplicons PCR a été également réalisée à différents pH et à différentes températures et temps d'incubation. L'analyse sur gel, dans les conditions ci-dessus, montre qu'à pH 3, la fragmentation est totale après seulement 10 min d'incubation à 95°C. A ce pH, la fragmentation est aussi totale à 60°C après 30 min d'incubation.

A pH 4, 30 min d'incubation sont nécessaires pour l'obtention d'une fragmentation totale des amplicons ADN même à 95°C. Ce résultat est très intéressant et montre que le site abasique généré à pH acide est instable et donc conduit. à la fragmentation de la chaîne ADN sans aucun autre traitement particulier.

### Exemple 17 : Marquage et fragmentation de l'ADN par le dérivé méta-bioPMDAM (3a) en deux étapes :

Le dérivé *méta*-bioPMDAM (3a) a été obtenu selon le schéma réactionnel décrit dans l'exemple 1.1.

Les amplicons ADN ont été préparés par amplification PCR selon le protocole décrit dans l'exemple 5.1.

### Marquage

A 10 µl de PCR, 38 µL d'eau pure (sigma), 50 µL de formiate de sodium à pH 3 (100 mM dans l'eau pure) sont ajoutés et le mélange est incubé pendant 30 minutes à 60°C. Puis 2 µL de *méta*-bioPMDAM (100 mM dans DMSO) sont ajoutés ensuite. La solution a été mélangée par vortex, puis incubée 15 minutes supplémentaires à 60°C.

Les essais sont réalisés en duplicata afin de pouvoir analyser la fragmentation de l'ADN sur gel et l'efficacité de marquage par hybridation et lecture de la puce à ADN.

### Purification

La purification est réalisée sur colonnes QIAQUICK™ (Nucleotide Removal kit, Qiagen, Hilden, Allemagne). Le protocole de purification utilisé est celui recommandé par le fournisseur.

Après purification, l'éluat est transféré dans un tube propre contenant 400 µL de tampon d'hybridation (1,75 mL 20X SSPE ; 2,9 mL Bétaine 5M; 290 µL DTAB 0,1M; 10 µL Antimousse 30%) sont ajoutés. Les références de ces substances sont, pour :
- la bétaine référence B-2754 Sigma, et
- la DTAB référence D-5047 Sigma.

La solution est mélangée par vortex et incubée 10 min à 95°C, afin de séparer les brins d'ADN qui ne sont pas séparés lors de l'étape de marquage pendant la fragmentation (étape de dénaturation). Le tube est ensuite plongé dans un mélange eau-glace à 0°C avant hybridation sur puce à ADN.

### Hybridation sur puce à ADN

Après l'étape de marquage pendant la fragmentation, les fragments obtenus sont hybridés sur les puces à ADN conçues pour l'analyse de la région 213-415 de la séquence M20940 « Genbank » de l'ARN 16S de *Mycobacterium tuberculosis.* Cette puce à ADN est décrite dans A. Troesch et al, J. Clin Microbiol., 37(1), p49-55, 1999.

Les étapes d'hybridation ont été réalisées sur les stations fluidiques (Affymetrix, Santa Clara, CA) en utilisant le protocole d'hybridation et les tampons décrits dans A. Troesch et al, J. Clin Microbiol., 37(1), p49-55, 1999. Une étape supplémentaire est nécessaire pour révéler la biotine (détection indirecte).

L'hybridation est révélée par le couplage de la streptavidine marquée à la phycoérythrine (PE) qui interagit avec la biotine de la *méta*-BioPMDAM dans les conditions suivantes :
300 µL d'eau pure ; 300 µL de tampon Tris 100 mM pH 7 / NaCl 1M / tween 0,05% / Antimousse 0,005% ; 6 µL de BSA (50 mg/mL) ; 6 µL de streptavidine-PE (300 µg / mL). Les références de ces substances sont, pour :
   - la Streptavidine-Phycoérythrine : référence : R0438, Dako, Danemark,
   - la Streptavidine-CY5 : référence : C0050, Dako, Danemark,
   - Antimousse référence M5-575, Ultra Additives Inc., et
   - le Tween référence P-7949, Sigma.

### Lecture de la puce à ADN :

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix (GeneChip® Instrument System et GeneChip® Information System, Santa Clara CA).

Le système de lecture fournit des intensités en signal et bruit de fond exprimées en rfu (« relative fluorescence unit »). Le pourcentage d'homologie est donné par rapport à une séquence référence qui dans ce cas est la séquence de *Mycobacterium tuberculosis.*

Les résultats en termes d'intensité moyenne du signal (I), du bruit de fond (B) et du pourcentage d'homologie (%) sont donnés dans le tableau ci-dessous :

De manière générale, on considère qu'un pourcentage d'homologie supérieur à 90% est un résultat satisfaisant bien que l'on cherche en général un résultat supérieur à 95%. Au dessus de 95%, les valeurs ne sont plus indiquées car elles ne sont pas significatives dans le cas de la puce à ADN Mycobactéries. Une intensité élevée avec un bruit de fond faible est le deuxième résultat recherché dans les exemples qui suivent. Dans tous les résultats, le bruit de fond B est déduit de l'intensité moyenne I.

### Analyse sur gel de polyacrylamide

Les échantillons destinés à être analysés sur gel sont séchés sous vide, repris par 10 µL d'eau pure et 10 µL de bleu formamide 2X.

La migration est effectuée sur gel d'acrylamide 8%, dans du TBE 1X, une (1) heure à 150 V.

Le pH acide a été utilisé pour la fragmentation de l'ADN. En effet à ce pH, le phénomène de dépurination engendre des sites abasiques très instables conduisant à une fragmentation presque immédiate des séquences ADN à température élevée. Ce type de fragmentation produit des fragments ADN-5' phosphate.

L'analyse sur gel montre que l'incubation des amplicons PCR à 60°C pendant 30 min en solution dans le tampons formiate (50 mM, pH 3) conduit à une fragmentation totale de ces amplicons. Ceci nous a permis d'évaluer le marquage pendant la fragmentation des amplicons ADN en présence du marqueur *méta*-bioPMDAM.

Les résultats de marquage pendant la fragmentation des amplicons ADN en termes de pourcentage d'homologie, d'intensité des signaux et du bruit de fond sont donnés dans le tableau 14 ci-dessous.

**Tableau 14: Marquage et fragmentation des amplicons ADN en termes d'homologie, d'intensité des signaux (I) et du bruit de fond (B)**

| **Conditions de marquage des amplicons PCR** | **Homologie (%)** | **I (rfu)** | **B (rfu)** | **I/B** |
|---|---|---|---|---|
| Tampon : formiate pH3, 50 mM Marqueur: 2 mM *méta*-bioPMDAM Incubation : 30 min à 60°C | > 95 | 4456 | 593 | 7,5 |

Cet exemple montre que les dérivés de l'invention peuvent être utilisés pour le marquage des fragments ADN produits par amplification enzymatique dans un protocole en deux étapes. Ils peuvent être également utilisés pour le marquage de l'ADN naturel non amplifié.

### Exemple 18 : Marquage de l'ADN par le dérivé Cy5-PMDAM (12bis) :

Le marquage de l'ADN par ce nouveau marqueur portant le fonction diazométhyle a été évalué en utilisant un fragment ADN synthétique.

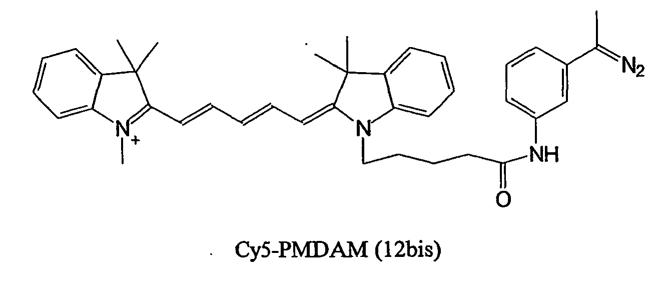

Le réactif de marquage Cy5-PMDAM (12bis) est préparé selon le protocole décrit dans l'exemple 2.

Un oligodésoxyribonucléotide (ODN) vingt (20) mers est préparé selon la méthode dite au phosphoramidite. On introduit un phosphate à l'extrémité 5' par un réactif de phosphorylation standard compatible avec la chimie au phosphoramidite. La séquence de cet ODN est constituée de toutes les bases naturelles de l'ADN (séquence de l'ODN : 5'-CTGAACGGTAGCATCTTGAC-3'). Cette séquence est complémentaire de séquences de capture d'une puce à ADN dite « modèle », synthétisée selon la technologie Affymetrix. Cette puce à ADN contient des sondes de capture identiques en séquence et qui sont réparties en damier sur sa surface. La lecture de cette puce à ADN donne des informations quant à la performance du marquage en terme d'intensité mais pas de résultat d'homologie.

Marquage : A 50 picomoles (pmoles) de cet ODN, 10 µL de Cy5-PMDAM (100 mM dans le DMSO) sont ajoutés. Le volume final est de 100 µL. Après homogénéisation, l'incubation est réalisée à 60°C pendant 30 minutes.

Purification et lecture : La purification pour éliminer l'excès de réactif de marquage est réalisée selon l'exemple 17. La lecture sur puce à ADN est effectuée selon l'exemple 17.

### Résultats :

La moyenne des intensités de marquage (I) lue sur la puce à ADN est de 16 644 rfu pour un bruit de fond (B) de 450 rfu.

Ce niveau d'intensité est très élevé et montre que le réactif de marquage Cy5-PMDAM (12bis) est tout à fait compatible avec le marquage des fragments d'ADN sur le groupement phosphate.

### Exemple 19 : Marquage et fragmentation des amplicons ADN obtenus par amplification PCR avec le réactif de marquage méta-bioPMDAM (3a):

Le dérivé *méta*-bioPMDAM (3a) a été obtenu selon le schéma réactionnel décrit dans l'exemple 1.1.

Les amplicons ADN ont été préparés par amplification PCR selon le protocole décrit dans l'exemple 5.1.

### Exemple 19.1 : Comparaison du marquage avec et sans fragmentation :

### a. Marquage dans les conditions de fragmentation :

A 10 µL de PCR sont ajoutés 50 µL de formiate de sodium pH 3 (50 mM) et 2 µL de *méta-*BioPMDAM 100 mM dans du DMSO). Le volume est ajusté à 100 µL. La solution est incubée 30 min à 60°C.

### b. Marquage sans fragmentation :

A 10 µL de PCR sont ajoutés 2 µL de *méta*-BioPMDAM (100 mM dans du DMSO). Le volume est ajusté à 100 µL. La solution est incubée 30 min à 60°C.

Le reste du protocole est identique à celui de l'exemple 17.

### Résultats

**Tableau 15 : Comparaison du marquage avec et sans fragmentation**

| **Protocole utilisé** | **Homologie (%)** | **I (rfu)** | **B (rfu)** | **I/B** |
|---|---|---|---|---|
| *a. Marquage dans les conditions de fragmentation* | >95 | 3995 | 569 | 7,0 |
| *b. Marquage sans fragmentation* | 94,1 | 500 | 542 | 0,9 |

Les résultats dans le tableau 15 ci-dessus montre que sans fragmentation, la moyenne des intensités obtenues est au même niveau que le bruit de fond (500 rfu). Le marquage pendant la fragmentation donne un niveau d'intensité bien supérieur (environ 4000 rfu) et un très bon pourcentage d'homologie. La combinaison des deux étapes représente donc bien une amélioration significative pour la détection d'un acide nucléique de longueur supérieure à cent (100) nucléotides.

### Exemple 19.2 : Effet de la dénaturation avant hybridation sur puce à ADN :

Deux réactions de marquage ont été réalisées en parallèle dans deux tubes séparés selon le protocole suivant : A 10 µL de PCR sont ajoutés 50 µL de tampon formiate de sodium pH 3 (50 mM) et 2 µL de *méta-bio*PMDAM (100mM dans du DMSO). Le volume total est ajusté à 100 µL et incubé 30 min à 60°C.

Après purification sur colonne (exemple 17), la solution issue du premier tube est incubée 10 min à 95°C (afin de désapparier le double brin d'ADN), puis le tube est plongé dans un mélange eau-glace à 0°C jusqu'à l'hybridation sur la puce ADN.

La solution issue du deuxième tube est hybridée sur la puce à ADN sans dénaturation préalable.

Les fragments biotinylés hybridés sur les sondes de capture à la surface de la puce à ADN sont révélés par l'introduction d'une streptavidine marquée par la phycoérythrine en utilisant les conditions décrites dans l'exemple 17.

### Résultats

**Tableau 16 : Effet de la dénaturation avant hybridation sur puce à ADN**

| **Conditions utilisées** | **Homologie (%)** | **I (rfu)** | **B (rfu)** | **I/B** |
|---|---|---|---|---|
| *Avec dénaturation* | >95 | 22812 | 570 | 40,1 |
| *Sans dénaturation* | 93,5 | 4795 | 681 | 7,0 |

Les résultats obtenus, présentés dans le tableau 16, avec la dénaturation pré-hybridation sont supérieurs à ceux obtenus sans l'étape de dénaturation. Ceci montre que la dénaturation de l'ADN est nécessaire pour obtenir un bon niveau d'intensité. La fragmentation via les sites abasiques est un moyen pour faciliter la dénaturation d'un ADN double brin et renforcer l'hybridation sur les sondes de captures.

Pour tester d'autres réactifs de marquage et compte tenu des résultats obtenus avec les différentes conditions ci-dessus, nous avons défini un protocole de référence utilisant la fragmentation au tampon formiate de sodium (50 mM, pH 3) et un étape de dénaturation pré-hybridation.

### Exemple 20 : Marquage et fragmentation des amplicons PCR par les réactifs biotinylés dans un protocole en une étape :

Les dérivés *méta*- et *ortho*-bioPMDAM ont été préparés selon le protocole décrit dans les exemples 1.1 et 1.3. Ils ont été solubilisés dans du DMSO anhydre à une concentration de 100 mM.

Le protocole est identique à celui de l'exemple 19.2 ci-dessus (marquage et fragmentation en une étape puis étape de dénaturation pré-hybridation).

### Résultats

**Tableau 17 : Marquage et fragmentation des amplicons PCR par les réactifs biotinylés dans un protocole en une étape**

| **Marqueur** | **Homologie (%)** | **I (rfu)** | **B (rfu)** | **I/B** |
|---|---|---|---|---|
| *ortho*-bioPMDAM | >95 | 25951 | 820 | 31,6 |
| *méta*-bioPMDAM | >95 | 22960 | 581 | 39,5 |

Le protocole optimisé avec fragmentation et marquage en une étape donne d'excellents résultats avec différents réactifs de marquage avec la fonction réactive diazométhyle, comme le montrent les résultats exposés dans le tableau 17.

### Exemple 21 : Marquage et fragmentation de l'ADN par le BioDPDAM :

Les amplicons ADN ont été préparés par amplification PCR selon le protocole décrit dans l'exemple 5.1.

La synthèse du réactif de marquage est décrite dans l'exemple 1.

Le protocole est identique à celui de l'exemple 19.2, y compris la dénaturation à 95 De avant l'étape d'hybridation.

### Résultats

**Tableau 18 : Marquage et fragmentation de l'ADN par le BioDPDAM**

| **Marqueur utilisé** | **Homologie (%)** | **I (rfu)** | **B (rfu)** | **I/B** |
|---|---|---|---|---|
| BioDPDAM | 93,0 | 32359 | 3610 | 9,1 |

Ce résultat, décrit dans le tableau 18, montre que des substitutions aussi importantes que le groupement phényle peuvent être utilisées pour optimiser la réactivité des réactifs de marquage portant une fonction diazométhyle.

### Exemple 22 : Marquage et fragmentation de l'ADN par la 5-(bromométhyl)fluorescéine :

Les amplicons ADN ont été préparés par amplification PCR selon le protocole décrit dans l'exemple 5.1.

A 10 µL de PCR sont ajoutés 50 µL de formiate de sodium pH3 (100 mM) et 2 µL de 5-(bromométhyl)fluorescéine (Molecular probes, Eugen, OR) (100 mM dans du DMSO). Le volume est ajusté à 100 µL. La solution est incubée 30 min à 60°C.

Les conditions de purification sont conformes à celles de l'exemple 17. Une étape de dénaturation est effectuée comme décrit dans l'exemple 190.2.

Les autres conditions d'hybridation et de lecture sont identiques à celles décrites dans l'article de A. Troesch et al. J. Clin. Microbiol., 37(1), p 49-55 1999. La fluorescéine est directement détectable sur le lecteur.

**Tableau 19 : Marquage et fragmentation de l'ADN par la 5-(bromométhyl)fluorescéine_**

| **Protocole utilisé** | **Homologie (%)** | **I (rfu)** | **B (rfu)** | **I/B** |
|---|---|---|---|---|
| *Marquage et fragmentation des amplicons PCR avec la 5-(bromo-méthyl)fluorescéine* | >95 | 855 | 183 | 4,7 |

Ce résultat du tableau 19 montre que la fragmentation de l'ADN par la création de sites abasiques est tout à fait compatible avec un réactif de marquage portant une fonction réactive halogénure d'alkyle. Ce protocole est réalisé en une étape (fragmentation et marquage), mais avec des intensités plus faibles qu'avec les marqueurs portant la fonction diazométhyle.

### Exemple 23 : Marquage et fragmentation des amplicons ADN en présence d'un autre agent chimique de fragmentation dérivé de la phénanthroline :

Des amplicons ADN ont été préparés par amplification PCR selon le protocole décrit dans l'exemple 5.1. Deux types de conditions sont utilisées :

### Conditions a :

A 10 µL de PCR sont ajoutés 20 µL de phénanthroline-FeSO₄ (25 mM) et 2 µL de *méta-*BioPMDAM (100 mM dans DMSO). Le volume total est ajusté à 100 µL. Le mélange est incubé 60 min à 95°C.

### Conditions b :

A 10 µL de PCR sont ajoutés 50 µL de tampon formiate de sodium pH3 (100 mM dans eau pure) et 2 µL de de *méta*-BioPMDAM (100 mM dans DMSO). Le volume total est ajusté à 100 µL. Le mélange est incubé 60 min à 95°C.

Les autres conditions du protocole sont identiques à celles de l'exemple 17.

**Tableau 20 : Marquage et fragmentation des amplicons ADN en présence de la phénanthroline**

| **Conditions** | **Homologie (%)** | **I (rfu)** | **B (rfu)** | **I/B** |
|---|---|---|---|---|
| Phénanthroline FeSO₄(5 mM) *méta*-bioPMDAM (2 mM) 60 min à 95°C | >95 | 2236 | 500 | 4,5 |
| Tampon formiate (50mM) pH3 *méta*-bioPMDAM (2 mM) 60 min à 95°C | >95 | 6786 | 565 | 12,0 |

Les deux conditions de fragmentation permettent un résultat satisfaisant comme démontré dans le tableau 20.

Le meilleur résultat est obtenu avec les conditions (b) utilisant la fragmentation à pH acide.

### Exemple 24 : Fragmentation des amplicons PCR marqués par incorporation de d-UTP-fluorescéine :

### Incorporation du nucléotide marqué

Une amplification PCR a été réalisée selon le protocole suivant afin générer des amplicons PCR marqués par la fluorescéine (marquage sur les bases).

A partir de cible ADN génomique *Mycobacterium tuberculosis* 16S (10⁺⁴ copies) en utilisant le kit *Fast Start* de Roche, 0,2 mM des désoxyribonucléotides d-ATP, d-CTP et d-GTP ainsi que 0,14 mM de d-TTP et 0,06 mM de d-UTP-12-fluoresceine, 0,3 µM d'amorces et 0,4 µL d'enzyme. Le pourcentage du nucléotide marqué par rapport à son homologue naturel d-UTP est de 30%. C'est ce rapport qui est généralement utilisé dans les réactions de marquage des amplicons par incorporation de nucléotides marqués.

La d-UTP-12-fluoresceine est disponible commercialement chez Roche Diagnostics référence 1373242, Mannheim, Allemagne).

Les paramètres de la PCR sont ceux de l'exemple 5.1.

### a. Fragmentation des amplicons PCR marqués à 30% par d-UTP-fluorescéine :

A 10 µL de PCR sont ajoutés 50 µL de tampon formiate de sodium pH 3 (50 mM). Le volume est ajusté à 100 µL. La solution est ensuite incubée 30 min à 60°C.

### b. Marquage pendant la fragmentation des amplicons PCR contenant 30% par d-UTP-fluorescéine :

A 10 µL de PCR sont ajoutés 50 µL de tampon formiate de sodium pH 3 (50 mM) et 2 µL de *méta*-BioPMDAM (100 mM dans du DMSO). Le volume est ajusté à 100 µL. La solution est ensuite incubée 30 min à 60°C. Cet essai correspond à un protocole de référence qui permet de comparer les différentes stratégies de marquage en s'affranchissant de la variabilité due à l'étape d'amplification.

Une étape de purification sur colonne et une étape de dénaturation à 95°C sont effectuées dans tous les cas comme dans l'exemple 17.

### Protocole (a1) :

Les acides nucléiques obtenus par fragmentation des amplicons marqués à la d-UTP-fluorescéine (conditions *a*)) sont hybridés sur puce à ADN et détectés dans un premier temps par lecture directe des signaux fluorescents émis par la fluorescéine comme décrit dans l'exemple 22.

### Protocole (a2) :

Une étape d'amplification du signal a été utilisée pour améliorer la sensibilité de marquage. L'amplification du signal a été réalisée par l'introduction, lors de l'étape d'hybridation, d'un anticorps anti-fluorescéine biotinylé (référence 216-065-084, Jackson ImmunoResearch) puis d'une streptavidine marquée par la phycoérythrine en utilisant les conditions successives suivantes :
- 300 µL d'eau pure,
- 300 µL de tampon Tris 100 mM pH 7 / NaCl 1M / tween 0,05% / Antimousse 0,005% , 2,4 µL de BSA (50 mg/mL), 1,2 µL d'anticorps anti-fluorescéine biotinylé. (1 mg / mL),
- 300 µL d'eau pure, et
- 300 µL de tampon Tris 100 mM pH 7 / NaCl 1M / tween 0,05% / Antimousse 0,005% ; 6 µL de BSA (50 mg/mL) ; 6 µL de streptavidine-PE. (300 µg / mL).

Dans ce protocole, la fluorescéine agit comme un haptène (traceur détectable indirectement par un anticorps marqué) et non pas comme un fluorophore.

### Protocole (b) :

Les fragments biotinylés (condition *b)* hybridés sur puce à ADN sont révélés par l'introduction d'une streptavidine marquée par la phycoérythrine en utilisant les conditions suivantes :
- 300 µL d'eau pure, et
- 300 µL de tampon Tris 100 mM pH 7 / NaCl 1M / tween 0,05% / Antimousse 0,005% ; 6 µL de BSA (50 mg/mL) ; 6 µL de streptavidine marquée. (300 µg / mL).

La lecture de la fluorescence émise à la surface de la puce à ADN après marquage et hybridation ainsi que la génération des données en termes d'intensité du signal et du pourcentage d'homologie sont réalisés par les systèmes de lecture et le logiciel fournis par Affymetrix. A ce propos, il est important de noter que le système de lecture utilisé contient deux filtres permettant de détecter directement :
- la fluorescéine dans le cas où les amplicons sont marqués au d-UTP-fluorescéine uniquement, selon le protocole *al*, ou bien
- la phycoérythrine dans le cas où les amplicons sont marqués :
   - au d-UTP-fluorescéine avec amplification du signal, selon le protocole a2, ou
   - par le *méta*-bioPMDAM pendant leur fragmentation, selon le protocole *b*.

Dans les deux cas où la révélation s'effectue par la phycoérythrine, l'utilisation d'un filtre permet de s'affranchir du signal généré par la fluorescéine et c'est bien le signal de la phycoérythrine qui est détecté.

### Résultats

**Tableau 21 : Fragmentation des amplicons PCR marqués par incorporation d-UTP-fluorescéine**

| **Protocole utilisé** | **Marqueur détecté** | **Homologie (%)** | **I (rfu)** | **B (rfu)** | **I/B** |
|---|---|---|---|---|---|
| *a1. Fragmentation des amplicom PCR marqués par la d-UTP-fluorescéine* | Flu* | 81,6 | 595 | 342 | 1,7 |
| *a2. Fragmentation des amplicons PCR marqués par la d-UTP-fluorescéine avec amplification du signal* | PE* | >95 | 22107 | 3461 | 6,4 |
| *b. Fragmentation et marquage sur les mêmes amplicons PCR modifiés par la d-UTP-fluorescéine et marqués par la méta-BioPMDAM* | PE* | >95 | 21700 | 1503 | 14,4 |

| | | | | | |
|---|---|---|---|---|---|
| * Flu =Fluorescéine et PE = Phycoérythrine | | | | | |

Les résultats, du tableau 21 ci-dessus, montrent que la fragmentation chimique utilisant la création de site abasique est compatible avec le marquage enzymatique des amplicons ADN et que le marquage peut avoir lieu avant la fragmentation.

Les niveaux d'intensité ainsi que le pourcentage d'homologie obtenus avec ce protocole d'incorporation enzymatique du fluorophore sont faibles en comparaison de ceux obtenus avec le marquage pendant la fragmentation utilisant le réactif de marquage avec une fonction diazométhyle comme le *méta*-bioPMDAM (conditions (b)).

Pour atteindre le niveau d'intensité obtenue avec le dérivé *méta*-bioPMDAM, une étape d'amplification du signal est nécessaire (conditions a2). Ceci montre bien l'efficacité de la fonction réactive diazométhyle par rapport à l'incorporation traditionnelle de base modifiée comme le d-UTP fluorescéine (protocole de référence (b)).

### Exemple 25 : Fragmentation de l'ADN double brin par sonication :

Des amplicons ADN ont été obtenus en utilisant le protocole décrit dans l'exemple 5.1. Ces amplicons ont été fragmentés par sonication en présence et en absence du marqueur.

### a. Marquage des amplicons PCR pendant la sonication :

A 10 µL de réaction PCR sont ajoutés 2 µL de *méta*-BioPMDAM (100 mM dans DMSO). Le volume est ajusté à 100 µL par de l'eau pure le pH est ajusté à 6,5. Le mélange est incubé 30 min à 60°C dans un bain d'une cuve à ultrasons (fréquence 35 kHz, modèle T460-H, Bioblock, France).

### b. Marquage pendant la fragmentation chimique des amplicons PCR (protocole de référence en une étape) :

A 10 µL de réaction PCR sont ajoutés 50 µL de formiate de sodium pH 3 (50 mM) et 2 µL de *méta*-BioPMDAM (100 mM dans du DMSO). Le volume est ajusté à 100 µL. La solution est incubée 30 min à 60°C.

Les essais sont réalisés en duplicata afin de pouvoir analyser la fragmentation de l'ADN sur gel et l'efficacité de marquage par hybridation et lecture de la puce à ADN comme décrit précédemment (exemple 17 sur la détection de la phycoérythrine).

### Analyse sur gel

L'analyse a été effectuée sur gel dénaturant de polyacrylamide (8 % polyacrylamide, 7 M urée, 1 X TBE) utilisant coloration au bromure d'éthidium.

L'analyse sur gel montre que les amplicons ADN sont fragmentés par sonication à 60°C.

### Résultats

**Tableau 22 : Fragmentation de l'ADN double brin par sonication**

| **Conditions** | **Homologie (%)** | **I (rfu)** | **B (rfu)** | **I/B** |
|---|---|---|---|---|
| *a. Marquage pendant la fragmentation par sonication* | 93,8 | 2271 | 631 | 3,6 |
| *b. Marquage pendant la fragmentation chimique (conditions de référence)* | >95 | 19639 | 1459 | 13,5 |

Les résultats de marquage du tableau 10 pendant la sonication (conditions a) sont satisfaisants. Ceci montre que la fragmentation physique par sonication des cibles ADN est compatible avec la chimie de marquage par des réactifs de marquage portant une fonction diazométhyle.

Les faibles résultats de marquage dans ce cas sont certainement dus au fait que le marqueur se dégrade sous l'effet des ultrasons. Les résultats avec la fragmentation par création de site abasique par action du pH acide sont meilleurs.

### Exemple 26 : Marquage, fragmentation et dénaturation de l'ADN en une étape :

Les amplicons ADN ont été préparés par amplification PCR selon le protocole décrit dans l'exemple 5.1.

Deux réactions de marquage ont été réalisées :

### a. Marquage, fragmentation et dénaturation à 95°C en une seule étape :

A 10 µL de PCR sont ajoutés 50 µL de tampon formiate de sodium pH3 (50 mM) et 2 µL du marqueur *méta*-BioPMDAM (100mM dans du DMSO anhydre). Le volume final est ajusté à 100 µL. La solution est ensuite incubée 30 min à 95°C. Dans ce cas, le mélangée réactionnel a été hybridé sur puce à ADN sans aucune purification préalable.

### b. Marquage et fragmentation à 60°C:

A 10 µL de PCR sont ajoutés 50 µL de tampon formiate de sodium pH3 (50 mM) et 2 µL du marqueur *méta*-Bio-DPDAM (3d) (100mM dans du DMSO anhydre). Le volume final est ajusté à 100 µL. La solution est ensuite incubée 30 min à 60°C. Le mélange réactionnel a été ensuite purifié selon le protocole décrit auparavant. Dans ce protocole et avant hybridation sur puce à ADN, les fragments ont été dénaturés selon le protocole décrit dans l'exemple 19.2.

### Résultats

**Tableau 23 : Marquage, fragmentation et dénaturation de l'ADN en une étape**

| **Protocole utilisé** | **Homologie (%)** | **I (rfu)** | **B (rfu)** | **I/B** |
|---|---|---|---|---|
| *a. Marquage, fragmentation et dénaturation à 95°C* | >95 | 5426 | 506 | 10,7 |
| *b. Marquage et fragmentation à 60°C puis dénaturation à 95°C* | >95 | 7015 | 818 | 6,8 |

L'exemple 20.2 a démontré l'importance de la dénaturation de l'ADN double brin pour la sensibilité de détection. Ces résultats du tableau 23 montrent qu'avec l'approche fragmentation par création de site abasique, le marquage, la fragmentation et la dénaturation de l'ADN peuvent être réalisées en une seule étape, ce qui représente une amélioration notable du point de vue de la simplicité et du temps pour l'utilisateur et cela sans affecter la sensibilité de détection.

### Exemple 27 : Synthèse d'autres dérivés PDAM comportant un bras de liaison à base de polyéthylène glycol :

### Exemple 27.1 : Synthèse du méta-Bio-EG3-PMDAM :

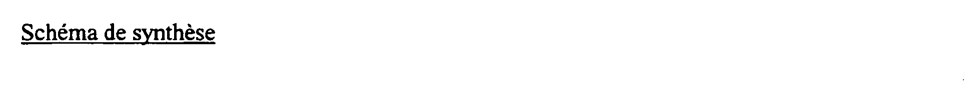

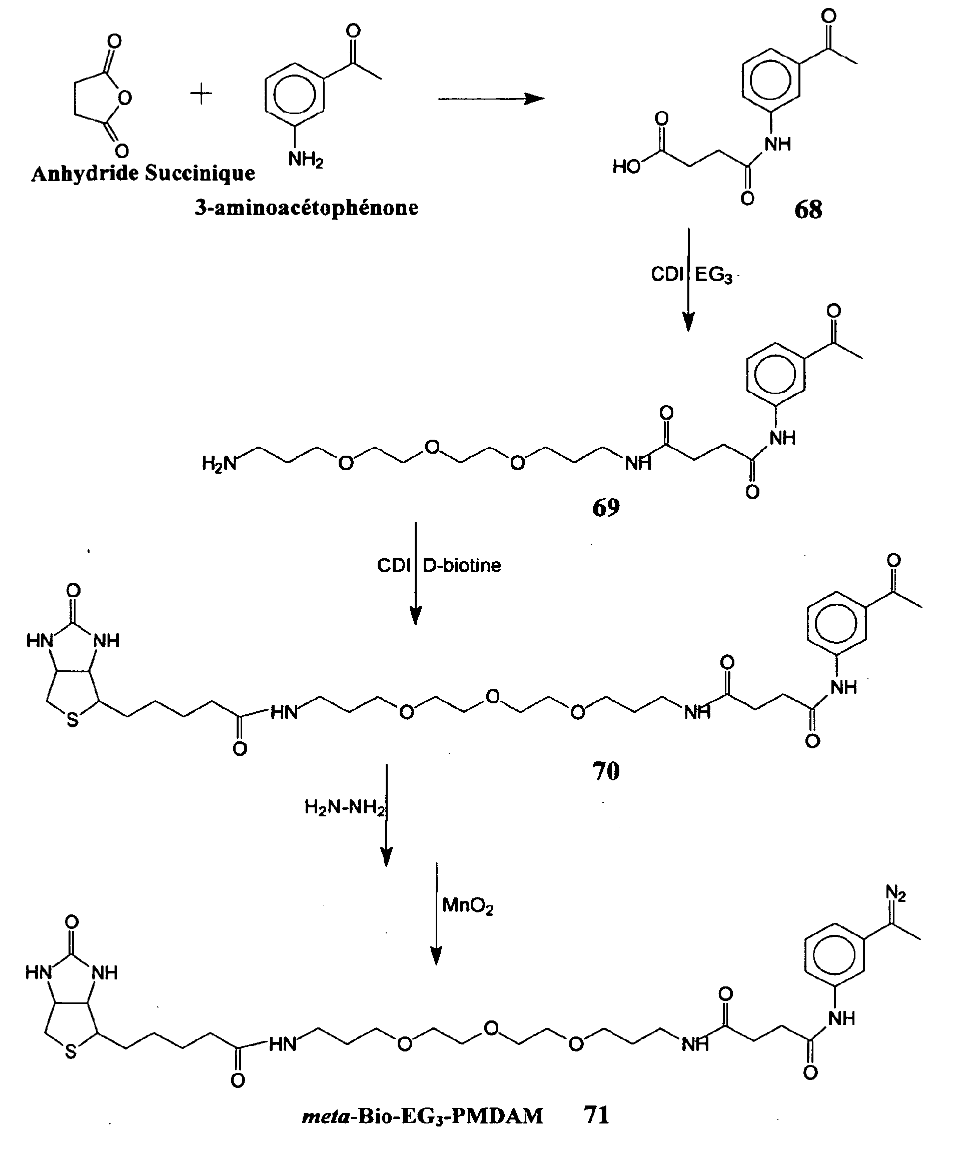

### Composé 68 :

On solubilise la 3-aminoacetophenone (14,5 g, 107 mmol) dans 50 ml de DMF anhydre. On ajoute l'anhydride succinique (10,7 g, 107 mmol) et on laisse sous agitation, sous argon et a température ambiante. Après 6 h, la solution est concentrée sous vide et 50 ml de méthanol sont ajoutés. Le précipité obtenu est filtré et lavé avec méthanol et éther. On obtient ainsi 19,4 g (81%) de produit **68** sous forme d'une poudre de couleur blanc cassé.
RMN ¹H (200 MHz, DMSO-d₆): δ = 2,5-2,6 (m, 7H); 7,45 (t, 1H); 7,64 (d, 1H); 7,83 (d, 1H); 8,19 (s, 1H); 10,16 (s, 1H); 12,12 (s, 1H).

### Composé 69 :

On solubilise 5,07 g (22 mmol) du composé **68** dans 10 ml de DMF anhydre, sous argon. On met sur glace et on ajoute 5,00 g (32 mmol) de carbonyldiimidazole. Apres 20 min, on ajoute lentement 20 ml (94,6 mmol) du 4, 7, 10-trioxatridecanediamine (EG₃). Après 3h de réaction à température ambiante, on évapore le DMF et reprend le résidu dans 100 ml de CH₂CL₂. On fait des extractions avec du NaHCO₃ saturé et H₂O, après quoi la phase organique est séchée avec du Na₂SO₄ anhydre et le solvant évaporé. On obtient ainsi 4,34 g (46%) du produit **69**.
RMN ¹H (200 MHz, DMSO-d₆): δ = 1,59 (m, 2H); 1,87 (m, 2H); 2,16 (s, 3H); 2,40 (m, 2H); 2,55 (m, 2H); 3,08 (m, 2H); 3,45 (m, 16H); 7,30 (t, 1H); 7,42 (d, 1H); 7,70 (d, 1H); 7,83 (t, 1H); 7,97 (s, 1H); 10,00 (s, 1H).

### Composé biotinylé 70 :

On solubilise la D-biotine (1,0 g, 4,1 mmol) dans 10 ml de DMF anhydre, sous argon. On refroidit sur glace et on ajoute le carbonyldiimidazole (CDI) (0,665 g, 4,1 mmol) dans 10 ml de DMF anhydre. Après 15 min, on ajoute le composé **69** (1,8 g, 4,1 mmol) dans 2 ml de DMF anhydre. On laisse réagir 3 h à 35°C, puis on évapore le DMF et on reprend dans 100ml de CH₂CL₂. On fait des extractions avec du NaHCO₃ saturé et H₂O, après quoi la phase organique est séchée avec du Na₂SO₄ anhydre et le solvant évaporé. La caractérisation par RMN du produit ainsi obtenu montre qu'on obtient un mélange du produit 70 et du EG₃ libre. Une autre étape de purification est effectuée avant de continuer la synthèse.

Le composé final, *méta*-Bio-EG₃-PMDAM, est obtenu après deux étapes de synthèse selon le schéma décrit dans l'exemple 1.

L'intérêt de cette synthèse est double. D'une part, on obtient le produit **69** en seulement deux étapes; ce produit peut être utilisé comme précurseur du diazo avec la possibilité d'y accrocher des molécules détectables de nature différente, grâce au groupement amine terminal. Ce groupement permet aussi de greffer le composé **69** sur des supports solides, avec l'objectif d'immobiliser des acides nucléiques. D'une autre part, le composé **71** possède le même centre réactif que la *méta*-Bio-PMDAM (notre molécule de référence), ce qui facilite l'analyse des avantages liés à l'inclusion du bras éthylèneglycol (EG₃).

Le réactif est stable à -20°C pendant au moins 1 mois.

### Exemple 27.2 : Synthèse du méta-Bio-EG4-PMDAM :

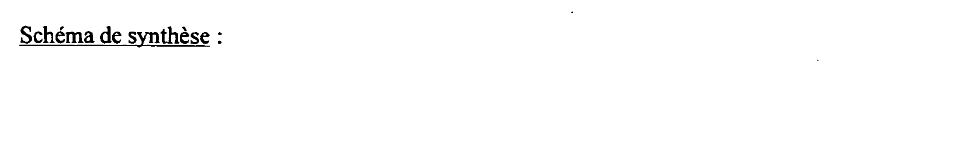

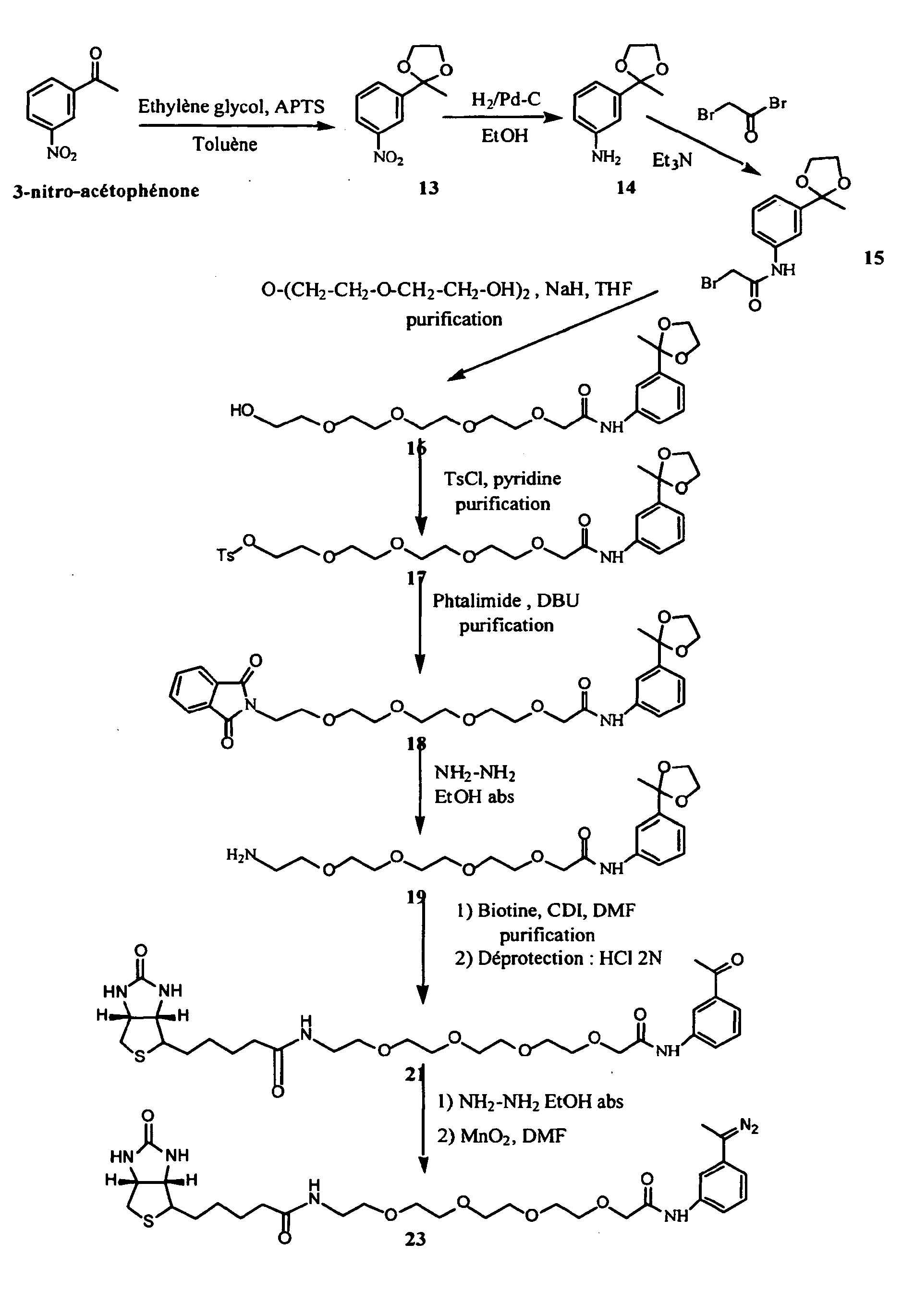

### • Protection de la 3-nitro-acétophenone 13 :

On dissout 33 g (0,20 mol) de 3-nitro-acétophénone dans 400 mL de toluène, puis on ajoute 40 mL (0,717 mol) d'éthylène glycol et 600 mg (3,15 mmol) d'acide *para*-toluène sulfonique (APTS). On monte un système Dean Stark. On chauffe la solution pendant 3 h à 130°C. Après avoir laissé la solution revenir à température ambiante, on ajoute 400 mL d'acétate d'éthyle, puis on lave celle-ci avec 8 mL d'une solution de NaHCO₃ saturée. On sèche la phase organique sur MgSO₄. Après évaporation, on obtient un solide jaune pâle 13 (39,72 g ; 0,190 mol) avec un rendement de 95 %.

RMN ¹H (200MHz, CDCl₃) δ =7,11 (t, 1H, *J* = 8 Hz, Ar-H) ; 6,87-6,78 (m, 2H, Ar-H) ; 6,59 (dd, 1H, *J* = 6,5 Hz, Ar-H) ; 4,00 (m, 2H, H₂C_{acétale}) ; 3,79 (m, 2H, H₂Cacétale) ; 1,61 (s, 3H, CH₃).

### • Préparation de l'amine 14 :

Le composé 13 (39,7 g ; 0,190 mol) est dissous dans 500 mL d'EtOH, puis 1 g de palladium sur charbon 10 % est ajouté. On chauffe pour tout dissoudre, puis on laisse la solution revenir à température ambiante. Après avoir fait le vide et mis la solution sous H₂, on laisse sous forte agitation pendant 5 h. La solution est ensuite filtrée à chaud puis évaporée. Le produit **14** est lavé au pentane, et isolé sous forme d'un solide (34 g ; 0,189 mol) avec un rendement de 99 %.

RMN ¹H (200MHz, CDCl₃) δ =7,14 (t, 1H, *J* = 8 Hz, Ar-H) ; 6,85 (m, 2H, *J* = 7,5 Hz, Ar-H) ; 6,79 (s, 1H, Ar-H) ; 6,59 (dd, 1H, *J* = 6,5 Hz, Ar-H) ; 4,00 (m, 2H, H₂C_{acétale}) ; 3,77 (m, 2H, H₂C_{acétale}) ; 1,61 (s, 3H, CH₃).

### • Composé bromé 15 :

L'amine **14** (12,3 g ; 68,7 mmol) et la triéthylamine (7 g ; 69 mmol) sont mises en solution dans 150 mL de DCM sous argon. On ajoute à -5 °C au goutte-à-goutte une solution de 13,8 g (60 mmol) de bromure de bromoacétyle dissous dans 150 mL de DCM. À la fin de l'addition, on ajoute 100 mL de NaHCO₃ aqueux 1 N. On lave la phase organique deux fois de suite avec NaHCO₃ aqueux et on sèche sur MgSO₄. Après évaporation à sec, 22,6 g d'huile marron est obtenue correspondant au composé **15** et utilisée telle quelle pour la réaction suivante.

RMN ¹H (200MHz, CDCl₃) δ = 8,29 (s large, 1H, NH) ; 7,62 (dt, 1H₄, *J* = 5 Hz, Ar-H) ; 7,47 (s, 1H, Ar-H₂) ; 7,38-7,19 (m, 2H, Ar-H₅,₆) ; 4,00 (m, 2H, Br-CH₂) ; 3,75 (m, 4H, H₂C-H₂C_{acétale}) ; 1,61 (s, 3H, CH₃).

### • Composé alcool 16 :

L'hydrure de sodium (3,3 g ; 82,5 mmol) est lavé trois fois avec du pentane puis mis en suspension dans 150 mL de THF, le tétra éthylène glycol (50 mL ; 0,29 mol) est alors ajouté à température ambiante. On laisse la réaction sous agitation pendant 15 min, puis on refroidit la solution à -5°C.

L'ajout du composé **15** préalablement dilué dans 25 mL de THF se fait au goutte-à-goutte. On laisse sous agitation pendant 30 min pour laisser revenir à température ambiante. On concentre la solution à 100 mL, puis on dilue celle-ci avec 500 mL de CHCl₃. On lave trois fois de suite cette phase organique avec 250 mL de NaHCO₃ aqueux 1 N, puis on la sèche sur MgSO₄ avant de l'évaporer. On purifie le produit sur colonne de silice par chromatographie-flash (colonne de 65 mm de diamètre) avec 1,5 L de MeOH-DCM 5 %, puis avec 500 mL de MeOH-DCM 7%, et enfin 500 mL de MeOH-DCM 10 %. Les fractions correspondant au composé 16 sont rassemblées, puis évaporées à sec pour donner 17,4 g (42,1 mmol) de produit, avec un rendement de 61 %.

RMN ¹H (200MHz, CDCl₃) δ = 8,86 (s large, 1H, NH) ; 7,71 (d, 1 H, *J* = 7,5 Hz, Ar-H₄) ; 7,51 (s, 1H, Ar-H₂) ; 7,29-7,24 (m, 2H, Ar-H₅,₆) ; 4,09 (m, 2H, CO-CH₂-O) ; 3,99 (m, 2H, H₂C_{acétale}) ; 3,72-3,53 (m, 20H, O-CH-CH₂-O, H₂C_{acétale} et HO-CH₂) 1,61 (s, 3H, CH₃).

### • Composé tosylate 17 :

L'alcool **16** (4,13 g;10,0 mmol) est mis en solution dans 5 mL de pyridine. On ajoute ensuite 2,0 g (10,5 mmol) de chlorure de tosyle à température ambiante. On met sous agitation sous argon pendant 10 h. On dilue avec 100 mL de DCM, on lave trois fois la phase organique avec 20 mL de NaHCO₃ aqueux 1 N, puis on la sèche sur MgSO₄ avant de la co-évaporer avec du toluène. Une purification sur colonne par chromatographie-flash (colonne de 50 mm de diamètre) est réalisée avec 500 mL de MeOH-DCM 2 %, puis 500 mL de MeOH-DCM 3 %, et enfin 500 mL de MeOH-DCM 4 %. Les fractions correspondant au produit **17** sont rassemblées puis évaporées à sec pour donner 3,68 g (6,48 mmol) d'huile avec un rendement estimé à 65 %.

RMN ¹H (300MHz, CDCl₃) δ = 8,86 (s large, 1H, NH) ; 7,76 (d, 4H, *J* = 5,5 Hz, Ar-H_{tosyl}) ; 7,60 (d, 1H, *J* = 7,5 Hz, Ar-H₄) ; 7,50 (s, 1H, Ar-H₂) ; 7,32-7,22 (m, 2H, Ar-H₅,₆) ; 4,10 (m, 2H, CO-CH₂-O) ; 4,00 (m, 2H, H₂C_{acétale}) ; 3,73-3,54 (m, 20H, O-CH₂-CH₂-O, H₂C_{aétale}, et HO-CH₂); 2,42 (s, 3H, Ar-CH₃) ; 1,61 (s, 3H, CH₃).

### • Composé phtalimide 18 :

On met le tosylate **17** (3,68 g ; 6,48 mmol) en solution avec 1,52 g (10,0 mmol) de DBU (1,8-diazobicyclo-[5.4.0]-undecène) puis on ajoute le phtalimide (1,47 g ; 10 mmol). La solution ainsi obtenue est chauffée à 85-90°C pendant 17 h puis évaporée. On purifie le produit sur colonne de silice par chromatographie-flash (colonne de 50 mm de diamètre) avec 1 L d'acétone-DCM 15 %, puis avec 1 L d'acétone-DCM 20 %. Les fractions correspondant au composé **18** sont rassemblées, puis évaporées à sec pour donner 3,15 g (5,8 mmol) de produit, avec un rendement de 90 %.

RMN ¹H (200MHz, CDCl₃) δ = 8,73 (s large, 1H, NH) ; 7,79 (m, 2H, Ar-Hₚₕₜₐ) ; 7,99 (m, 2H, Ar-Hₚₕₜₐ et Ar-H₄) ; 7,49 (s, 1H, Ar-H₂) ; 7,27-7,18 (m, 2H, Ar-H₅,₆) ; 4,10 (m, 2H, CO-CH₂-O) ; 4,00 (m, 2H, H₂C_{acétale}) ; 3,69-3,56 (m, 20H, O-CH₂-CH₂-O,H₂C_{acétale} et Nₚₕₜₐ-CH₂) ; 1,61 (s, 3H, CH₃).

### • Composé amine 19 :

Le produit **18** est dissous dans 20 mL d'EtOH absolu en chauffant à reflux à 75-80°C. L'hydrazine (1,07 mL ; 22,1 mmol) est ensuite ajoutée, et la réaction est laissée sous agitation pendant 1 h 15. Le précipité obtenu est filtré sur fritté et la phase éthanolique évaporée. Le précipité blanc est ensuite lavé au DCM, et la phase DCM est évaporée. L'huile jaune obtenue (2,3 g ; 5,57 mmol) est directement utilisée pour la réaction suivante, même si elle contient de l'imidazole qui pourra être éliminé par la suite, lors de l'étape de déprotection de l'acétal.

RMN ¹H (300MHz, CDCl₃) δ = 8,83 (s large, 1H, NH) ; 7,69 (d, 1H, *J* = 7,5 Hz, Ar-H₄) ; 7,51 (s, 1H, Ar-H₂); 7,30-7,19 (m, 2H, Ar-H₅,₆) ; 4,10 (m, 2H, CO-CH₂-O) ; 4,00 (m, 2H, H₂C_{acétale}) ; 3,69-3,56 (m, 20H, O-CH₂-CH₂-O, H₂C_{acétale} et H₂N-CH₂) ; 1,61 (s, 3H, CH₃).

### • Composé biotinylé 20 :

On solubilise la D-Biotine (1,05 g ; 4,32 mmol) dans 10 mL de DMF anhydre. On ajoute sous argon 790 mg (4,87 mmol) de carbonyle diimidazole (CDI). Après 10 min d'agitation, on ajoute l'amine **19** diluée dans 5 mL de DMF. La solution est laissée 40 min sous agitation, puis évaporée avant d'être purifiée sur colonne par chromatographie-flash.

Pour ceci on utilise une colonne de 50 mm de diamètre avec comme éluant 500 mL de MeOH-DCM 5 %, puis 500 mL de MeOH-DCM 10 %, et enfin 500 mL de MeOH-DCM 15 %. Les fractions correspondant au produit **20** sont rassemblées puis évaporées à sec pour donner une huile jaune (1,66 g ; 2,6 mmol).

L'huile jaune obtenue (2,4 g) contenant d'après le spectre RMN environ 30 % en poids d'imidazole. On en déduit donc que le rendement de la réaction aboutissant au produit **20** est d'environ 60 % par rapport à la biotine de départ.

RMN ¹H (300MHz, CDCl₃) δ = 8,80 (s large, 1H, NH) ; 7,66 (m, 3H, Ar-H₄ et H_{imidazole}) ; 7,54 (s, 1H, Ar-H₂) ; 7,28-7,24 (m, 2H, Ar-H₅,₆) ; 7,07 (s, 2H, H_{imidazole}), 6,59 (t, 1H, NH₁₅); 6,06 (s large, 1H, NH_{B1}) ; 5,19 (s large, 1H, NH_{B3}) ; 4,45 (m, 1H, H_{B6a}) ; 4,27 (m, 1H, H_{B3a}) ; 4,10 (s, 2H, H_{3'}) ; 4,00 (m, 2H, H₂C_{acétale}) ; 3,75-3,49 (m, 18H, O-CH₂-CH₂-O et H₂C_{acétale}) ; 3,36 (m, 2H, H_{14'}) ; 3,09 (m, 1H, H_{B4}) ; 2,85 et 2,66 (système ABX, 2H, ²*J*_{AB}= 5 Hz, ³*J*_{AX} = 12 Hz, ³*J*_{BX} = 0 Hz, H_{B6}) ; 2,16 (t, 2H, *J* = 8 Hz, H_{B10}) ; 1,61 (s, 3H, CH₃) ; 1,59-1,3 (m, 6H, H_{B9}, _{B8, B7}).

### • Composé cétone 21 :

L'acétal **20** est dissous dans 80 mL de chloroforme, puis 30 mL de HCI 2 N sont ajoutés. On laisse sous forte agitation pendant 45 min. La phase organique est récupérée puis séchée sur NaHCO₃ anhydre. Après filtration, la solution est évaporée et l'huile obtenue est lavée au pentane pour donner le produit **21** (1,48 g ; 2,48 mmol) avec un rendement de 99 %.

RMN ¹H (300MHz, CDCl₃) δ = 8,99 (s large, 1H, NH) ; 8,07 (s, 1H, Ar-H₂) ; 7,98 (d, 2H, *J* = 8 Hz, Ar-H₄) ; 7,66 (d, 2H, *J* = 8 Hz, Ar-H₆) ; 7,42 (t, 2H, *J* = 8 Hz, Ar-H₅) ; 6,38 (t, 1H, NH_{15'}) ; 5,78 (s large, 1H, NH_{B1}) ; 4,96 (s large, 1H, NH_{B3}) ; 4,47 (m, 1H, H_{B6a}) ; 4,29 (m, 1H, H_{B3a}) ; 4,13 (s, 2H, H_{3'}) ; 3,76-3,37 (m, 16H, O-CH₂CH₂-O) ; 3,32 (m, 2H, H_{14'}) ; 3,11 (m, 1H, H_{B4}) ; 2,89 et 2,75 (système ABX, 2H, ²*J*_{AB}= 5 Hz, ³*J*_{AX} = 12 Hz, ³*J*_{BX} = 0 Hz, H_{B6}); 2,59 (s, 3H, CH₃) ; 2,16 (t, 2H, *J* = 8 Hz, H_{B10}) ; 1,64-1,40 (m, 6H, H_{B9, B8, B7}).

### • Composé hydrazone 22 :

La cétone **21** est dissoute dans 20 mL d'EtOH absolu. On chauffe à reflux à 75 -80°C. On ajoute ensuite l'hydrazine (816 µL ; 16,81 mmol) et on laisse sous agitation pendant 3 h. Après filtration, on évapore à sec, on redissout dans l'éthanol jusqu'à l'obtention d'une mousse blanche collante. Dans un deuxième temps, on dissout cette mousse dans 50 mL de chloroforme puis on ajoute 20 mL d'une solution de NaHCO₃ saturée. On lave bien puis on récupère la phase organique. On la sèche sur Na₂CO₃ anhydre et après filtration, on évapore à sec afin d'obtenir une nouvelle mousse collante. Celle-ci correspond au produit **22** (842 mg g ; 1,38 mmol) et est obtenue avec un rendement de 66 %.

RMN ¹H (300MHz, CDCl₃) δ = 8,81 (s large, 1H, NH) ; 8,82 (s, 1H, Ar-H₂) ; 7,64 (d, 2H, *J* = 8 Hz, Ar-H₄) ; 7,32 (m, 4H, Ar-H₅,₆) ; 6,43 (t, 1H, NH_{15'}) ; 5,89 (s large, 1 H, NH_{B1}) ; 5,46 (s large, 2H, NH₂) ; 4,99 (s large, 1H, NH_{B3}) ; 4,44 (m, 1 H, H_{B6a}) 4,27 (m, 1H, H_{B3a}) ; 4,11 (s, 2H, H_{3'}); 3,70-3,37 (m, 16H, O-CH₂-CH₂-O) ; 3,32 (m, 2H, H_{14'}); 3,08 (m, 1H, H_{B4}) ; 2,87 et 2,67 (système ABX, 2H, ²*J*_{AB}*=* 5 Hz, ³*J*_{AX} = 12 Hz, ³*J*_{BX} = 0 Hz, H_{B6}) 2,11 (m, 5H, CH₃ et H_{B10}); 1,64-1,40 (m, 6H, H_{B9, B8, B7}).

### • Composé diazo 23 :

L'hydrazone **22** (100mg ; 0,164 mmol) est dissoute dans 1 mL de DMF sous argon. On ajoute 80 mg de MnO₂ activé et on laisse sous forte agitation pendant 30 min. Le mélange est filtré à travers une couche mixte Célite (3 cm) - tamis moléculaire en poudre (1 cm). La solution est ensuite évaporée à sec. L'huile obtenue en fin d'évaporation est triturée jusqu'à l'obtention d'un poudre rose correspondant au composé **23** (78 mg ; 0,128 mmol ; 78 %).

RMN ¹H (300MHz, DMSO-d₆) δ = 9,60 (s large, 1H, NH) ; 7,89 (s, 1H, Ar-H₂) ; 7,76 (t, 1H, NH_{15'}) ; 7,35-7,25 (m, 4H, Ar-H₅,₆) ; 6,64 (d, 2H, *J* = 8 Hz, Ar-H₄) ; 6,36 (s large, 1H, NH_{B1}) ; 6,32 (s large, 1H, NH_{B3}) ; 4,28 (m, 1H, H_{B6a}) ; 4,08 (m, 1H, H_{B3a}) ; 4,06 (s, 2H, H_{3'}) ; 3,55-3,31 (m, 16H, O-CH₂-CH₂-O) ; 3,17 (m, 2H, H_{14'}) ; 3,08 (m, 1H, H_{B4}) ; 2,80 et 2,59 (système ABX, 2H, ²*J*_{AB}= 5 Hz, ³*J*_{AX} = 12 Hz, ³*J*_{BX} = 0 Hz, H_{B6}) ; 2,13 (m, 5H, CH₃) ; 2,13 (t, 2H, *J* = 8 Hz, H_{B10}) ; 1,99-1,30 (m, 6H, H_{B9}, _{B8}, _{B7}).

La réactivité du composé 23 a été testée sur 3'-Uridine-monophosphate et suivie par électrophorèse capillaire. Les conditions d'analyse sont celles de l'exemple 6.1. Les résultats montrent un temps de demi réaction de 30 minutes.

La stabilité du réactif est supérieure à 1 mois à -20°C.

### Exemple 27.3 : Synthèse du méta-Bio-EG3-PDAM :

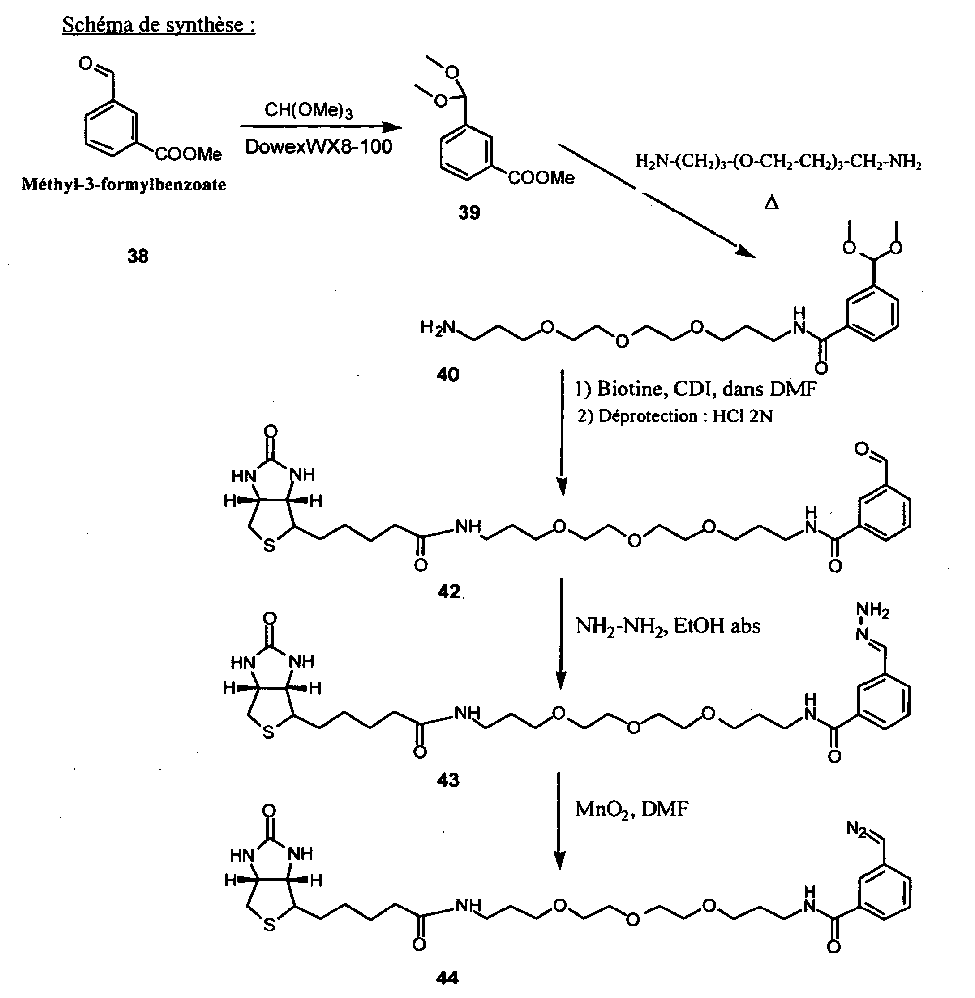

### • Protection du méthyl-3-formylbenzoate 38 :

La résine Dowex 50WX8-100 (2 g) est mise en solution dans 25 mL de MeOH et 25 mL d'orthoformiate de triméthyle puis laissé sous agitation pendant 15 min. Après décantation, on lave la résine deux fois de suite avec 20 mL de MeOH. On met ensuite cette résine dans 100 mL de MeOH, 50 mL de CH(OMe)₃ et on ajoute 7,12 g (43,4 mmol) de méthyl-3-formylbenzoate. La solution est laissée 15 min sous agitation, puis filtrée sur papier plissé avant évaporation. Le produit **39** (9 g ; 43,1 mmol) est isolé sous forme d'un liquide jaune clair avec un rendement de 99 %.

RMN ¹H (200MHz, CDCl₃) δ = 8,10 (s, H, Ar-H₂) ;7,9 (d, H, *J* = 8 Hz, Ar-H₄) ; 7,63 (d, H, *J* = 8 Hz, Ar-H₆) ; 7,42 (t, H, *J* = 8 Hz, Ar-H₅) ; 5,40 (s, 1H, CH) ; 3,90 (s, 3H, -CO-O-CH₃) ; 3,31 (s, 6H, -O-CH₃).

### • Composé 40 :

On solubilise le composé **39** (2 g ; 9,5 mmol) dans 10,4 mL (47,6 mmol) de 4,7,10-Trioxa-1,13-tridécanediamine. La solution obtenue est chauffée à 165°C pendant 2 h. Le mélange est ensuite dissous dans 80 mL de DCM et lavé 4 fois avec 20 mL d'eau. Après séchage sur MgSO₄ et évaporation, le produit **40** est isolé avec un rendement de 60 % (2,27 g ; 5,69 mmol).

RMN ¹H (200MHz, CDCl₃) δ = 7,84 (s, H, Ar-H₂) ;7,75 (d, H, *J* = 8 Hz, Ar-H₄) ; 7,53 (d, H, *J* = 8 Hz, Ar-H₆) ; 7,39 (t, H, *J* = 8 Hz, Ar-H₅) ; 5,38 (s, 1H, CH); 3,64-3,43 (m, 14H, H_{7',8',10',11'} et H_{5',13'} et H_{3'}) ; 3,29 (s, 6H, -O-CH₃) ; 2,72 (m, 2H, H_{15'}) 1,87 (m, 2H, H_{4'}) ; 1,64 (m, 2H, H_{14'}) ; 1,30 (s large, 2H, NH₂).

### • Composé biotinylé 41 :

La D-biotine (344 mg ; 1,40 mmol) est mise en suspension dans 4 mL de DMF puis 250 mg (1,54 mmol) de CDI sont ajoutés. Cette solution est laissée sous agitation pendant 30 min à température ambiante. Le composé **40** (616 mg ; 1,54 mmol) est dissous dans 2 mL de DMF, puis ajouté petit à petit à la solution précédente. Le mélange ainsi obtenu est laissé sous agitation pendant 50 min à température ambiante. Après évaporation, une purification sur colonne par chromatographie-flash (colonne de 30 mm de diamètre) est réalisée avec 750 mL de MeOH-DCM 10 %, puis avec 250 mL de MeOH-DCM 15 %. Les fractions correspondant au produit **41** sont rassemblées puis évaporées à sec pour donner 740 mg d'huile avec un rendement estimé à 50 %.

RMN ¹H (200MHz, CDCl₃) δ = 7,87 (s, H, Ar-H₂) ;7,78 (d, H, *J* = 8 Hz, Ar-H₄) ; 7,65 (s, 1H, H_{imidamle}) ; 7,53 (d, H, *J =* 8 Hz, Ar-H₆) ; 7,39 (t, H, *J* = 8 Hz, Ar-H₅) ; 7,07 (s, 2H, H_{imidazole}) ; 6,65 (t, 1H, NH_{16'}), 5,95 (s large, 1H, NH_{B1}) ; 5,38 (s, 1H, CH) ; 5,15 (s large, 1H, NH_{B3}) ; 4,43 (m, 1H, H_{B6a}) ; 4,27 (m, 1H, H_{B3a}) ; 3,59-3,44 (m, 14H, H_{7',8',10',11'} et H_{5',13'} et H_{3'}) ; 3,29 (m, 8H, H_{15'} et 2-O-CH₃) ;3,07 (m, 1H, H_{B4}); 2,84 et 2,66 (système ABX, 2H, ²*J*_{AB} = 5 Hz, ³*J*_{AX} = 12 Hz, ³*J*_{BX} = 0 Hz, H_{B6}) ; 2,13 (t, 2H, *J* = 8 Hz, H_{B10}) ;1,85 (m, 2H, H_{4'}) ; 1,66 (m, 2H, H_{14'}) ; 1,40-1,37 (m, 6H, H_{B7}, _{B8}, _{B9}).

### • Composé aldéhydique 42 :

L'acétal **41** est dissous dans 20 mL de chloroforme, puis 5 mL de HCI 2N sont ajoutés . Le mélange diphasique est vigoureusement agité pendant 15 min. On récupère la phase organique qu'on sèche sur NaHCO₃ anhydre. On filtre, on évapore et le composé **42** est obtenu sous forme d'une huile jaune (593 mg ; 1,02 mmol) avec un rendement global de 87 % à partir de la biotine.

RMN ¹H (300MHz, CDCl₃) δ = 10,04 (s, 1H, CHO) ;8,34 (s, H, Ar-H₂) ; 8,16 (d, H, *J* = 8 Hz, Ar-H₄) ; 7,96 (d, H, *J* = 8 Hz, Ar-H₆) ; 7,72 (t, 1H, NH_{2'}) ; 7,39 (t, H, *J* = 8 Hz, Ar-H₅) ; 6,51 (t, 1 H, NH_{16'}) ; 6,00 (s large, 1 H, NH_{B1}) ; 5,30 (s large, 1H, NH_{B3}) ; 4,46 (m, 1H, H_{B6a}) ; 4,27 (m, 1H, H_{B3a}) ; 3,66-3,56 (m, 10H, H_{7', 8',10',11'} et H_{5'}) ; 3,50-3,29 (m, 4H, H_{3',13'}) ; 3,28 (m, 2H, H_{15'}) ; 2,95 (m, 1H, H_{B4}); 2,84 et 2,71 (système ABX, 2H, ²*J*_{AB}= 5 Hz, ³*J*_{AX} = 12 Hz, ³*J*_{BX} = 0 Hz, H_{B6}) ; 2,15 (t, 2H, *J* = 8 Hz, H_{B10}) ; 1,89 (m, 2H, H_{4'}) ; 1,72-1,63 (m, 6H, H_{14'}, H_{B7}, _{B9}) ; 1, 23 (m, 2H, H_{B8}).

### • Composé hydrazone 43 :

L'aldéhyde **42** (593 mg ; 1,02 mmol) est dissous dans 10 mL d'éthanol absolu. L'hydrazine (400 µL ; 8,19 mmol) est ajoutée, puis le mélange réactionnel est chauffé à reflux pendant 50 min. L'huile jaune obtenue après évaporation, est triturée avec de l'éther jusqu'à l'obtention d'une poudre beige correspondant au produit **43** (404 mg ; 0,68 mmol) avec un rendement de 66 %. Une purification sur colonne par chromatographie-flash (colonne de 15 mm de diamètre) est réalisée ensuite sur un échantillon de 150 mg (0,253 mmol) avec 200 mL de MeOH-DCM 20% . Les fractions sont rassemblées puis évaporées à sec pour donner 144 mg de produit **43** avec un rendement de 76 %.

RMN ¹H (300MHz, CDCl₃) δ = 7,95 (s, H, Ar-H₂) ; 8,16 (d, H, *J* = 8 Hz, Ar-H₄) ; 7,76 (s, 1H, CH) ; 7,96 (d, H, *J=* 8 Hz, Ar-H₆) ; 7,38 (t, H, *J=* 8 Hz, Ar-H₅) ; 6,45 (t, 1H, NH_{16'}) ; 5,98 (s large, 1H, NH_{B1}) ; 5,72 (s large, 2H, NH₂) ; 5,18 (s large, 1H, NH_{B3}) ; 4,44 (m, 1H, H_{B6a}) ; 4,26 (m, 1H, H_{B3a}) ; 3,62-3,56 (m, 10H, H_{7', 8',10',11'} et H_{5'}) ; 3,48-3,45 (m, 4H, H_{3',13'}) ; 3,27 (m, 2H, H_{15'}) ; 3,07 (m, 1H, H_{B4}) ; 2,84 et 2,68 (système ABX, 2H, ²*J*_{AB}= 5 Hz, ³*J*_{AX} = 12 Hz, ³*J*_{Bx} = 0 Hz, H_{B6}) ; 2,11 (t, 2H, *J* = 8 Hz, H_{B10}) ; 1,86 (m, 2H, H_{4'}) ; 1,72-1,59 (m, 6H, H_{14'}, H_{B7}, _{B9}) ; 1, 21 (m, 2H, H_{B8}).

### • Composé diazo 44 :

On solubilise l'hydrazone **43** (100 mg ; 0,187 mmol) dans 4 mL de DMF. On ajoute alors MnO₂ (200 mg ; 2,3 mmol). Après 13 min d'agitation à température ordinaire, le mélange est filtré sur millipore contenant de la célite (épaisseur : 2 cm) et du tamis moléculaire en poudre 3 Å (0,5 cm). Le mélange réactionnel est évaporé à sec. L'huile résiduelle obtenue est lavée à l'éther, trois fois de suite. Le composé **44** (290 mg, 0,491 mmol) est obtenu sous forme d'un solide orange avec un rendement de 83 %.

RMN ¹H (300MHz, DMSO-d₆) δ = 8,39 (t, 1H, NH_{2'}) ; 7,78 (t, 1H, NH_{16'}) ; 7,39-7,34 (m, Ar-H) ; 7,09 (d, Ar-H) ; 6,38 (s large, 1H, NH_{B1}) ; 6,32 (s large, 1H, NH_{B3}) ; 5,78 (s, 1H, CH-N₂) ; 4,27 (m, 1H, H_{B6a}) ; 4,11 (m, 1H, H_{B3a}) ; 3,51-3,44 (m, 10H, H_{7', 8', 10',11'} et H_{5'}); 3,37 (m, 2H, H_{15'}) ; 3,32 (m, 4H, H_{3',13'}) ; 3,05 (m, 1H, H_{B4}); 2,79 et 2,58 (système ABX, 2H, ²*J*_{AB}= 5 Hz, ³*J*_{Ax} = 12 Hz, ³*J*_{Bx} = 0 Hz, H_{B6}) ; 2,02 (t, 2H, *J* = 8 Hz, H_{B10}) ;1,69 (m, 2H, H_{4'}) ; 1,59-1,48 (m, 6H, H_{14'}, H_{B7}, _{B9}) ; 1, 25 (m, 2H, H_{B8}).

La stabilité du produit est supérieure à 1 mois à -20°C.

### Exemple 28 : Synthèse du méta-Fluo-EG3-PMDAM :

Comme cela a déjà été évoqué dans les exemples 22 et 24, la biotine peut être remplacée par un autre marqueur tel que la fluorescéine. Cet exemple montre que l'on peut également relier la fonction diazo, portée par le PMDAM, à ce marqueur fluorescéine par l'intermédiaire d'un bras de liaison polyéthylène glycol.

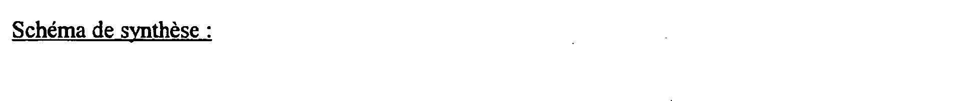

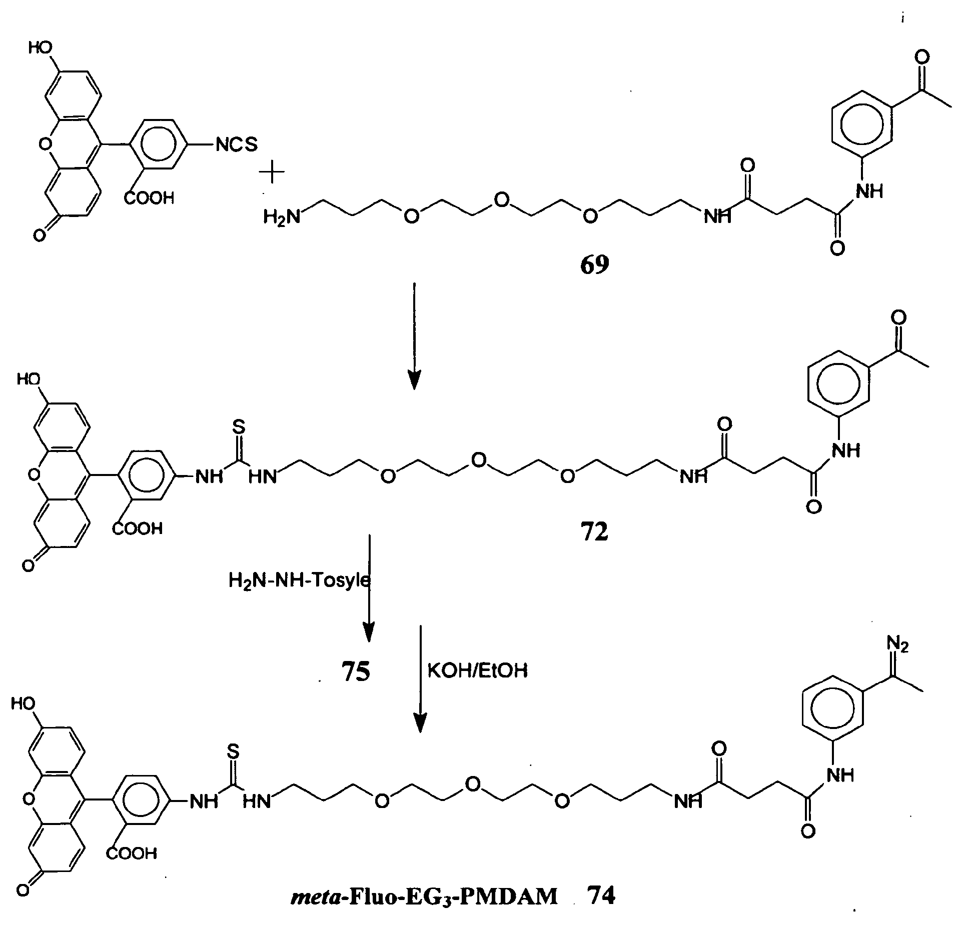

### Composé 72 :

On solubilise l'isothiocyanate de fluorescéine- (250 mg, 0,64 mmol) dans 1,6 ml de DMF anhydre, avec 2% de pyridine, sous argon. On ajoute le produit **69** (0,356 g, 0,81 mmol) dissout dans 1,6 ml de DMF anhydre. On laisse réagir 3,5 h à température ambiante, puis on évapore le DMF et on reprend dans 25 ml de H₂O. On fait trois extractions avec 50 ml de CH₂Cl₂, et la phase aqueuse est évaporée. On obtient 255 mg (48%) du produit **72**.

### Composé méta-Fluo-EG₃-Hydrazone-Tosyle 75 :

On dissout le composé **72** (255 mg, 0,31 mmol) dans 1,5 ml d'éthanol à reflux. On ajoute la *p-*toluen-sulfonyl-hydrazine (69,2 mg, 0,37 mmol) dans 1,5 ml d'éthanol et on laisse réagir pendant 6 h. On évapore a sec, et on lave le solide avec CH₂Cl₂, H₂O et éther. On obtient 18,5 mg (74%) du produit **75** sous forme d'une poudre orange.

RMN ¹H (200 MHz, DMSO-d₆): δ = 1,6-1,8 (m, 4H); 2,13 (s, 1H); 2,28 (s, 1H); 2,36 (s, 1H); 2,80 (m, 1H); 3,07 (m, 2H); 3,46 (m, 12H); 6,5-6,7 (m, 6H); 7,1-8,3 (m, 9H).

### Composé méta-Fluo-EG₃-PMDAM 74 :

On solubilise l'hydrazone **75** (176 mg, 0,18 mmol) dans 720 µl d'une solution de KOH à 10% dans du méthanol anhydre. La solution est laissée a reflux pendant 3 h. On laisse refroidir la solution et un précipité apparaît. La solution est filtrée et évaporée a sec. Le résidu est lavé a l'éther et séché.

L'analyse par RMN montre la disparition des signaux a 2,36 et 2,13 ppm (correspondant aux Méthyles du tosyle et de l'hydrazone) et l'apparition d'un pic à 1,96 ppm (correspondant au méthyle du diazo).

### Exemple 29: Intermédiaire diazométhyle permettant un marquage ultérieur :

Il peut être intéressant d'avoir non pas un marquage direct par le réactif de marquage diazométhyle portant aussi le marqueur R² mais de procéder en deux étapes avec un marquage indirect. Dans ce cas le réactif de marquage comprenant la fonction diazométhyle est dit préfonctionnalisé c'est à dire qu'il comprend aussi une fonction chimique apte à réagir ultérieurement avec des marqueurs directs ou indirects. La pré-fonctionnalisation peut intervenir par introduction d'une fonction réactive de covalence au sein du réactif de marquage qui peut réagir avec une fonction anti-réactive de covalence du marqueur direct ou indirect. Ces fonctions peuvent être constituées par une fonction chimique organique électrophile et une fonction chimique organique nucléophile, ou inversement.

Un exemple d'une telle stratégie de marquage est illustrée par le schéma ci-dessous

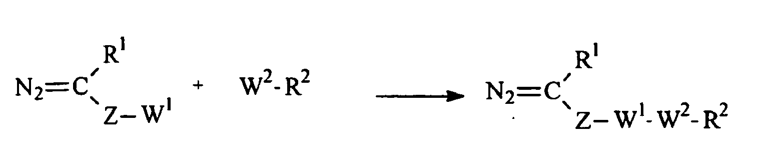

Dans lequel le réactif de marquage comprend, outre une fonction diazométhyle, une fonction W¹ électrophile ou nucléophile susceptible de réagir avec un marqueur R² comprenant une fonction W² complémentaire de W¹.

Par exemple si W¹ est une fonction méthylcétone ou aldéhyde, W² est une fonction alcoxyamine.

Dans un procédé de marquage d'une molécule biologique comme un acide nucléique, l'acide nucléique est mis en contact avec le réactif de marquage coprenant la fonction diazométhyle et dans une étape ultérieure, le marqueur W²- R² réagit avec l'acide nucléique par l'intermédiaire de la fonction W¹.

L'une des utilisations est par exemple constituée par un procédé d'amplification d'une séquence d'un acide nucléique ou par un procédé d'amplification du signal. Des informations complémentaires sur ce type de marquage peuvent être trouvées dans la demande de brevet WO-A-98/05766, sous priorité du 2 août 1996. et dans la demande de brevet WO-A-00/40590 sous priorité du 05 Janvier 1999.

### Exemple 29.1 : Synthèse du MeCO-PMDAM:

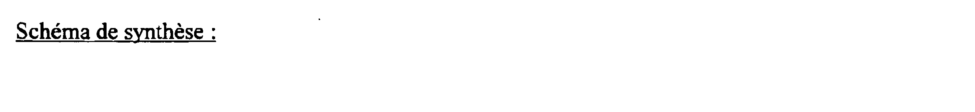

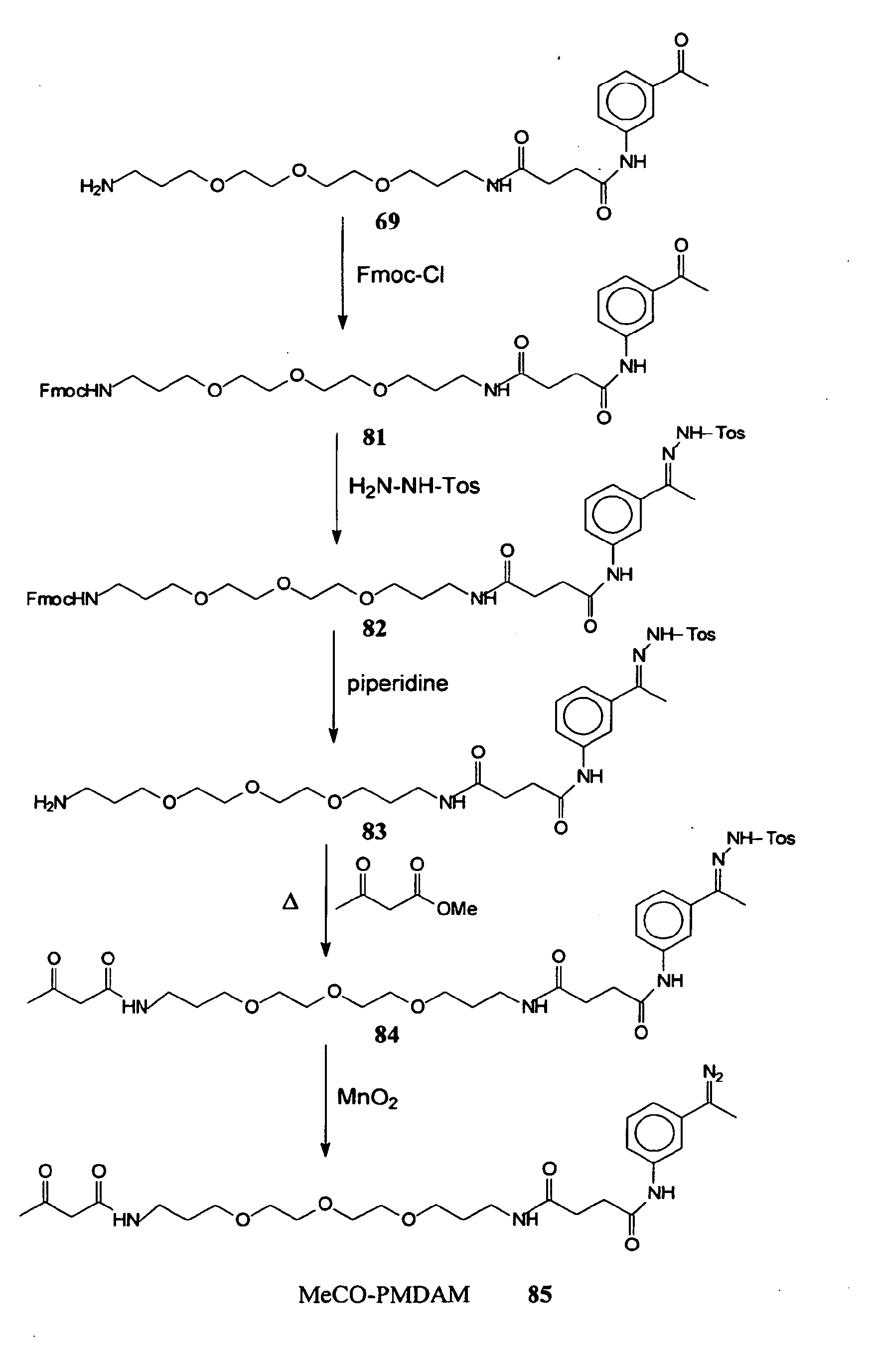

Le produit **85,** dont la synthèse est décrite dans cet exemple, permet de réaliser le marquage des acides nucléiques naturels, grâce à la réactivité de la fonction diazométhyl avec les groupements phosphate, et d'introduire ainsi une fonction méthylcétone, qui peut être utilisée par la suite pour introduire une molécule détectable (fluorescente, biotine) possédant un groupement alcoxyamine.

Cette synthèse est basée sur des méthodes connues et utilisées en routine en chimie. Le produit de départ est le même que pour la synthèse des marqueurs **71** et **74.** La première étape consiste en la protection de l'amine terminale par le Fluorenylmethyl-formate (Fmoc, **99).** Le choix de ce groupement protecteur est basé sur sa stabilité et conditions de clivage.

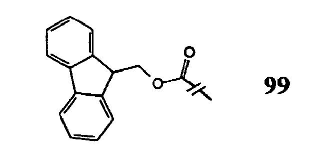

Après formation de l'hydrazone protégée **82** par la méthode utilisée précédemment (exemple *méta*-Fluo-EG₃-PMDAM, ), l'amine terminale est déprotégée dans des conditions basiques douces qui garantissent la stabilité de l'hydrazone. L'acétoacétate de méthyle est utilisé pour créer la fonction méthylcetone par une réaction d'acylation de l'amine terminale (voir formation des composés **26** et **36**). La formation du diazométhyl est ensuite réalisée par l'une des méthodes décrites précédemment.

### Exemple 290.2 : Synthèse du H₂NO-PMDAM :

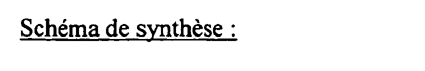

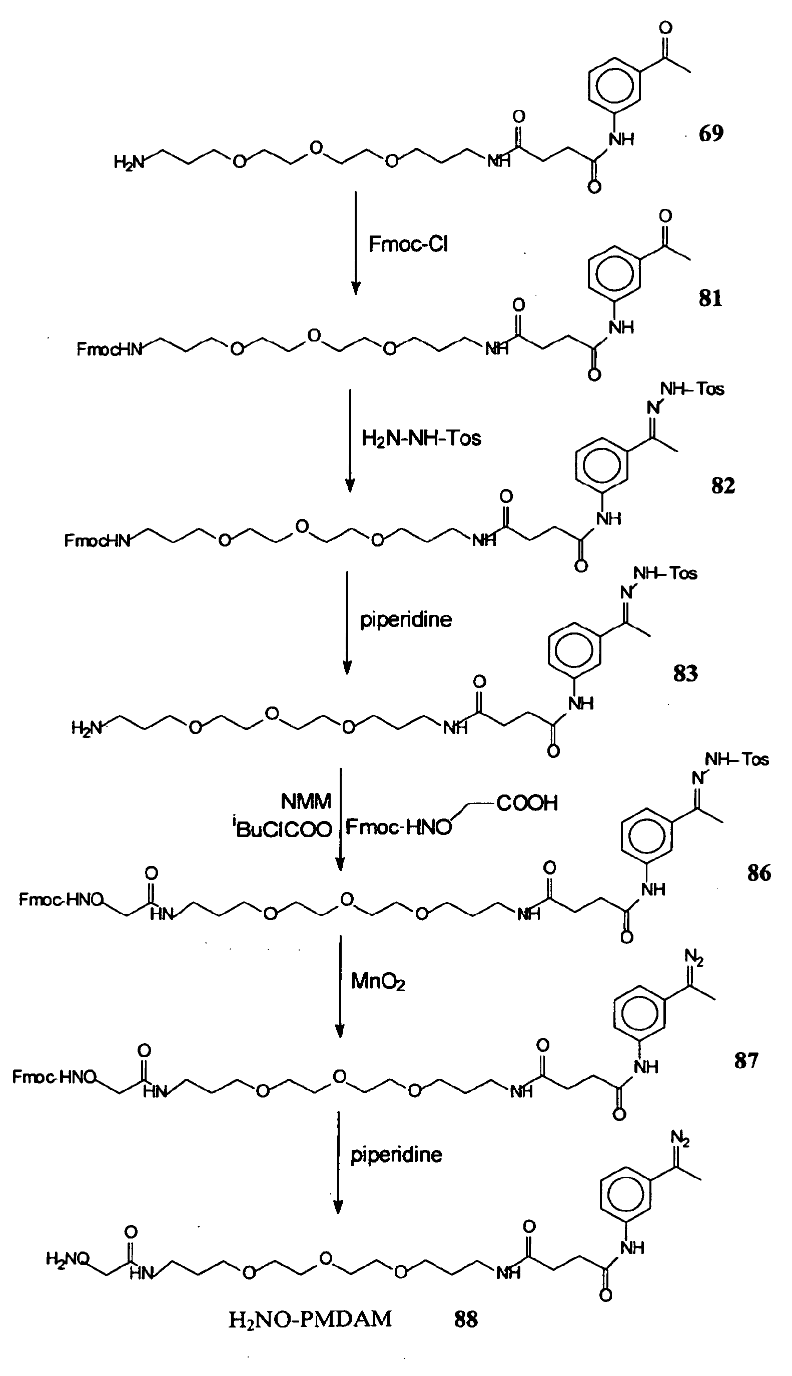

Le produit **88,** dont la synthèse est décrite dans cet exemple, permet de réaliser le marquage des acides nucléiques naturels, grâce a la réactivité de la fonction diazométhyl avec les groupements phosphate, et d'introduire ainsi une fonction alcoxyamine, qui peut être utilisée par la suite pour introduire une molécule détectable (fluorescente, biotine) possédant un groupement méthylcétone.

Cette synthèse est basée sur le modèle de la précédente, c'est-à-dire l'utilisation du précurseur **69,** du Fmoc pour la protection de l'amine et du tosyle pour la protection de l'hydrazone. L'introduction de la fonction alcoxyamine (composé **86)** se fait par l'usage du carboxyméthoxylamine (commercial) protégé par la fonction Fmoc (Thèse E. Trévisiol, LEDSS Grenoble, 1999). Etant donné les conditions douces de la déprotection finale (composé **88),** celle ci est réalisée de suite après la formation du diazométhyl.

### Exemple 30 : Préparation de dérivés PDAM permettant l'amplification du signal:

### Exemple 30.1 : Synthèse de marqueurs bis-biotinylés tels que le [Bio-EG3]2-PDAM :

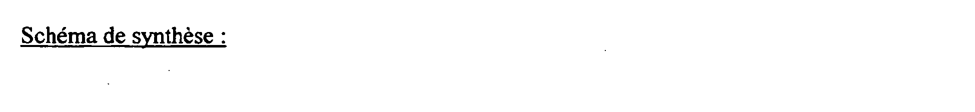

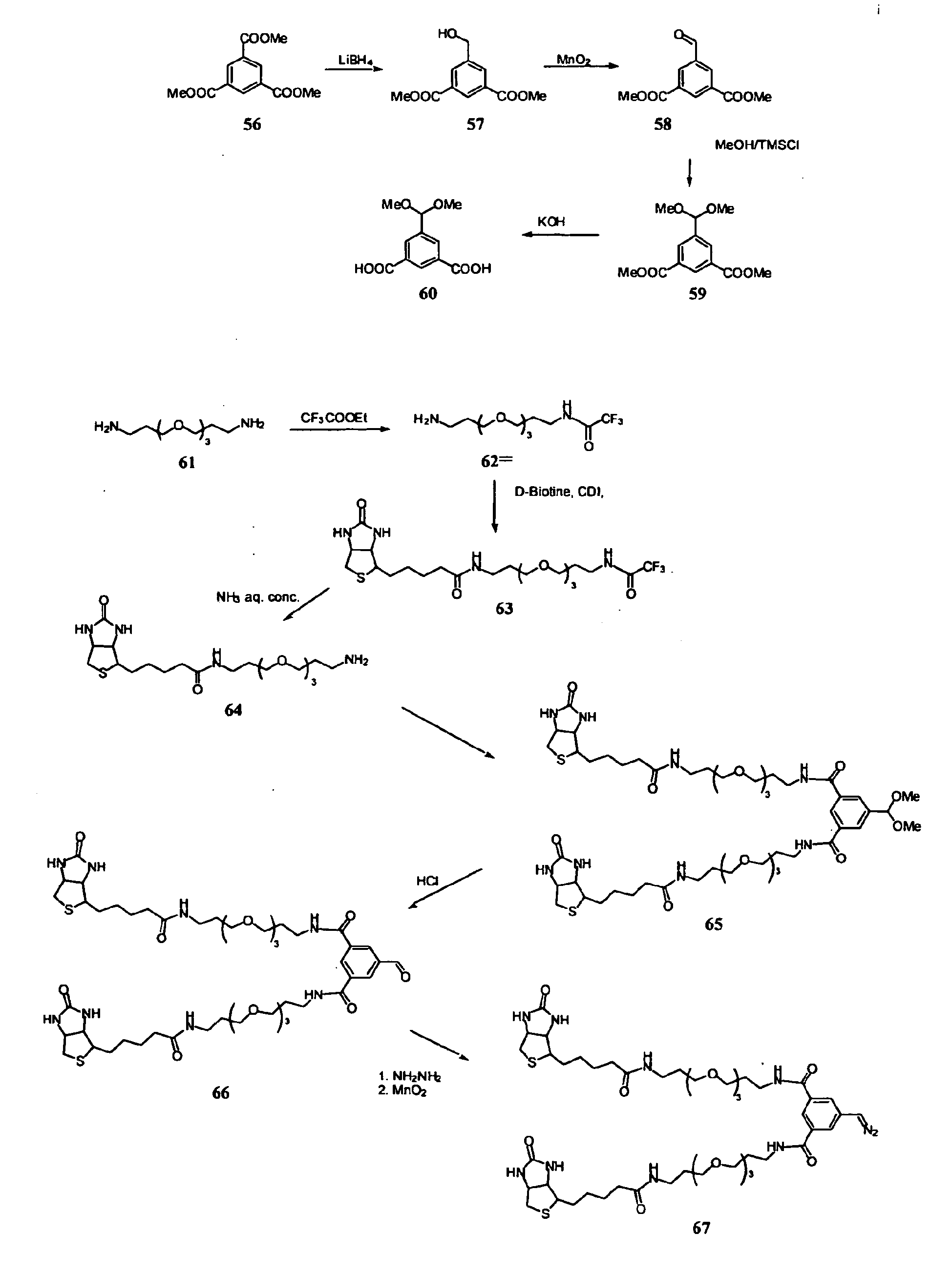

### • Réduction du benzène-1,3,5-tricarboxylate de triméthyle 56 en alcool 57 :

On dissout le triester **56** (12,6 g ; 50,0 mmol) dans 100 mL de THF puis ajoute 1,1 g (50,5 mmol) de LiBH₄ à température ambiante. La solution rouge est chauffée à 40-45°C sous agitation sous argon pendant 1 h. Après refroidissement (glace), on détruit l'hydrure en excès avec précaution (dégagement de H₂) par l'addition d'eau (200 mL) puis de HCl 2N (30 mL). On observe le virage de couleur en jaune claire. On extrait cette solution avec CH₂Cl₂ (100mL puis 3 fois 50 mL), la phase organique est lavée avec NaHCO₃ anhydre, séchée sur MgSO₄ puis le solvant est évaporé jusqu'à l'obtention d'une huile (11,1 g). Par chromatographie flash sur colonne de silice (diamètre = 40 mm, éluant : acétate d'éthyle/cyclohéxane = 1/1), on obtient l'alcool **57** (6,38 g, 57 %).

RMN ¹H ( 200 MHz, CDCl₃) : δ = 8,53(t, 1H, *J* = 2 Hz) ; 8,18 (d, 2H, *J* = 2 Hz) ; 4,76 (s, 2H) ; 3,91 (s, 6H) ; 2,30 (s, 1H).

### • Oxydation de l'alcool 57 en aldéhyde 58:

On dissout l'alcool **57** (5,86 g ; 26,1 mmol) dans 100 mL de THF puis ajoute 40,0 g de MnO₂ petit à petit en 5 min à température ambiante. La solution est laissée une nuit sous agitation sous argon. On filtre la solution à travers un entonnoir Büchner muni d'une couche de Célite 545, on lave avec CH₂Cl₂ puis évapore les solvants. Le solide brut (4,4 g) est purifié par chromatographie flash sur une colonne de silice (diamètre = 50 mm, éluant : acétate d'éthyle/cyclohéxane 3/7). On obtient 3,44 g (59 %) de l'aldéhyde **58.**

RMN ¹H ( 200 MHz, CDCl₃) : δ = 10,11 (s, 1H) ; 8,89(t, 1H, *J =* 1 Hz) ; 8,69 (d, 2H, *J =* 1 Hz) ; 3,98 (s, 6H).

### • Formation de l'acétal 59:

On dissout l'aldéhyde **58** (3,21 g ; 14,4 mmol) dans 30 mL de méthanol puis ajoute 6,0 mL de TMSCl. La solution est laissée à température ambiante sous agitation sous argon pendant 1 h.

On dilue la solution avec 200 mL de CH₂Cl₂, et agite avec 1 M NaHCO₃ (100 mL) (attention dégagement de CO₂). On sépare les deux phases, extrait la phase aqueuse trois fois avec CH₂Cl₂ (25 mL), on rassemble les phases organiques, on sèche sur MgSO₄ puis évapore le solvant. On obtient 3,55 g (92 %) de l'acétal 59.

RMN ¹H ( 200 MHz, CDCl₃) : δ = 8,63(t, 1H, *J* = 2 Hz) ; 8,29 (d, 2H, *J* = 2 Hz) ; 5,45 (s, 2H) ; 3,93 (s, 6H) ; 3,32 (s, 6H).

### • Hydroyse du diester 59 en diacide 60 :

On dissout le diester **59** (3,18 g ; 11,9 mmol) dans 10 mL de THF puis ajoute une solution de KOH (2,0 g, pastille 85 %) dans 10 mL de méthanol. Après 15 min à température ambiante, on évapore les solvants. Le résidu est dissous dans H₂O (50 mL). On ajoute H₃PO₄ (environ 2,5 mL, 85 %) jusqu'à pH 3 et le précipité blanc est filtré sur le fritté (#3), lavé avec de l'eau et séché sous vide. On obtient 2, 59 g (91 %) du diacide **60**.

RMN ¹H ( 200 MHz, DMSO-*d*₆) : δ = 8,43 (t, 1H, *J* = 1 Hz) ; 8,15 (d, 2H, *J* = 1 Hz) ; 5,53 (s, 1H) ; 3,27 (s, 6H).

### • Trifluoracétamide 62 :

On dissout la diamine **61** (66 g ; 0,30 mol) dans 250 mL de CH₂Cl₂ puis ajoute le trifluoracétate d'éthyle (11,8 mL, 0,10 mol) goutte à goutte en 5 min à 10 °C sous agitation sous argon. Après 15 min à température ambiante, on transfère la solution dans une ampoule à décanter, on lave avec H₂O (3 × 100 mL), on sèche sur MgSO₄ et évapore le solvant. On obtient 22,4 g (71 %) du monoamide **62** de pureté d'environ 85 % (déterminée par F¹⁹ RMN). Ce composé est conservé à -20 °C et utilisé sans purification.

RMN ¹H ( 200 MHz, CDCl₃) : δ = 3,5-3,6 (m, 12H) ; 3,42 (t, 2H, *J* = 6 Hz) ; 2,75 (t, 2H, *J* = 6 Hz) ; 1,81 (quantiplet, 2H, *J* = 6 Hz) ; 1,67 (quantiplet, 2H, *J* = 6 Hz) ; 1,30 (s. large, 2H). RMN ¹⁹F ( 190 MHz, CDCl₃) δ = -76,3.

### • Composé 63 :

A une suspension de D-biotine (6,39 g ; 26,2 mmol) dans 50 mL de DMF, on ajoute le carbonyldiimidazole (CDI, 6,32 g , 80 %, 31,2 mmol). Le mélange est chauffé à 55-60 °C sous agitation sous argon pendant 30 min. On observe initialement une dissolution complète de matériel puis une prise en masse avec une précipitation de solide blanc (CO₂ évolution). On ajoute l'amine (huile) à l'aide de 5 mL de CH₂Cl₂ pour rincer et le mélange est chauffé à 55-60 °C pendant 3 h. On évapore le DMF sous vide (< 1 mmHg) et le résidu est agité avec CH₂Cl₂ (700 mL) et 2N HCl (100 mL). Après une filtration des deux phases à travers une couche de Célite 545, on sépare les phases, la phase aqueuse est extraite avec CH₂Cl₂ (15 × 100 mL), les phases organiques sont rassemblées, séchées sur NaHCO₃ anhydre et MgSO₄, puis le solvant est évaporé. Le résidu huileux est trituré avec 150 mL d'éther pour obtenir une suspension. Le solide pâteux est difficile à filtrer. On décante le surnageant et on répète le lavage à l'éther. Après séchage sous vide, on obtient 9,13 g (64 %) de composé 63.

RMN ¹H ( 200 MHz, CDCl₃) : δ = 3,5-3,6 (m, 12H) ; 3,42 (t, 2H, *J* = 6 Hz) ; 2,75 (t, 2H, *J* = 6 Hz) ; 1,81 (quantiplet, 2H, *J* = 6 Hz) ; 1,67 (quantiplet, 2H, *J* = 6 Hz) ; 1,30 (s. large, 2H).

### • Composé 64 :

On chauffe une solution du composé **63** dans l'ammoniaque (100 mL, 22 % aqueuse) dans un ballon de 250 mL bouché avec un septum à 55-60 °C pendant 2 h. Après refroidissement, on évapore le solvant. Le résidu est dissous dans le méthanol (20 mL) et passé sur une colonne résine échangeuse d'anion Dowex 21K [hauteur 12 cm × diamètre 35 mm, forme OH⁻ obtenu par lavage préalable avec NaOH 1N (1,5 L) puis H₂O (1,5 L) puis méthanol (1,5 L)]. Le composé **64** libre de l'ion trifluoracétate passe dans les premières fractions avec 200 mL du méthanol. Après évaporation, on triture le résidu avec 50 mL d'éther puis on le décante. Le lavage à l'éther est répété cinq fois de suite. Après séchage, on obtient le composé **64** (6,43 g, 86%).

RMN ¹H ( 300 MHz, CDCl₃) : δ = 6,77 (t, 1H, *J* = 4 Hz) ; 6,32 (s, 1H) ; 5,52 (s, 1H) : 4,45 (m, 1H) ; 4,28 (m, 1H), 3,50-3,68 (m, 12H) ; 3,30 (m, 2H), 3,11 (m, 1H) ; 2,86 (dd, 1H, *J* = 13 et 5 Hz), 2,75 (t, 2H, *J =* 13 Hz), 2,68 (d, 1H, *J* = 13 Hz) ; 2,16 (t, 2H, *J =* 7 Hz) ; 1,60-1,85 (m, 8H) ; 1,41 (m, 2H).

### • [Bio-EG₃]₂- acétal 65 :

A une suspension du diacide **60** (120 mg ; 0,500 mmol) dans le dichloroéthane (5 mL), on ajoute le carbonyldiimidazole (225 mg, 90 %, 1,25 mmol) et on chauffe à 55-60 °C pendant 30 min sous agitation sous argon. On ajoute l'amine **64** (550mg ; 1,23 mmol) et la solution est chauffée à 55-60 °C pendant 6 h. Après évaporation, on passe sur une colonne de silice (diamètre : 25 mm, éluant : méthanol 15-30 % dans CH₂Cl₂). On obtient 413 mg (75 % ) de composé **65**.

RMN ¹H ( 300 MHz, CDCl₃) : δ = 8,34 (s, 1H) ; 8,06 (s, 2H) ; 7,87 (m, 2H) ; 6,85 (m, 2H) ; 6,60 (s, 2H) ; 5,93 (s, 2H) : 5,40 (s, 1H) ; 4,45 (m, 2H) ; 4,27 (m, 2H), 3,43-3,68 (m, 24H) ; 3,31 (s, 6H) ; 3,25 (m, 4H), 3,08 (m, 2H) ; 2,83 (dd, 2H, *J* = 13 et 5 Hz), 2,70 (t, 2H, *J* = 13 Hz) ; 2,13 (t, 4H, *J* = 7 Hz) ; 1,89 (quintuplet, 4H, *J* = 7 Hz) ; 1,55-1,70 (m, 12H) ; 1,37 (m, 4H).

### • [Bio-EG₃]₂- aldéhyde 66 :

L'acétal **65** (413 mg ; 0,376 mmol) en solution dans le méthanol est traité avec HCl 2N (0,5 mL). Après évaporation et lavage avec l'éther, on obtient l'aldéhyde **66** (0,37 g, 90 %).

RMN ¹H ( 300 MHz, DMSO-*d*₆) : δ = 10,11 (s,1H) ; 8,82 (t, 2H, *J =* 6 Hz) ; 8,62 (s, 1H) ; 8,47 (s, 2H) ; 7,73 (t, 2H, *J* = 5 Hz) ; 4,30 (m, 2H) ; 4,11 (m, 2H), 3,30-3,60 (m, 24H) ; 3,06 (m, 6H) ; 2,80 (dd, 2H, *J =* 12 et 5 Hz), 2,56 (t, 2H, *J=* 12 Hz) ; 2,03 (t, 4H, *J* = 7 Hz) ; 1,78 (quintuplet, 4H, *J* = 7 Hz) ; 1,35-1,60 (m, 12H) ; 1,28 (m, 4H).

### • Bio-EG₃]₂- PDAM 67 :

L'aldéhyde **66** est transformé en diazométhane **67** selon la méthode utilisée pour la préparation des diazométhanes (Exemple 1).

La stabilité du réactif est supérieure à 1 mois à -20°C.

### Exemple 30.2 : Synthèse du méta-Bio7-EG3-PMDAM :

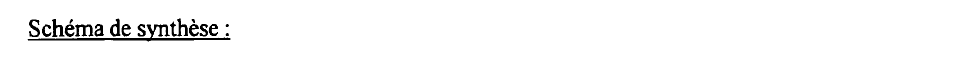

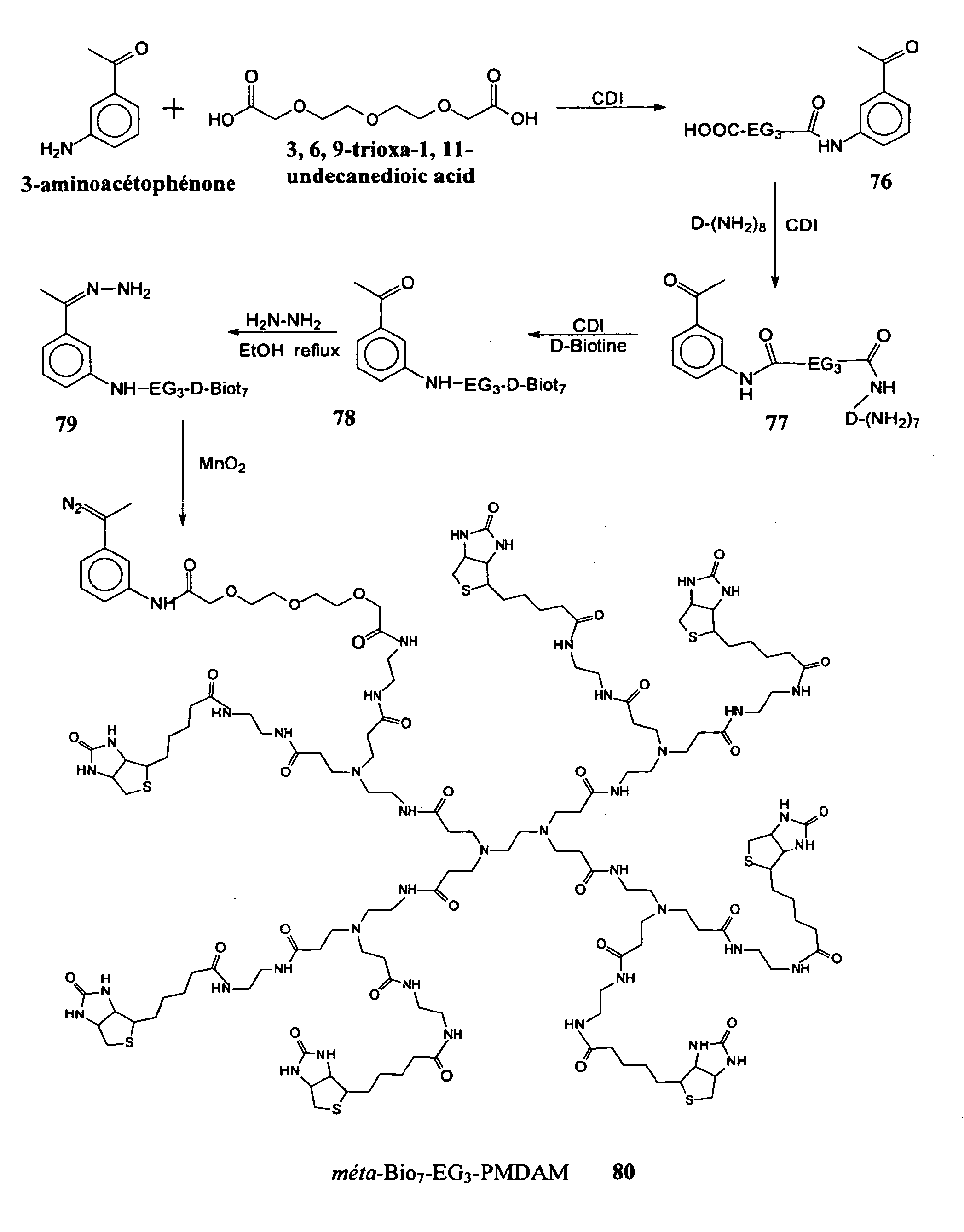

### Composé EG₃-acétophénone 76 :

Le 3, 6, 9-trioxa-1, 11-undecanedioic acid (EG₃, 12,64 ml, 74 mmol) est dissout dans 80 ml de DMF anhydre sous argon et refroidit dans un bain de glace. Le dicyclohexylcarbodiimide (DCC, 11,45 g, 55,5 mmol)) est ensuite dissout dans 20 ml de DMF anhydre et ajouté lentement. Après 30 min, on ajoute le 3-aminoacétophénone (5,0 g, 37 mmol) et on laisse réagir 1 h à température ambiante sous argon. Le DMF est ensuite évaporé sous vide et 70 ml de CH₂CL₂ sont ajoutés. La solution est filtrée et extraite avec 3x25 ml d'acide acétique 1%. Les phases aqueuses sont réunies et lavées avec 25 ml de CH₂CL₂. Les phases organiques sont mélangées, séchées avec du sulfate de sodium anhydre et évaporées à sec. Le produit est recristallisé dans le couple MeOH:H₂O. On obtient ainsi 8,74 g (70%) du produit 76.

RMN ¹H (200 MHz, DMSO-d₆): δ = 2,55 (s, 3H); 3,5-3,7 (m, 8H); 4,0 (s, 2H); 4,1 (s, 2H); 7,45 (t, 1H); 7,65 (d, 1H); 7,90 (d, 2H); 8,2 (s, 1H); 9,8 (s, 1H).

### Composé (NH₂)₇-EG₃-acétophénone 77 :

Le produit **76** (120mg, 0,35 mmol) est dissout dans 15 ml de DMF anhydre sous argon, refroidit sur glace, et le DCC (110 mg, 0,53 mmol) est ensuite ajouté. Après 30 min, on ajoute cette solution sur une solution du dendrimère commercial « Starburst PAMAM Dendrimer, Generation 1 » (Aldrich, St Quentin Fallavier) (1 g, 0,71 mmol, dans 5 ml de méthanol), lentement et sous forte agitation. On laisse réagir 1 h à température ambiante et on évapore. Le résidu est repris dans 10 ml de CH₂CL₂ et extrait deux fois avec 30 ml d'acide acétique 1 %.

### Composé Biot₇EG₃- acétophenone 78 :

La D-Biotine (1,73 g, 7,08 mmol) est solubilisée dans 80 ml de DMF anhydre sous argon, et la solution est refroidie sur glace. On ajoute successivement la N-méthylmorpholine (NMM, 856 µl, 7,7 mmol) et le chloroformiate d'isobutyle (1022 µl, 7,7 mmol). Après 30 min, on ajoute le produit 77 (1,13 g, 0,7 mmol, dans 5 ml de méthanol) et on laisse réagir 3 h sur glace et sous argon. On concentre sous vide jusqu'à 50 ml et on ajoute 100 ml de CH₂Cl₂ . Un précipité se forme, qui est filtré, lavé à l'éther, et séché sous vide. On obtient 1,3 g de **78** sous forme d'une poudre blanche.

### Composé Biot₇-EG₃-Hydrazone 79 :

Le composé **78** (300 mg, 0,09 mmol) est dissout dans 10 ml d'éthanol absolu à reflux. On ajoute l'hydrazine monohydrate (20 ml, 0,40 mmol) et on laisse réagir 3 h à reflux. Après refroidissement, un précipité se forme, qui est filtré, lavé a l'éther et séché sous vide. On obtient ainsi 109 mg (36%) du produit **79,** sous forme d'une poudre blanche.

### Composé Biot₇-EG₃-PMDAM 80 :

On solubilise l'hydrazone **79** (100 mg, 0,03 mmol) dans 5 ml de DMF anhydre à 70°C. On laisse revenir à température ambiante et on ajoute le MnO₂ (31 mg, 0, 36 mmol). On laisse réagir 10 min et on élimine l'oxyde de manganèse par filtration sur un fritté avec de la élite (0,5 cm et du tamis moléculaire en poudre (0,5 cm). Le filtrat est évaporé a sec, lavé a l'éther e seché sous vide. On obtient ainsi 78 mg (78%) du produit **80.**

Les dendrimères sont des molécules arborescentes possédant aux extrémités plusieurs groupements réactifs tels que des amines, carboxyles, hydroxyles ou autres (pour revue, voir Newcome et al, (1996) Dendritic Molecules: Concept, Syntheses, Perspectives. VCH Ed., Weinheim, Germany). La synthèse de ces molécules est aujourd'hui bien maîtrisée, et des nombreux dendrimères sont commercialisés par l'industrie chimique. Le choix du PAMAM (Sigma-Aldrich) a été fait sur la base de sa stabilité, solubilité et souplesse, puisque plusieurs versions de cette molécule, avec un nombre et type de terminaisons différentes, sont disponibles. Le "PAMAM Generation 1" permet d'ajouter sept molécules du marqueur (en une seule étape de synthèse) pour chaque groupement diazométhyl.

### Exemple 31 : Marquage et fragmentation en deux étapes d'amplicons ADN avec le méta-BioPMDAM :

Les amplicons ADN ont été préparés par amplification PCR selon le protocole décrit dans l'exemple 5.1. Deux réactions de marquage ont été réalisées.

### a. Marquage et fragmentation en deux étapes :

A 10 µL de PCR sont ajoutés 10 µL de *méta*-BioPMDAM (100 mM dans du DMSO) et 77 µL d'eau Dnase/Rnase free. La solution est incubée 10 min à 95°C. Puis 3 µL d'HCl à 0,1M sont ajoutés et la solution est incubée 10 min à 95°C.

### b. Marquage et fragmentation en une étape :

A 10 µL de PCR sont ajoutés 10 µL de *méta*-BioPMDAM (100 mM dans du DMSO), 5 µL d'HCl à 0,1 M et 75 µL d'eau Dnase/Rnase free. La solution est incubée 30 min à 60°C.

Le reste du protocole est identique à celui de l'exemple 17.

### Résultats :

**Tableau 24 : Etude comparative du marquage et de la fragmentation en deux étapes distinctes et en une seule étape**

| **Protocole utilisé** | **Homologie (%)** | **I (rfu)** | **B (rfu)** | **I/B** |
|---|---|---|---|---|
| a. Marquage et fragmentation en deux étapes | 99,5 | 14129 | 624 | 22,7 |
| b. Marquage et fragmentation en une étape | 98,9 | 4431 | 667 | 6,6 |

Comme le démontre le tableau 24, les résultats obtenus avec le protocole en une étape sont satisfaisants. Ceux obtenus avec un marquage et une fragmentation en deux étapes sont encore meilleurs. Cet exemple montre que les étapes de marquage et clivage peuvent être dissociées pour améliorer le marquage en fonction de la cible utilisée.

### Exemple 32 : Marquage et fragmentation d'amplicons ADN dans différents formats de réaction :

Les amplicons d'ADN ont été préparés par amplification PCR selon le protocole décrit dans l'exemple 5.1. Trois réactions de marquage ont été réalisées.

### a. Marquage et fragmentation dans un format de 250 µL :

A 50 µL de PCR sont ajoutés 75 µL de *méta*-BioPMDAM (100 mM dans du DMSO) et 102,5 µL d'eau Dnase/Rnase free. La solution est incubée 25 min à 95°C. Puis 22,5 µL d'HCl à 0,1 M sont ajoutés et la solution est incubée 5 min à 95°C.

### b. Marquage et fragmentation dans un format de 200 µL :

A 50 µL de PCR sont ajoutés 75 µL de *méta*-BioPMDAM (100 mM dans du DMSO) et 52,5 µL d'eau Dnase/Rnase free. La solution est incubée 25 min à 95°C. Puis 22,5 µL d'HCl à 0,1 M sont ajoutés et la solution est incubée 5 min à 95°C.

### c. Marquage et fragmentation dans un format de 150 µL :

A 50 µL de PCR sont ajoutés 75 µL de *méta*-BioPMDAM (100 mM dans du DMSO) et 2,5 µL d'eau Dnase/Rnase free. La solution est incubée 25 min à 95°C.

Puis 22,5 µL d'HCl 0,1M sont ajoutés et la solution est incubée 5 min à 95°C.

Le reste du protocole est identique à celui de l'exemple 17.

### Résultats :

**Tableau 25 : Marquage et fragmentation selon différents formats**

| **Protocole utilisé** | **Homologie (%)** | **I (rfu)** | **B (rfu)** | **I/B** |
|---|---|---|---|---|
| *a. Format de 250* µ*L* | 100,0 | 5606 | 549 | 10,2 |
| *b. Format de 200* µ*L* | 99,4 | 5886 | 557 | 10,6 |
| *c. Format de 150* µ*L* | 99,4 | 6800 | 537 | 12,7 |

Les résultats obtenus en terme de signal et de pourcentage d'homologie sont très satisfaisants dans tous les cas de figures. De plus, bien que le format de la réaction varie de 150 à 250 µL, les résultats ont des valeurs similaires.

Cet exemple montre une flexibilité du format réactionnel du protocole de marquage pouvant accepter différents volumes et notamment différents volumes de produits d'amplification.

### Exemple 33 : Comparaison entre un protocole utilisant une étape de purification avant fragmentation et un protocole utilisant une étape de purification après fragmentation :

Les amplicons d'ADN ont été préparés par amplification PCR selon le protocole décrit dans l'exemple 5.1. Deux réactions de marquage ont été réalisées.

### a. Marquage purification puis fragmentation des amplicons ADN :

A 10 µL de PCR sont ajoutés 10 µL de *méta*-BioPMDAM (100 mM dans du DMSO) et 80 µL d'eau Dnase/Rnase free. La solution est incubée 10 min à 95°C. Puis la purification est réalisée selon le protocole décrit dans l'exemple 17. A la solution d'amplicons marqués purifiés 6 µL d'HCl à 0,1 M sont ajoutés. La solution est incubée 10 min à 95°C. Quatre cents (400) µL de tampon d'hybridation préchauffé à 95°C durant 10 min sont ajoutés.

La composition du tampon d'hybridation ainsi que le reste du protocole sont identiques à celui de l'exemple 17.

### b. Marquage fragmentation puis purification des amplicons ADN :

A 10 µL de PCR sont ajoutés 10 µL de *méta*-BioPMDAM (100 mM dans du DMSO) et 77 µL d'eau Dnase/Rnase free. La solution est incubée 10 min à 95°C.

Puis 3 µL d'HCl à 0,1 M sont ajoutés et la solution est incubée de nouveau 10 min à 95°C. Le reste du protocole est identique à celui de l'exemple 17.

### Résultats :

**Tableau 26 : Comparaison entre protocole utilisant une étape de purification avant fragmentation et protocole utilisant une étape de purification après fragmentation_**

| **Protocole utilisé** | **Homologie (%)** | **I (rfu)** | **B (rfu)** | **I/B** |
|---|---|---|---|---|
| a. Purification avant fragmentation | 98,9 | 6256 | 473 | 13 |
| b. Fragmentation avant purification | 96,1 | 6066 | 556 | 11 |

Ce résultat, présenté dans le tableau 26, montre que l'étape de purification peut être introduite entre les étapes de marquage et de fragmentation. De plus l'introduction de la purification entre les étapes de marquage et de fragmentation permet de réaliser la dénaturation pendant le clivage et d'hybrider sur la puce la totalité des fragments d'amplicons marqués.

### Exemple 35 : Capture et amplification d'ADN sur une membrane de Nylon portant des groupements diazométhyles :

Une membrane de nylon activée de façon à porter des groupements diazométhyles a été utilisée afin de capturer de l'ADN bactérien, dans le but de l'amplifier par PCR.

### Exemple 35.1 : Modification du filtre Biodyne C :

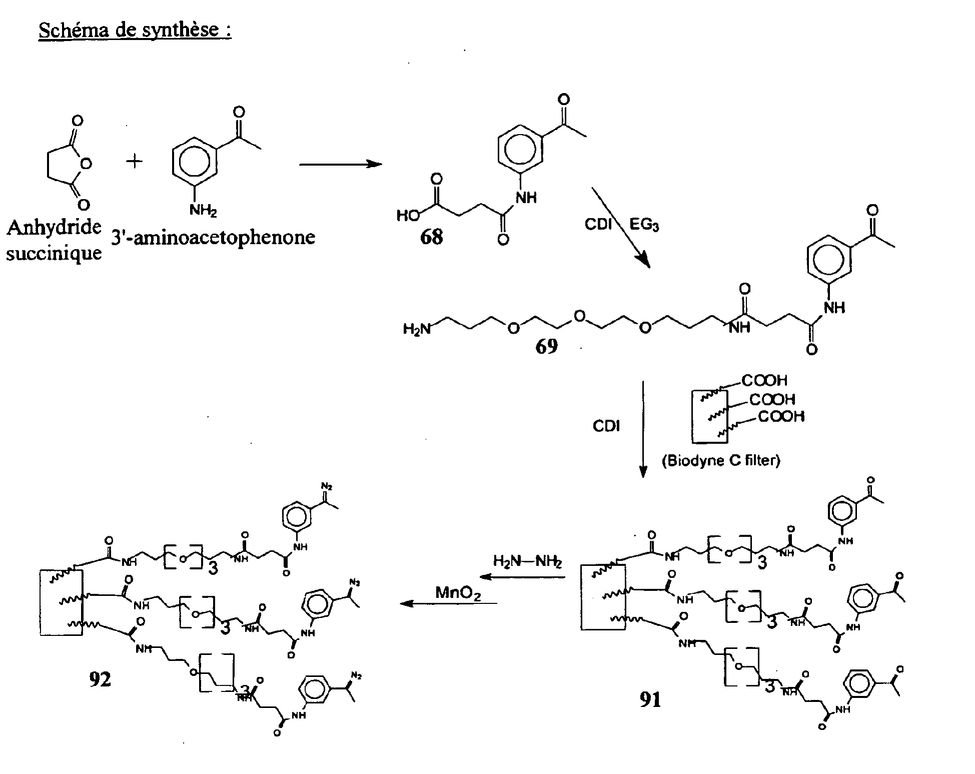

### Composé 68 :

On solubilise la 3-aminoacetophenone (14,5 g, 107 mmol) dans 50 ml de DMF anhydre. On ajoute l'anhydride succinique (10,7 g, 107 mmol) et on laisse sous agitation, sous argon et à température ambiante. Après 6 h, la solution est concentrée sous vide et 50 ml de méthanol sont ajoutés. Le précipité obtenu est filtré et lavé avec méthanol et éther. On obtient ainsi 19,4 g (81 %) de produit **68** sous forme d'une poudre de couleur blanc cassé.

RMN ¹H (200 MHz, DMSO-d₆): δ= 2,5-2,6 (m, 7H); 7,45 (t, 1H); 7,64 (d, 1H); 7,83 (d, 1H); 8,19 (s, 1H); 10,16 (s, 1H); 12,12 (s, 1H).

### Composé 69 :

On solubilise 5,07 g (22 mmol) du composé **68** dans 10 ml de DMF anhydre, sous argon. On met sur glace et on ajoute 5,00 g (32 mmol) de carbonyldiimidazole. Apres 20 min, on ajoute lentement 20 ml (94,6 mmol) du 4,7,10-trioxatridecanediamine (EG₃). Après 3h de réaction à température ambiante, on évapore le DMF et on reprend le résidu dans 100 ml de CH₂Cl₂. On fait des extractions avec du NaHCO₃ saturé et H₂O, après quoi la phase organique est séchée avec du Na₂SO₄ anhydre et le solvant évaporé. On obtient ainsi 4,34 g (46 %) du produit **69**.

RMN ¹H (200 MHz, DMSO-d₆): δ= 1,59 (m, 2H); 1,87 (m, 2H); 2,16 (s, 3H); 2,40 (m, 2H); 2,55 (m, 2H); 3,08 (m, 2H); 3,45 (m, 16H); 7,30 (t, 1H); 7,42 (d, 1H); 7,70 (d, 1H); 7,83 (t, 1H); 7,97 (s, 1H); 10,00 (s, 1H).

### Composé 91 :

Un rectangle de 4 cm² est découpé sur une feuille de filtre Biodyne C (référence 60314 ; Pall Gelman Laboratory ; Ann Arbor ; Michigan ; USA), introduit dans un flacon et mis en contact avec 0,97 g (6 mmol) de carbonyldiimidazole (CDI) dans 3 ml de DMF anhydre, sur glace, sous argon et sous forte agitation. Après 20 min, la solution est éliminée et le filtre lavé avec du DMF. Une quantité de 0,53 g du produit **68** (1 mmol) dans 3 ml de DMF anhydre est ensuite ajoutée, et la réaction se réalise pendant la nuit à température ambiante. La solution est ensuite enlevée et le filtre est rincé avec de l'éthanol, séché sous vide et gardé sous argon.

### Composé 92 :

Le filtre modifié **91** est mis dans une solution de 97 ml d'hydrazine hydrate (2 mmol) dans 4 ml d'éthanol absolu. La solution est mise à reflux pendant 5 h. Après avoir laissé refroidir, le filtre est lavé avec H₂O, éthanol et éther, séché sous vide et mis sous argon. Ensuite, on ajoute 4 ml de DMF anhydre et 86 mg de MnO₂ (1 mmol), et on laisse réagir sous forte agitation. Après 20 min, la solution est rejetée, et le filtre est rincé avec du DMF et de l'éther. Le filtre modifié-diazométhyl **92** est conservé sous argon, à une température de -19 à -31°C.

### Exemple 35.2 : Essais biologiques :

La membrane activée est découpée en petits fragments de 2 mm² qui vont être incubés pendant 30 minutes à température ambiante dans 25 µl de lysat bactérien de *Mycobacterium tuberculosis* et 375 µl d'eau pure (Sigma).

Le lysat bactérien de *Mycobacterium tuberculosis* est préparé par lyse mécanique. Plus précisément, elle est réalisée par sonication, l'échantillon liquide traité contenant des billes de verre. Un tel procédé est déjà bien décrit par la Demanderesse dans sa demande de brevet WO-A-99/15621, en ce qui concerne les billes, et dans sa demande de brevet WO-A-00/60049, en ce qui concerne la sonication. La sonication peut également être réalisée à l'aide d'un bain liquide.

Toutefois d'autres techniques, connues de l'homme du métier, peuvent être utilisées comme celles décrites dans le brevet US-A-5,902,746 et les demandes de brevet WO-A-98/54306 et WO-A-00/05338. Tous ces titres de Propriété Industrielle appartiennent à la Demanderesse. L'ADN bactérien a été quantifié par Picogreen (P-7589 ; Molecular Probes, Eugene, OR, USA) suivant le protocole décrit par le fournisseur, à une concentration de 10⁷ copies par µL.

La membrane est ensuite placée à 65°C pendant 60 min dans 100 mL de tampon de lavage (5 % Formamide (Sigma), 1X SSPE (Perbio), 0.01 % triton X-100) afin d'éliminer les acides nucléiques non spécifiques adsorbés sur la membrane, puis celle-ci est stockée dans 1 mL d'eau pure avant amplification.

La PCR est pratiquée comme décrite dans le paragraphe 5.1, le volume réactionnel étant complété par une quantité suffisante d'eau pure.

Parallèlement, des contrôles sont effectués suivant le même processus avec des membranes ne pouvant pas lier de façon covalente les acides nucléiques :
- Membrane Biodyne C non modifiée (membrane A),
- Membrane Biodyne modifiée chimiquement suivant le processus décrit, mais non traitée par du DMF anhydre et du MnO₂ ; ce contrôle permet de vérifier le comportement de la membrane en l'absence de groupements diazométhyles (membrane B), et
- Membrane Biodyne C non modifiée, mais traitée 20 min sous forte agitation par du DMF anhydre et du MnO₂, ce contrôle permettant de vérifier que cette dernière étape ne modifie pas l'adsorption de l'ADN sur la membrane (membrane C).

Afin de contrôler l'absence d'inhibition de la PCR provoquée par le traitement des membranes, un autre fragment des membranes A, B, C est amplifié simultanément avec 25 µl de lysat bactériens.(tubes A', B', C')

Les produits de PCR sont ensuite quantifiés par Picogreen suivant le protocole décrit par le fournisseur.

### Résultat :

**Tableau 27 : Quantification de l'ADN obtenu par PCR à partir d'ADN de lysat bactérien capturé sur support solide**

| **Tests effectués** | **Signal (rfu)** |
|---|---|
| Membrane modifiée | 111 |
| Membrane non modifiée (A) | 18 |
| Membrane non modifiée, co-amplifiée avec 25 µL de lysat bactérien (A') | 260 |
| Membrane modifiée non activée (B) | 26 |
| Membrane modifiée non activée, co-amplifiée avec 25 µL de lysat bactérien (B') | 264 |
| Membrane non modifiée ayant subit une activation (C) | 21 |
| Membrane non modifiée ayant subit une activation, co-amplifiée avec 25 µL de lysat bactérien (C') | 268 |

Ces résultats du tableau 27 indiquent qu'il est possible de capturer de façon covalente sur un support solide, des acides nucléiques, issus de lysat, grâce à la chimie diazométhyle. L'amplification observée n'est pas due à une adsorption non spécifique de l'ADN sur la membrane. D'autre part, on observe avec les contrôles effectués avec les PCR que les membranes ne provoquent pas d'inhibition de la réaction d'amplification.

Afin de contrôler la nature du produit amplifié sur la membrane, le produit d'amplification a été analysé par passage sur puce à ADN, suivant le protocole décrit auparavant.

## Revendications

1. Réactif de marquage stable à la température de formule (0) : 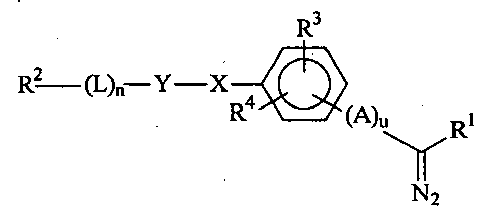 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-, et
• le substituant portant la fonction diazo étant en position *ortho* ou *méta.*

2. Réactif de marquage, selon la revendication 1, de formule (1) : 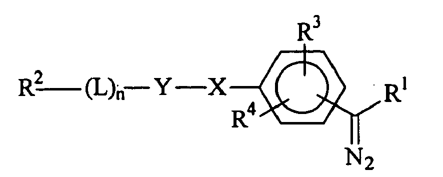 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle, et
• -Y-X- représente -CONH-, -NHCO-, -CH₂O-, -CH₂S-.

3. Réactif, selon l'une quelconque des revendications 1 ou 2, de formule (2) : 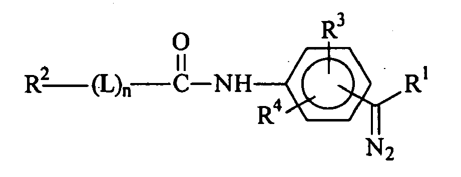 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

4. Réactif, selon l'une quelconque des revendications 1 ou 2, de formule (3) : 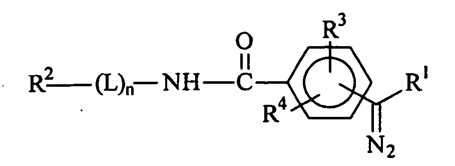 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R²-(L)ₙ-Y-X-, OR, SR, NR₂, R, NHCOR, CONHR, COOR avec R = alkyle ou aryle.

5. Réactif, selon l'une quelconque des revendications 1 ou 2, de formule (4) : 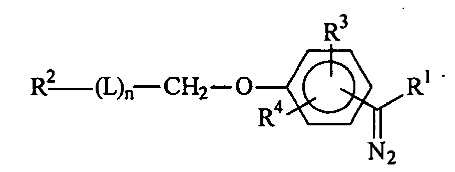 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, CI, Br, F, I, R² -(L)ₙ-Y-X-, OR, SR, NR₂, R, NHCOR, CONHR, COOR avec R = alkyle ou aryle.

6. Réactif, selon la revendication 1, de formule (21) : 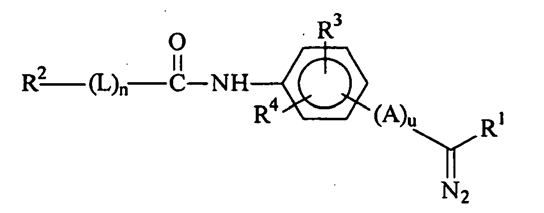 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

7. Réactif, selon la revendication 1, de formule (22) : 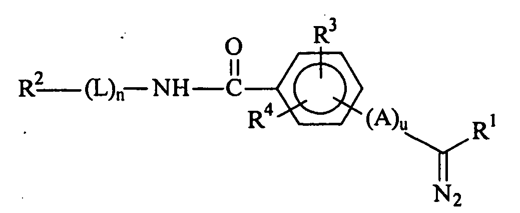 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

8. Réactif, selon la revendication 1, de formule (23) : 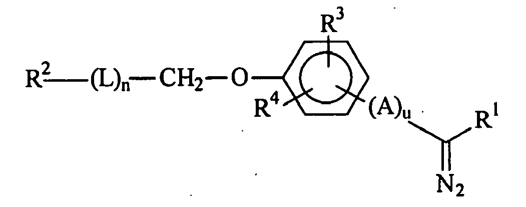 dans laquelle :
• R¹ représente H ou un groupe alkyle, aryle ou aryle substitué,
• R² représente un marqueur détectable ou au moins deux marqueurs détectables reliés entre eux par au moins une structure multinérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

9. Réactif, selon l'une quelconque des revendications 1 à 8, **caractérisé par le fait que** R³ et R⁴ représentent indépendamment l'un de l'autre : H, NO₂, OCH₃, -CO-NH-(CH₂)₃-(O-CH₂-CH₂)₃-CH₂-NH-R², -CO-NH-(CH₂)₃-(O-CH₂-CH₂)₄-CH₂-NH-R².

10. Réactif, selon l'une quelconque des revendications 1 à 3, de formule (2') : 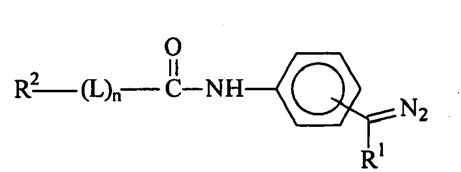 dans laquelle :
• R¹ représente H ou un groupe alkyle, aryle ou aryle substitué,
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

11. Réactif, selon l'une quelconque des revendications 1 ou 6, de formule (24) : 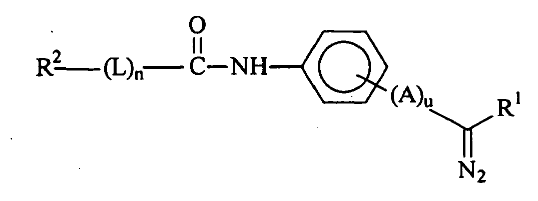 dans laquelle :
• R¹ représente H ou un groupe alkyle, aryle ou aryle substitué,
• R² représente un marqueur détectable ou au moins deux marqueurs détectables reliés entre eux par au moins une structure multimérique, et
• A est un bras de liaison comportant au moins une double liaison covalente permettant la conjugaison de la fonction diazo avec le cycle aromatique et u égal 1.

12. Réactif, selon l'une quelconque des revendications 1 à 11, dans laquelle la structure R²-(L)ₙ- est de formule (5) : 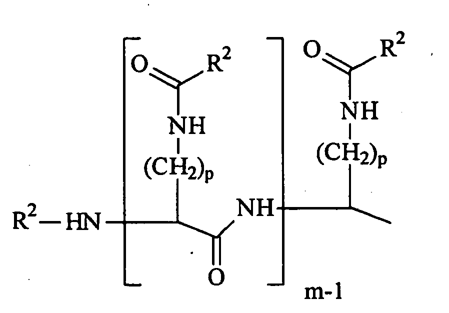 dans laquelle :
• R² représente un marqueur détectable,
• m est un nombre entier compris entre 1 et 100, et
• p est un nombre entier compris entre 1 et 10.

13. Réactif, selon l'une quelconque des revendications 1 à 5, de formule (6) : 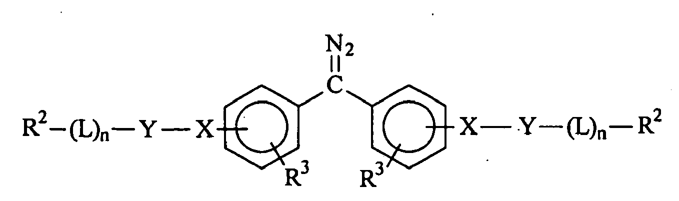 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CONHR, COOR avec R = alkyle ou aryle,
• L est un bras de liaison comportant un enchaînement linéaire d'au moins deux liaisons covalentes et n un nombre entier égal à 0 ou 1, et
• -Y-X- représente -CONH-, -NHCO-, -CH₂O-, -CH₂S-.

14. Réactif, selon l'une quelconque des revendications 1 à 5, de formule (25) : 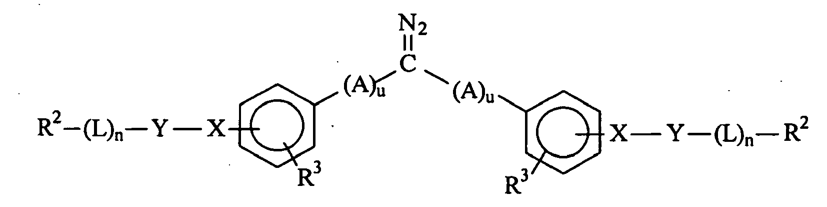 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CONHR, COOR avec R = alkyle ou aryle,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• -Y-X- représente -CONH-, -NHCO-, -CH₂O-, -CH₂S-.

15. Réactif, selon l'une quelconque des revendications 1 à 3, de formule (14) : 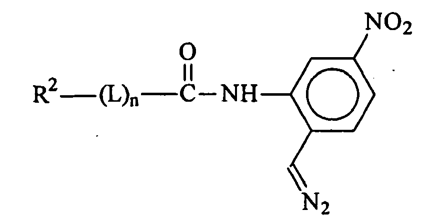 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

16. Réactif, selon l'une quelconque des revendications 1 ou 6, de formule (26) : 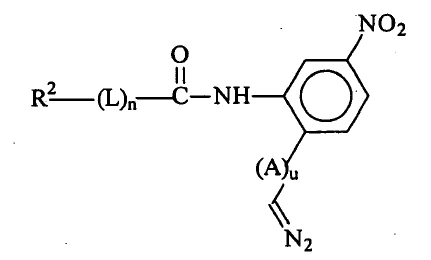 dans laquelle :
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

17. Réactif, selon l'une quelconque des revendications 1 à 3, de formule (15) : 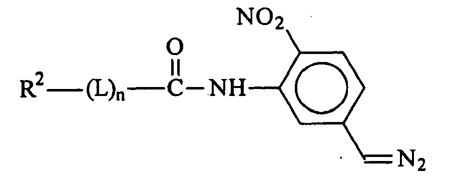 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

18. Réactif, selon l'une quelconque des revendications 1 ou 6, de formule (27) : 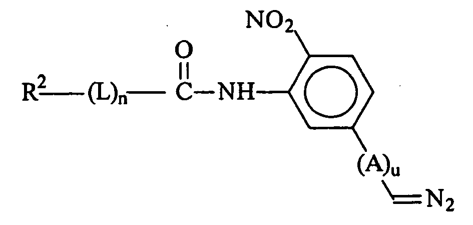 dans laquelle :
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

19. Réactif, selon l'une quelconque des revendications 1 à 18, **caractérisé par le fait que** L comprend un motif -(O-CH₂-CH₂)-, répété de 1 à 20 fois, préférentiellement de 1 à 10 fois, et encore plus préférentiellement de 2 à 5 fois.

20. Procédé de synthèse d'un réactif de marquage, selon l'une quelconque des revendications 1 à 19, comprenant les étapes suivantes :
a) on dispose d'un dérivé de formule (16 bis) : 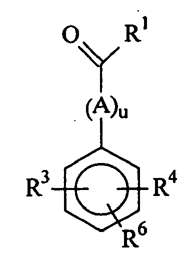 dans laquelle :
• R¹ représente H ou un groupe alkyle ou aryle ou aryle substitué,
• R³ et R⁴ représentent indépendamment l'un de l'autre : H, NO₂, Cl, Br, F, I, R⁶, OR, SR, NR₂, R, NHCOR, CONHR, COOR avec R = alkyle ou aryle,
• R⁶ représente COOH ,COOM, NH₂, OH ou SH avec M = alkyle, en particulier méthyle ou éthyle, et R⁶ étant en position *ortho* ou *méta*, et
• A est un bras de liaison comportant au moins une double liaison covalente permettant la conjugaison de la fonction diazométhyle avec le cycle aromatique et u est un nombre entier égal à 0 ou 1,
b) on dispose d'un marqueur ou d'un précurseur de marqueur possédant une fonction réactive R⁷ complémentaire de R⁶,
c) on fait réagir ensemble la fonction complémentaire dudit marqueur ou précurseur de marqueur avec la fonction R⁶ du dérivé de formule (16 bis) en présence d'au moins un agent de couplage pour former une liaison covalente,
d) on fait réagir l'hydrazine ou un de ses dérivés sur la fonction cétone ou aldéhyde pour former une hydrazone, et
e) on transforme l'hydrazone en fonction diazométhyle à l'aide d'un traitement approprié.

21. Procédé de synthèse d'un réactif de marquage, selon l'une quelconque des revendications 1 à 19, comprenant les étapes suivantes :
a) on dispose d'un dérivé de formule (16 bis) : 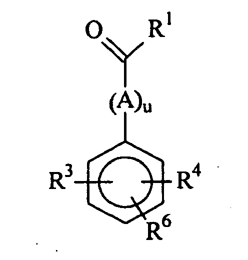 dans laquelle :
• R¹ représente H ou un groupe alkyle ou aryle ou aryle substitué,
• R³ et R⁴ représentent indépendamment l'un de l'autre : H, NO₂, CI, Br, F, I, R⁶, OR, SR, NR₂, R, NHCOR, CONHR, COOR avec R est alkyle ou aryle,
• R⁶ représente COOH ,COOM, NH₂, OH ou SH avec M est alkyle, en particulier méthyle ou éthyle, et R⁶ étant en position *ortho* ou *méta*, et
• A est un bras de liaison comportant au moins une double liaison covalente permettant la conjugaison de la fonction diazométhyle avec le cycle aromatique et u est un nombre entier égal à 0 ou 1,
b) on dispose d'un bras de liaison L possédant au moins deux fonctions réactives R⁸ identiques ou différentes, la première fonction R⁸ complémentaire de R⁶ et la deuxième fonction R⁸ complémentaire de R⁷ et on dispose en outre d'un marqueur ou d'un précurseur de marqueur possédant une fonction réactive R⁷,
c) on fait réagir la première fonction réactive R⁸ du bras de liaison L avec le dérivé de formule (16 bis), en présence d'au moins un agent de couplage, pour former une liaison covalente, puis on fait réagir la deuxième fonction réactive R⁸ du bras de liaison L avec le marqueur ou le précurseur de marqueur, en présence d'au moins un agent de couplage pour former une liaison covalente,
d) on fait réagir l'hydrazine ou un de ses dérivés sur la fonction cétone ou aldéhyde pour former une hydrazone, et
e) on transforme l'hydrazone en fonction diazométhyle à l'aide d'un traitement approprié.

22. Procédé de synthèse, selon l'une quelconque des revendications 20 ou 21, **caractérisé par le fait qu'**il comprend :
• une étape supplémentaire de protection de la fonction cétone ou aldéhyde du composé (16 bis), et
• une étape supplémentaire ultérieure de déprotection de ladite fonction cétone ou aldéhyde

23. Procédé de synthèse, selon la revendication 20, **caractérisé en ce qu'**il consiste à effectuer les étapes suivantes :
a) on dispose d'un dérivé de formule (16) : 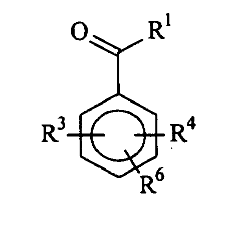 dans laquelle :
• R¹ représente H ou un groupe alkyle ou aryle ou aryle substitué,
• R³ et R⁴ représentent indépendamment l'un de l'autre : H, NO₂, Cl, Br, F, I, R⁶, OR, SR, NR₂, R, NHCOR, CONHR, COOR avec R = alkyle ou aryle, et
• R⁶ représente COOH , NH₂, OH ou SH.
b) on dispose d'un marqueur ou d'un précurseur de marqueur possédant une fonction réactive R⁷ complémentaire de R⁶,
c) on fait réagir ensemble la fonction complémentaire dudit marqueur ou précurseur de marqueur avec la fonction R⁶ du dérivé de formule (16) en présence d'au moins un agent de couplage pour former une liaison covalente,
d) on fait réagir l'hydrazine ou un de ses dérivés sur la fonction cétone ou aldéhyde pour former une hydrazone, et
e) on transforme l'hydrazone en fonction diazométhyle à l'aide d'un traitement approprié.

24. Procédé de synthèse d'un réactif de marquage, selon la revendication 10, comprenant les étapes suivantes :
a) on dispose d'un dérivé de formule (17): 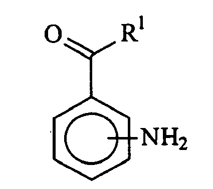 dans laquelle R¹ représente H ou un groupe alkyle ou aryle ou aryle substitué, et le groupe amino est en position *ortho* ou *méta*,
b) on dispose d'un marqueur ou d'un précurseur de marqueur possédant une fonction acide carboxylique,
c) on fait réagir ensemble la fonction carboxylique dudit marqueur ou précurseur de marqueur et la fonction amine primaire du dérivé de formule (17) en présence d'au moins un agent de couplage pour former une liaison amide,
d) on fait réagir de l'hydrazine sur la fonction cétone ou aldéhyde provenant du dérivé de formule (17) pour former une hydrazone, et
e) on oxyde ladite hydrazone en présence de MnO₂ pour former une fonction diazométhyle.

25. Procédé pour le marquage d'une molécule biologique, en particulier un acide nucléique, comprenant la mise en contact en solution homogène, dans un tampon sensiblement aqueux, d'une molécule biologique et d'un réactif, selon l'une quelconque des revendications 1 à 19.

26. Molécule biologique marquée susceptible d'être obtenue par le procédé, selon la revendication 25.

27. Procédé de marquage et de fragmentation d'un acide nucléique simple ou double brin comprenant les étapes suivantes :
- fragmenter l'acide nucléique,
- attacher un marqueur sur au moins un des fragments par l'intermédiaire d'un réactif de marquage choisi parmi les composés de formule (0) selon la revendication 1:
ledit réactif se couplant de manière covalente et majoritaire sur au moins un phosphate dudit fragment.

28. Procédé, selon la revendication 27, **caractérisé par le fait que** le réactif de marquage est choisi parmi les composés de formule (1) selon la revendication 2

29. Procédé, selon l'une quelconque des revendications 27 ou 28, **caractérisé par le fait que** la fragmentation et le marquage sont effectués en deux étapes.

30. Procédé, selon l'une quelconque des revendications 27 ou 28, **caractérisé par le fait que** la fragmentation et le marquage sont effectués en une étape.

31. Procédé, selon l'un quelconque des revendications 27 à 30, **caractérisé par le fait que** le marquage s'effectue en solution homogène sensiblement aqueuse.

32. Procédé, selon l'une quelconque des revendications 27 à 31, **caractérisé par le fait que** la fragmentation s'effectue par voie enzymatique, physique ou chimique.

33. Acide nucléique marqué susceptible d'être obtenu par le procédé, selon l'une quelconque des revendications 27 à 32.

34. Kit de détection d'un acide nucléique cible comprenant un acide nucléique marqué, selon la revendication 33.

35. Support solide sur lequel est fixé un réactif, selon l'une quelconque des revendications 1 à 19.

36. Procédé de capture d'acides nucléiques comprenant les étapes suivantes :
• on dispose d'un support solide sur lequel est fixé directement ou indirectement au moins une molécule de formule (0) selon la revendication 1,
• on met en contact un échantillon biologique susceptible de contenir des acides nucléiques libres, et
• on lave le support solide où la (ou les) molécule(s) sont fixée(s) de manière covalente au moins à un acide nucléique.

## Claims

1. Temperature-stable labeling reagent of formula (0) : 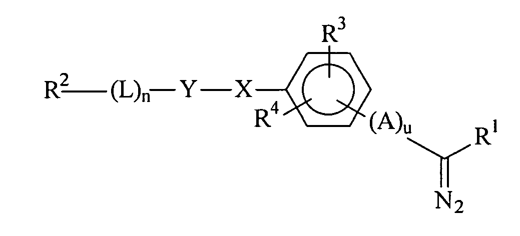 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1,
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl,
• A is a linking arm containing at least one covalent double bond allowing conjugation of the diazo functional group with the aromatic ring and u is an integer between 0 and 2, preferably equal to 0 or 1,
• -Y-X- represents -CONH-, -NHCO-, -CH₂O-, -CH₂S-, and
• the substituent carrying the diazo functional group being in the *ortho* or *meta* position.

2. Labeling reagent according to Claim 1 of formula (1) : 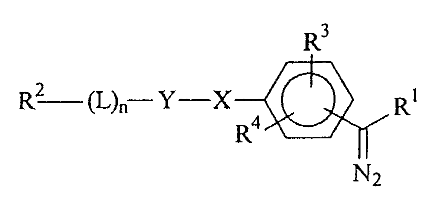 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1,
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl, and
• -Y-X- represents -CONH-, -NHCO-, -CH₂O-, -CH₂S-.

3. Reagent according to either of Claims 1 and 2 of formula (2): 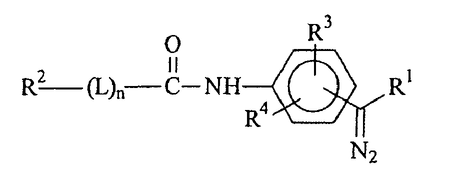 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1, and
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl.

4. Reagent according to either of Claims 1 and 2 of formula (3): 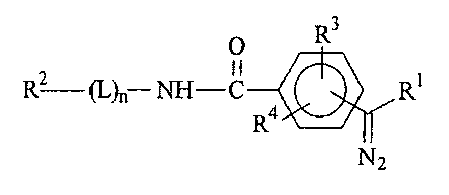 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1, and
• R³ and R⁴ represent independently of each other: H, NO₂ Cl, Br, F, I, R²-(L)ₙ-Y-X-, OR, SR, NR₂, R, NHCOR, CONHR, COOR with R = alkyl or aryl.

5. Reagent according to either of Claims 1 and 2 of formula (4): 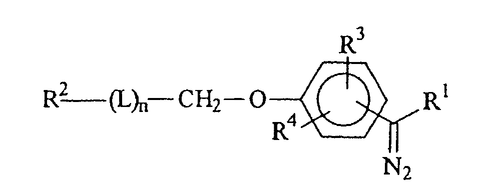 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1, and
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl.

6. Reagent according to Claim 1 of formula (21): 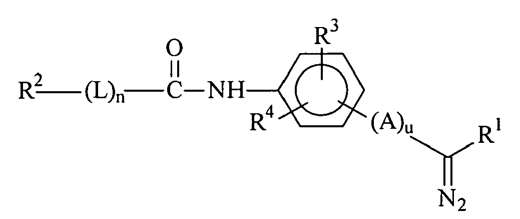 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1,
• A is a linking arm containing at least one covalent double bond allowing conjugation of the diazo functional group with the aromatic ring and u is • equal to 1, and
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl.

7. Reagent according to Claim 1 of formula (22): 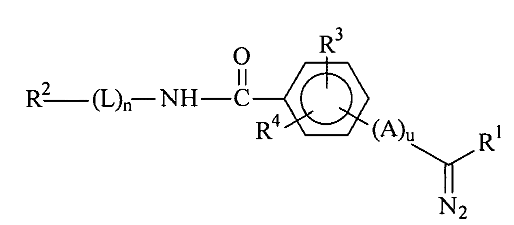 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1,
• A is a linking arm containing at least one covalent double bond allowing conjugation of the diazo functional group with the aromatic ring and u is equal to 1, and
• R³ and R⁴ represent independently of each other: H, NO₂, Cl, Br, F, I, R²-(L)ₙ-Y-X-, OR, SR, NR₂, R, NHCOR, CONHR, COOR with R = alkyl or aryl.

8. Reagent according to Claim 1 of formula (23): 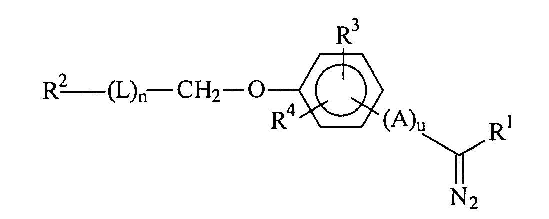 in which:
• R¹ represents H or an alkyl, aryl or substituted aryl group,
• R² represents a detectable marker or at least two detectable markers linked together by at least one multimeric structure,
• L is a linking arm containing a linear succession of at least two covalent bonds and n an integer equal to 0 or 1,
• A is a linking arm containing at least one covalent double bond allowing conjugation of the diazo functional group with the aromatic ring and u is equal to 1, and
• R³ and R⁴ represent independently of each other: H, NO₂ Cl, Br, F, I, R²-(L)ₙ-Y-X-, OR, SR, NR₂, R, NHCOR, CONHR, COOR with R = alkyl or aryl.

9. Reagent according to any one of Claims 1 to 8, **characterized in that** R³ and R⁴ represent independently of each other: H, NO₂ OCH₃, -CO-NH-(CH₂)₃-(O-CH₂-CH₂)₃-CH₂-NH-R², -CO-NH-(CH₂)₃-(O-CH₂-CH₂)₄-CH₂-NH-R².

10. Reagent according to any one of Claims 1 to 3 of formula (2'): 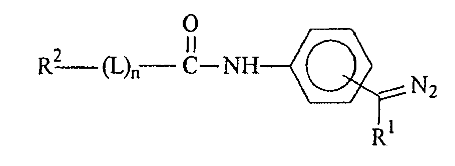 in which:
• R¹ represents H or an alkyl, aryl or substituted aryl group,
• R² represents a detectable marker or at least two detectable markers linked together by at least one multimeric structure, and
• L is a linking arm containing a linear succession of at least two covalent bonds and n an integer equal to 0 or 1.

11. Reagent according to either of Claims 1 and 6 of formula (24): 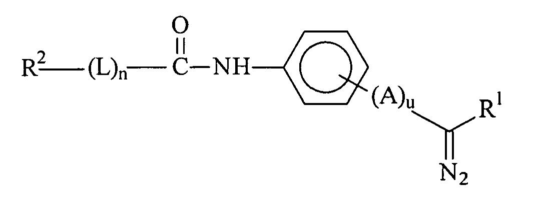 in which:
• R¹ represents H or an alkyl, aryl or substituted aryl group,
• R² represents a detectable marker or at least two detectable markers linked together by at least one multimeric structure, and
• A is a linking arm containing at least one covalent double bond allowing conjugation of the diazo functional group with the aromatic ring and u is equal to 1.

12. Reagent according to any one of Claims 1 to 11, in which the structure R²-(L)ₙ- is of formula (5) : 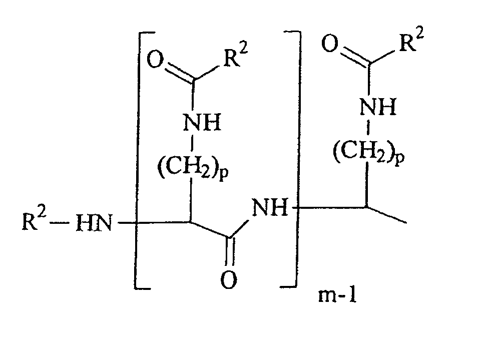 in which:
• R² represents a detectable marker,
• m is an integer between 1 and 100, and
• p is an integer between 1 and 10.

13. Reagent according to any one of Claims 1 to 5 of formula (6): 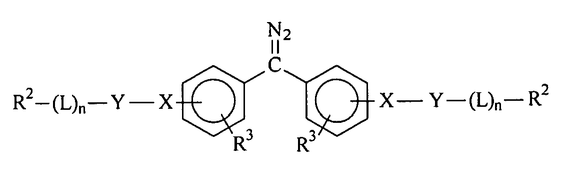 in which:
• R² represents a detectable marker or at least two detectable markers linked together by at least one multimeric structure,
• R³ represents H, NO₂, Cl, Br, F, I, R²-(L)ₙ-Y:-X-, OR, SR, NR₂, R, NHCOR, CONHR, COOR with R = alkyl or aryl,
• L is a linking arm containing a linear succession of at least two covalent bonds and n an integer equal to 0 or 1, and
• -Y-X- represents -CONH-, -NHCO-, -CH₂O-, -CH₂S-.

14. Reagent according to any one of Claims 1 to 5 of formula (25): 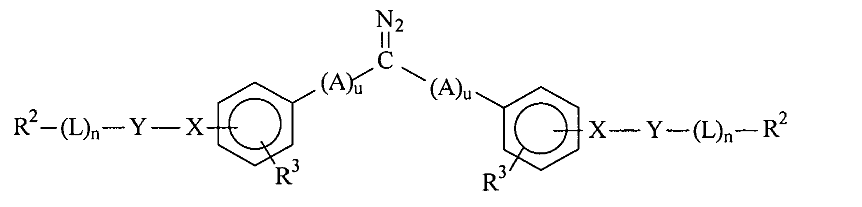 in which:
• R² represents a detectable marker or at least two detectable markers linked together by at least one multimeric structure,
• R³ represents H, NO₂, Cl, Br, F, I, R²-(L)ₙ-Y-X-, OR, SR, NR₂, R, NHCOR, CONHR, COOR with R = alkyl or aryl,
• L is a linking arm containing a linear succession of at least two covalent bonds and n an integer equal to 0 or 1,
• A is a linking arm containing at least one covalent double bond allowing conjugation of the diazo functional group with the aromatic ring and u is equal to 1, and
• -Y-X- represents -CONH-, -NHCO-, -CH₂O-, -CH₂S-.

15. Reagent according to any one of Claims 1 to 3 of formula (14): 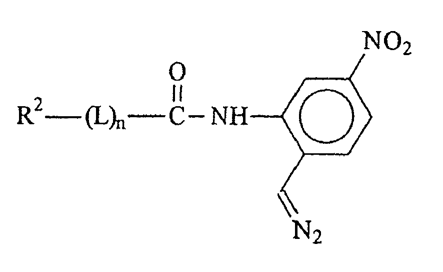 in which:
• R² represents a detectable marker or at least two detectable markers linked together by at least one multimeric structure, and
• L is a linking arm containing a linear succession of at least two covalent bonds and n an integer equal to 0 or 1.

16. Reagent according to either of Claims 1 and 6 of formula (26): 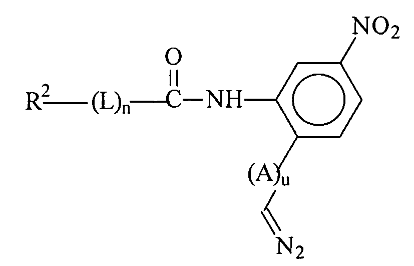 in which:
• R² represents a detectable marker or at least two detectable markers linked together by at least one multimeric structure,
• A is a linking arm containing at least one covalent double bond allowing conjugation of the diazo functional group with the aromatic ring and u is equal to 1, and
• L is a linking arm containing a linear succession of at least two covalent bonds and n an integer equal to 0 or 1.

17. Reagent according to any one of Claims 1 to 3 of formula (15): 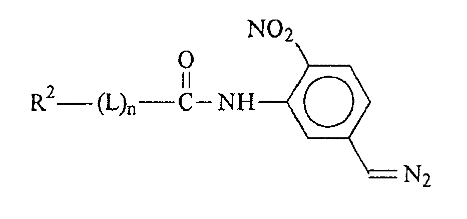 in which:
• R² represents a detectable marker or at least two detectable markers linked together by at least one multimeric structure,
• L is a linking arm containing a linear succession of at least two covalent bonds and n an integer equal to 0 or 1.

18. Reagent according to either of Claims 1 and 6 of formula (27) : 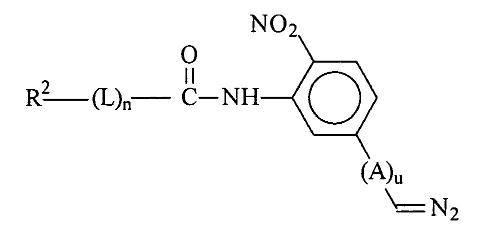 in which:
• R² represents a detectable marker or at least two detectable markers linked together by at least one multimeric structure,
• A is a linking arm containing at least one covalent double bond allowing conjugation of the diazo functional group with the aromatic ring and u is equal to 1, and
• L is a linking arm containing a linear succession of at least two covalent bonds and n an integer equal to 0 or 1.

19. Reagent according to any one of Claims 1 to 18, **characterized in that** L comprises a unit -(O-CH₂-CH₂)-, repeated from 1 to 20 times, preferably from 1 to 10 times, and still more preferably from 2 to 5 times.

20. Process for synthesizing a labeling reagent, according to any one of Claims 1 to 19, comprising the following steps:
a) there is made available a derivative of formula (16a) : 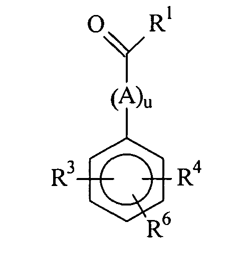 in which:
• R¹ represents H or an alkyl or aryl or substituted aryl group,
• R³ and R⁴ represent independently of each other: H, NO₂, Cl, Br, F, I, R⁶, OR, SR, NR₂, R, NHCOR, CONHR, COOR with R = alkyl or aryl,
• R⁶ represents COOH, COOM, NH₂, OH or SH with M = alkyl, in particular methyl or ethyl, and R⁶ being in the *ortho* or *meta* position, and
• A is a linking arm containing at least one covalent double bond allowing conjugation of the diazomethyl functional group with the aromatic ring and u is an integer equal to 0 or 1,
b) there is made available a marker or a marker precursor possessing a reactive functional group R⁷ complementary to R⁶,
c) the complementary functional group of said marker or marker precursor is reacted with the functional group R⁶ of the derivative of formula (16a) in the presence of at least one coupling agent to form a covalent bond,
d) hydrazine or one of its derivatives is reacted with the ketone or aldehyde functional group to form a hydrazone, and
e) the hydrazone is converted to a diazomethyl functional group using an appropriate treatment.

21. Process for synthesizing a labeling reagent, according to any one of Claims 1 to 19, comprising the following steps:
a) there is made available a derivative of formula (16a) : 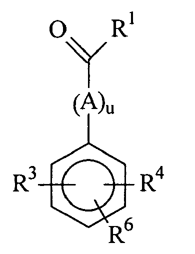 in which:
• R¹ represents H or an alkyl or aryl or substituted aryl group,
• R³ and R⁴ represent independently of each other: H, NO₂, Cl, Br, F, I, R⁶, OR, SR, NR₂, R, NHCOR, CONHR, COOR with R = alkyl or aryl,
• R⁶ represents COOH, COOM, NH₂, OH or SH with M = alkyl, in particular methyl or ethyl, and R⁶ being in the *ortho* or *meta* position, and
• A is a linking arm containing at least one covalent double bond allowing conjugation of the diazomethyl functional group with the aromatic ring and u is an integer equal to 0 or 1,
b) there is made available a linking arm L possessing at least two reactive functional groups R⁸ which are identical or different, the first functional group R⁸ complementary to R⁶ and the second functional group R⁸ complementary to R⁷, and there is made available, in addition, a marker or a marker precursor possessing a reactive functional group R⁷,
c) the first reactive functional group R⁸ of the linking arm L is reacted with the derivative of formula (16a) in the presence of at least one coupling agent to form a covalent bond and then the second reactive functional group R⁸ of the linking arm L is reacted with the marker or the marker precursor in the presence of at least one coupling agent to form a covalent bond,
d) hydrazine or one of its derivatives is reacted with the ketone or aldehyde functional group to form a hydrazone, and
e) the hydrazone is converted to a diazomethyl functional group using an appropriate treatment.

22. Process according to either of Claims 20 and 21, **characterized in that** it comprises:
• an additional step of protecting the ketone or aldehyde functional group of compound (16a), and
• an additional subsequent step of deprotecting said ketone or aldehyde functional group.

23. Process according to Claim 20, **characterized in that** it consists in carrying out the following steps:
a) there is made available a derivative of formula (16) : 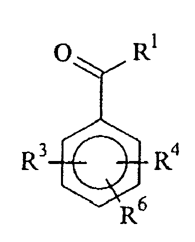 in which:
• R¹ represents H or an alkyl or aryl or substituted aryl group,
• R³ and R⁴ represent independently of each other: H, NO₂, Cl, Br, F, I, R⁶, OR, SR, NR₂, R, NHCOR, CONHR, COOR with R = alkyl or aryl, and
• R⁶ represents COOH, NH₂, OH or SH.
b) there is made available a marker or a marker precursor possessing a reactive functional group R⁷ complementary to R⁶,
c) the complementary functional group of said marker or marker precursor is reacted with the R⁶ functional group of the derivative of formula (16) in the presence of at least one coupling agent to form a covalent bond,
d) hydrazine or one of its derivatives is reacted with the ketone or aldehyde functional group to form a hydrazone, and
e) the hydrazone is converted to a diazomethyl functional group by means of an appropriate treatment.

24. Process for synthesizing a labeling reagent according to Claim 10, comprising the following steps:
a) there is made available a derivative of formula (17): 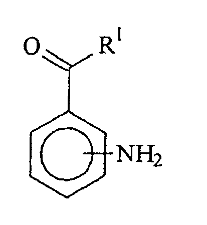 in which R¹ represents H or an alkyl or aryl or substituted aryl group, and the amino group being in the *ortho* or *meta* position,
b) there is made available a marker or a marker precursor possessing a carboxylic acid functional group,
c) the carboxylic functional group of said marker or marker precursor and the primary amine functional group of the derivative of formula (17) are reacted together in the presence of at least one coupling agent to form an amide bond,
d) hydrazine is reacted with the ketone or aldehyde functional group from the derivative of formula (17) to form a hydrazone, and
e) said hydrazone is oxidized in the presence of MnO₂ to form a diazomethyl functional group.

25. Method for labeling a biological molecule, in particular a nucleic acid, comprising bringing a biological molecule and a reagent according to any one of Claims 1 to 19 into contact in a homogeneous solution, in a substantially aqueous buffer.

26. Labeled biological molecule capable of being obtained by the method according to Claim 25.

27. Method for labeling and fragmenting a single- or double-stranded nucleic acid, comprising the following steps in any order:
- fragmenting the nucleic acid,
- attaching a marker to at least one of the fragments via a labeling reagent chosen from the compounds of formula (0)according to Claim 1;
said reagent being covalently and predominantly coupled to at least one phosphate of said fragment.

28. Method according to Claim 27, **characterized in that** the labeling reagent is chosen from the compounds of formula (1) according to Claim 2.

29. Method according to either of Claims 27 and 28, **characterized in that** the fragmentation and the labeling are carried out in two steps.

30. Method according to either of Claims 27 and 28, **characterized in that** the fragmentation and the labeling are carried out in one step.

31. Method according to any one of Claims 27 to 30, **characterized in that** the labeling is carried out in a substantially aqueous homogeneous solution.

32. Method according to any one of Claims 27 to 31, **characterized in that** the fragmentation is carried out by the enzymatic, physical or chemical route.

33. Labeled nucleic acid capable of being obtained by the method according to any one of Claims 27 to 32.

34. Kit for detecting a target nucleic acid comprising a labeled nucleic acid according to Claim 33.

35. Solid support onto which a reagent according to any one of Claims 1 to 19 is attached.

36. Method for capturing nucleic acids comprising the following steps:
• a solid support is made available to which at least one molecule of formula (0) according to Claim 1 is directly or indirectly attached,
• a biological sample likely to contain free nucleic acids is brought into contact, and
• the solid support where the molecule(s) is (are) covalently attached at least to one nucleic acid is washed.

## Patentansprüche

1. Temperaturbeständiges Markierungsreagens mit der Formel (0): 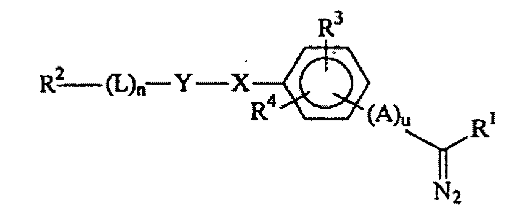 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist,
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen,
• A eine Brückengruppe ist, die wenigstens eine kovalente Doppelbindung aufweist, die die Konjugation zwischen der Diazofunktion und dem aromatischen Ring zulässt, und u eine ganze Zahl von 0 bis 2, bevorzugt 0 oder 1, ist,
• -Y-X- -CONH-, NHCO-, -CH₂O-, -CH₂S- darstellt und
• der Substituent die Diazofunktion in *ortho*- oder *meta*-Position trägt.

2. Markierungsreagens nach Anspruch 1 mit der Formel (1): 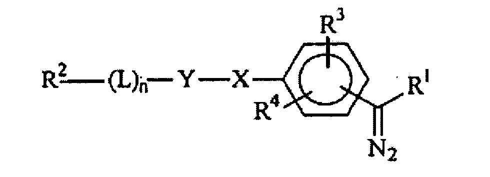 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist,
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen und
• -Y-X- -CONH-, -NHCO-, -CH₂O-, -CH₂S- darstellt.

3. Reagens nach Anspruch 1 oder 2 mit der Formel (2) : 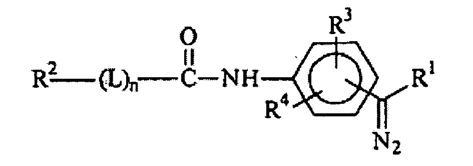 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

4. Reagens nach Anspruch 1 oder 2 mit der Formel (3) : 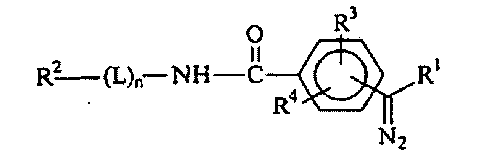 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

5. Reagens nach Anspruch 1 oder 2 mit der Formel (4) : 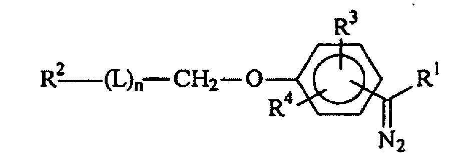 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, • darstellt,
• L eine Brückengruppe ist, die eine lineare Kette aus wenigstens zwei kovalenten Bindungen aufweist, und n eine ganze Zahl von gleich 0 oder 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

6. Reagens nach Anspruch 1 mit der Formel (21): 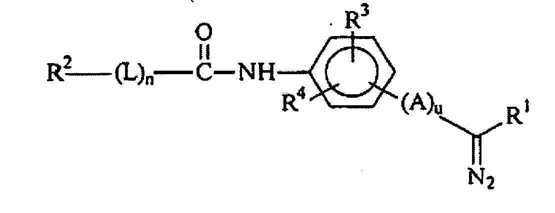 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist,
• A eine Brückengruppe ist, die wenigstens eine kovalente Doppelbindung aufweist, die die Konjugation zwischen der Diazofunktion und dem aromatischen Ring zulässt, und u gleich 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

7. Reagens nach Anspruch 1 mit der Formel (22): 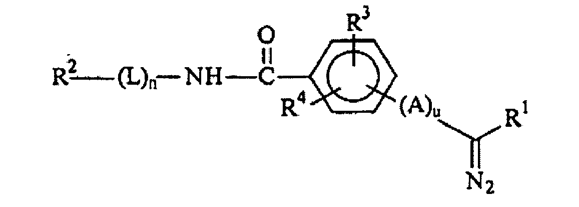 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist,
• A eine Brückengruppe ist, die wenigstens eine kovalente Doppelbindung aufweist, die die Konjugation zwischen der Diazofunktion und dem aromatischen Ring zulässt, und u gleich 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

8. Reagens nach Anspruch 1 mit der Formel (23): 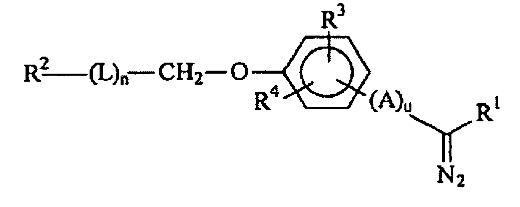 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt,
• L eine Brückengruppe ist, die eine lineare Kette aus wenigstens zwei kovalenten Bindungen aufweist, und n eine ganze Zahl von gleich 0 oder 1 ist,
• A eine Brückengruppe ist, die wenigstens eine kovalente Doppelbindung aufweist, die die Konjugation zwischen der Diazofunktion und dem aromatischen Ring zulässt, und u gleich 1 ist und
• R³ und R⁴ unabhängig voneinander: H, NO₂, Cl, Br, F, I, R²-(L)ₙ-Y-X-, OR, SR, NR₂, R, NHCOR, CONHR, COOR mit R = Alkyl oder Aryl darstellen.

9. Reagens nach einem der Ansprüche 1 bis 8, **dadurch gekennzeichnet, dass** R³ und R⁴ unabhängig voneinander: H, NO₂, OCH₃, -CO-NH-(CH₂)₃-(O-CH₂-CH₂)₃-CH₂-NH-R², -CO-NH-(CH₂)₃-(O-CH₂-CH₂)₄-CH₂-NH-R² darstellen.

10. Reagens nach einem der Ansprüche 1 bis 3 mit der Formel (2'): 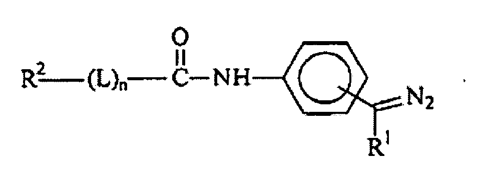 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt und
• L eine Brückengruppe ist, die eine lineare Kette aus wenigstens zwei kovalenten Bindungen aufweist, und n eine ganze Zahl von gleich 0 oder 1 ist.

11. Reagens nach einem der Ansprüche 1 oder 6 mit der Formel (24): 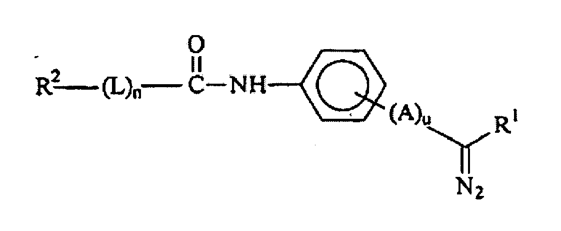 in der:
• R¹ H oder eine Alkyl-, eine Aryl- oder eine substituierte Arylgruppe darstellt,
• R² einen nachweisbaren Marker oder wenigstens zwei nachweisbare Marker, die durch wenigstens eine multimere Struktur miteinander verbunden sind, darstellt und
• A eine Brückengruppe ist, die wenigstens eine kovalente Doppelbindung aufweist, die die Konjugation zwischen der Diazofunktion und dem aromatischen Ring zulässt, und u gleich 1 ist.

12. Reagens nach einem der Ansprüche 1 bis 11, in der die Struktur R²-(L)ₙ- die Formel (5): 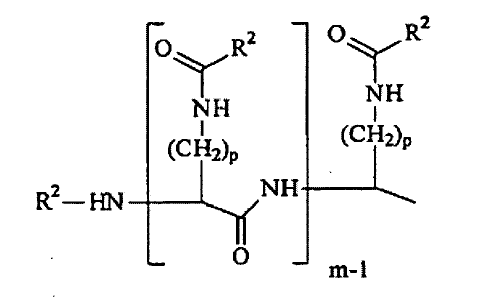 aufweist, in der:
• R² einen nachweisbaren Marker darstellt,
• m eine ganze Zahl von 1 bis 100 ist und
• p eine ganze Zahl von 1 bis 10 ist.

13. Reagens nach einem der Ansprüche 1 bis 5 mit der Formel (6): 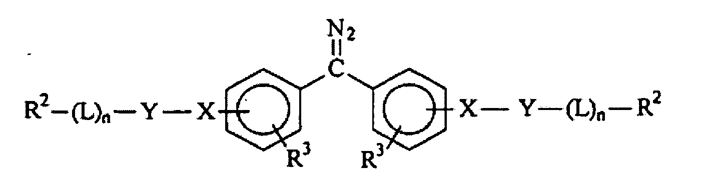 in der:
• R² einen nachweisbaren Marker oder wenigstens zwei nachweisbare Marker, die durch wenigstens eine multimere Struktur miteinander verbunden sind, darstellt,
• R³ H, NO₂, Cl, Br, F, I, R²-(L)ₙ-Y-X-, OR, SR, NR₂, R, NHCOR, CONHR, COOR mit R = Alkyl oder Aryl darstellt,
• L eine Brückengruppe ist, die eine lineare Kette aus wenigstens zwei kovalenten Bindungen aufweist, und n eine ganze Zahl von gleich 0 oder 1 ist und
• -Y-X- -CONH-, -NHCO-, -CH₂O-, -CH₂S- darstellt.

14. Reagens nach einem der Ansprüche 1 bis 5 mit der Formel (25): 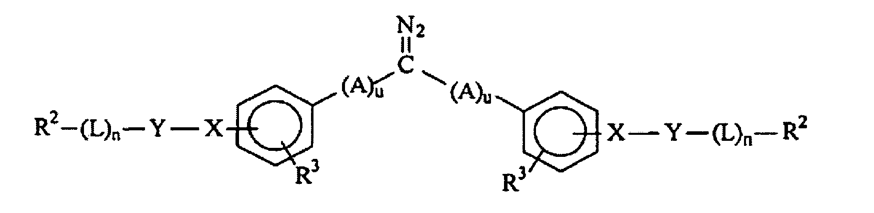 in der:
• R² einen nachweisbaren Marker oder wenigstens zwei nachweisbare Marker, die durch wenigstens eine multimere Struktur miteinander verbunden sind, darstellt,
• R³ H, NO₂, Cl, Br, F, I, R²-(L)ₙ-Y-X-, OR, SR, NR₂, R, NHCOR, CONHR, COOR mit R = Alkyl oder Aryl darstellt,
• L eine Brückengruppe ist, die eine lineare Kette aus wenigstens zwei kovalenten Bindungen aufweist, und n eine ganze Zahl von gleich 0 oder 1 ist,
• A eine Brückengruppe ist, die wenigstens eine kovalente Doppelbindung aufweist, die die Konjugation zwischen der Diazofunktion und dem aromatischen Ring zulässt, und u gleich 1 ist und
• -Y-X- -CONH-, -NHCO-, -CH₂O-, -CH₂S- darstellt.

15. Reagens nach einem der Ansprüche 1 bis 3 mit der Formel (14): 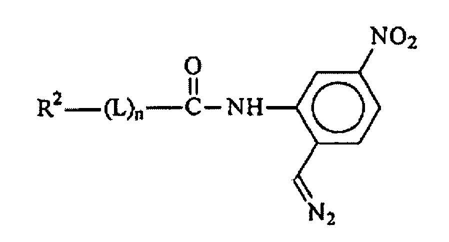 in der:
• R² einen nachweisbaren Marker oder wenigstens zwei nachweisbare Marker, die durch wenigstens eine multimere Struktur miteinander verbunden sind, darstellt und
• L eine Brückengruppe ist, die eine lineare Kette aus wenigstens zwei kovalenten Bindungen aufweist, und n eine ganze Zahl von gleich 0 oder 1 ist.

16. Reagens nach einem der Ansprüche 1 oder 6 mit der Formel (26): 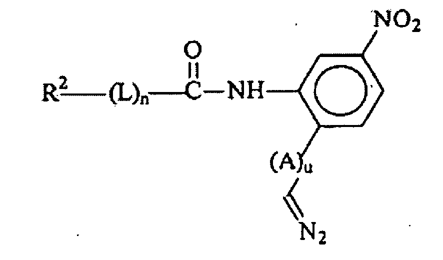 in der:
• R² einen nachweisbaren Marker oder wenigstens zwei nachweisbare Marker, die durch wenigstens eine multimere Struktur miteinander verbunden sind, darstellt,
• A eine Brückengruppe ist, die wenigstens eine kovalente Doppelbindung aufweist, die die Konjugation zwischen der Diazofunktion und dem aromatischen Ring zulässt, und u gleich 1 ist und
• L eine Brückengruppe ist, die eine lineare Kette aus wenigstens zwei kovalenten Bindungen aufweist, und n eine ganze Zahl von gleich 0 oder 1 ist.

17. Reagens nach einem der Ansprüche 1 bis 3 mit der Formel (15): 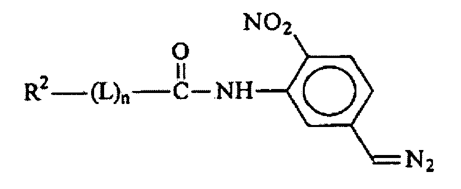 in der:
• R² einen nachweisbaren Marker oder wenigstens zwei nachweisbare Marker, die durch wenigstens eine multimere Struktur miteinander verbunden sind, darstellt und
• L eine Brückengruppe ist, die eine lineare Kette aus wenigstens zwei kovalenten Bindungen aufweist, und n eine ganze Zahl von gleich 0 oder 1 ist.

18. Reagens nach einem der Ansprüche 1 oder 6 mit der Formel (27): 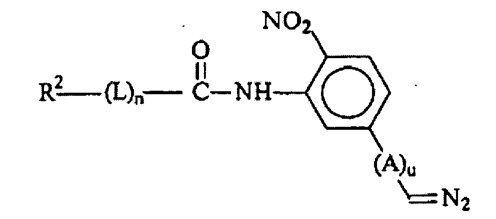 in der:
• R² einen nachweisbaren Marker oder wenigstens zwei nachweisbare Marker, die durch wenigstens eine multimere Struktur miteinander verbunden sind, darstellt,
• A eine Brückengruppe ist, die wenigstens eine kovalente Doppelbindung aufweist, die die Konjugation zwischen der Diazofunktion und dem aromatischen Ring zulässt, und u gleich 1 ist und
• L eine Brückengruppe ist, die eine lineare Kette aus wenigstens zwei kovalenten Bindungen aufweist, und n eine ganze Zahl von gleich 0 oder 1 ist.

19. Reagens nach einem der Ansprüche 1 bis 18, **dadurch gekennzeichnet, dass** L eine -(O-CH₂-CH₂)-Einheit umfasst, die 1- bis 20-mal, bevorzugt 1- bis 10-mal und besonders bevorzugt 2- bis 5-mal, wiederholt wird.

20. Verfahren zur Synthese eines Markierungsreagens gemäß einem der Ansprüche 1 bis 19, umfassend die folgenden Schritte:
a) Bereitstellen eines Derivats mit der Formel (16a): 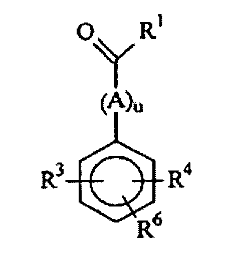 in der:
• R¹ H oder eine Alkyl- oder eine Aryl- oder eine substituierte Arylgruppe darstellt,
• R³ und R⁴ unabhängig voneinander: H, NO₂, Cl, Br, F, I, R⁶, OR, SR, NR₂, R, NHCOR, CONHR, COOR mit R = Alkyl oder Aryl darstellen,
• R⁶ COOH, COOM, NH₂, OH oder SH mit M = Alkyl, insbesondere Methyl oder Ethyl, darstellt und sich R⁶ in der *ortho*- oder *meta*-Position befindet und
• A eine Brückengruppe ist, die wenigstens eine kovalente Doppelbindung aufweist, die die Konjugation zwischen der Diazomethylfunktion und dem aromatischen Ring zulässt, und u eine ganze Zahl von gleich 0 oder 1 ist,
b) Bereitstellen eines Markers oder eines Vorläufers des Markers, der eine zu R⁶ komplementäre reaktive Funktion R⁷ besitzt,
c) Umsetzen der komplementären Funktion des Markers oder des Vorläufers des Markers mit der Funktion R⁶ des Derivats mit der Formel (16a) in Gegenwart wenigstens eines Kopplungsmittels, um eine kovalente Bindung zu bilden,
d) Umsetzen von Hydrazin oder eines seiner Derivate an der Keton- oder Aldehydfunktion, um ein Hydrazon zu bilden, und
e) Umwandeln des Hydrazons in eine Diazomethylfunktion mittels einer geeigneten Behandlung.

21. Verfahren zur Synthese eines Markierungsreagens gemäß einem der Ansprüche 1 bis 19, umfassend die folgenden Schritte:
a) Bereitstellen eines Derivats mit der Formel (16a): 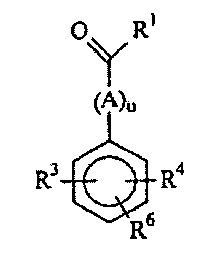 in der:
• R¹ H oder eine Alkyl- oder eine Aryl- oder eine substituierte Arylgruppe darstellt,
• R³ und R⁴ unabhängig voneinander: H, NO₂, Cl, Br, F, I, R⁶, OR, SR, NR₂, R, NHCOR, CONHR, COOR mit R = Alkyl oder Aryl darstellen,
• R⁶ COOH, COOM, NH₂, OH oder SH mit M = Alkyl, insbesondere Methyl oder Ethyl, darstellt und sich R⁶ in der *ortho*- oder *meta*-Position befindet und
• A eine Brückengruppe ist, die wenigstens eine kovalente Doppelbindung aufweist, die die Konjugation zwischen der Diazomethylfunktion und dem aromatischen Ring zulässt, und u eine ganze Zahl von gleich 0 oder 1 ist,
b) Bereitstellen einer verzweigten Verbindung L, die wenigstens zwei identische oder verschiedene reaktive Funktionen R⁸ besitzt, wobei die erste Funktion R⁸ zu R⁶ komplementär ist und die zweite Funktion R⁸ zu R⁷ komplementär ist, und zudem Bereitstellen eines Markers oder eines Vorläufers des Markers, der eine reaktive Funktion R⁷ besitzt,
c) Umsetzen der ersten reaktiven Funktion R⁸ der verzweigten Verbindung L mit dem Derivat mit der Formel (16a) in Gegenwart wenigstens eines Kopplungsmittels, um eine kovalente Bindung zu bilden, anschließend Umsetzen der zweiten reaktiven Funktion R⁸ der verzweigten Verbindung L mit dem Marker oder dem Vorläufer des Markers in Gegenwart wenigstens eines Kopplungsmittels, um eine kovalente Bindung zu bilden,
d) Umsetzen von Hydrazin oder eines seiner Derivate an der Keton- oder Aldehydfunktion, um ein Hydrazon auszubilden, und
e) Umwandeln des Hydrazons in eine Diazomethylfunktion mittels einer geeigneten Behandlung.

22. Syntheseverfahren nach einem der Ansprüche 20 oder 21, **dadurch gekennzeichnet, dass** es umfasst:
• einen zusätzlichen Schritt des Hinzufügens einer Schutzgruppe für die Keton- oder Aldehydfunktion der Verbindung (16a) und
• einen zusätzlichen späteren Schritt des Entfernens der Schutzgruppe an der Keton- oder Aldehydfunktion.

23. Syntheseverfahren nach Anspruch 20, **dadurch gekennzeichnet, dass** es aus dem Durchführen der folgenden Schritte besteht:
a) Bereitstellen eines Derivats mit der Formel (16): 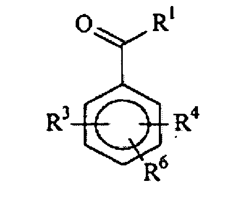 in der:
• R¹ H oder eine Alkyl- oder eine Aryl- oder eine substituierte Arylgruppe darstellt,
• R³ und R⁴ unabhängig voneinander: H, NO₂, Cl, Br, F, I, R⁶, OR, SR, NR₂, R, NHCOR, CONHR, COOR mit R = Alkyl oder Aryl darstellen und
• R⁶ COOH, NH₂, OH oder SH darstellt,
b) Bereitstellen eines Markers oder eines Vorläufers des Markers, der eine zu R⁶ komplementäre reaktive Funktion R⁷ besitzt,
c) Umsetzen der komplementären Funktion des Markers oder des Vorläufers des Markers mit der Funktion R⁶ des Derivats mit der Formel (16) in Gegenwart wenigstens eines Kopplungsmittels, um eine kovalente Bindung zu bilden,
d) Umsetzen von Hydrazin oder eines seiner Derivate an der Keton- oder Aldehydfunktion, um ein Hydrazon zu bilden, und
e) Umwandeln des Hydrazons in eine Diazomethylfunktion mittels einer geeigneten Behandlung.

24. Verfahren zur Synthese eines Markierungsreagens nach Anspruch 10, umfassend die folgenden Schritte:
a) Bereitstellen eines Derivats mit der Formel (17): 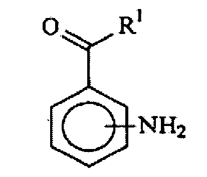 in der R¹ H oder eine Alkyl- oder eine Aryl- oder eine substituierte Arylgruppe darstellt und sich die Aminogruppe in der *ortho*- oder *meta*-Position befindet,
b) Bereitstellen eines Markers oder eines Vorläufers des Markers, der eine Carbonsäurefunktion besitzt,
c) Umsetzen der Carbonsäurefunktion des Markers oder des Vorläufers des Markers mit der primären Aminfunktion des Derivats mit der Formel (17) in Gegenwart wenigstens eines Kopplungsmittels, um eine Amidbindung zu bilden,
d) Umsetzen von Hydrazin an der aus dem Derivat mit der Formel (17) stammenden Keton- oder Aldehydfunktion, um ein Hydrazon zu bilden, und
e) Oxidieren des Hydrazons in Gegenwart von MnO₂, um eine Diazomethylfunktion zu bilden.

25. Verfahren zum Markieren eines biologischen Moleküls, insbesondere einer Nukleinsäure, umfassend das In-Kontakt-Bringen eines biologischen Moleküls und eines Reagens gemäß einem der Ansprüche 1 bis 19 in einer homogenen Lösung in einem weitgehend wässrigen Puffer.

26. Markiertes biologisches Molekül, das mit dem Verfahren gemäß Anspruch 25 erhältlich ist.

27. Verfahren zum Markieren und Fragmentieren einer einzelsträngigen oder doppelsträngigen Nukleinsäure, umfassend die folgenden Schritte:
- Fragmentieren der Nukleinsäure,
- Anbinden eines Markers an wenigstens eines der Fragmente über ein Markierungsreagens, das ausgewählt ist aus den Verbindungen mit der Formel (0) gemäß Anspruch 1,
wobei das Reagens kovalent und vorwiegend über wenigstens ein Phosphat des Fragments gekoppelt wird.

28. Verfahren nach Anspruch 27, **dadurch gekennzeichnet, dass** das Markierungsreagens ausgewählt ist aus den Verbindungen mit der Formel (1) gemäß Anspruch 2.

29. Verfahren nach einem der Ansprüche 27 oder 28, **dadurch gekennzeichnet, dass** die Fragmentierung und die Markierung in zwei Schritten durchgeführt werden.

30. Verfahren nach einem der Ansprüche 27 oder 28, **dadurch gekennzeichnet, dass** die Fragmentierung und die Markierung in einem Schritt durchgeführt werden.

31. Verfahren nach einem der Ansprüche 27 bis 30, **dadurch gekennzeichnet, dass** die Markierung in einer weitgehend wässrigen, homogenen Lösung durchgeführt wird.

32. Verfahren nach einem der Ansprüche 27 bis 31, **dadurch gekennzeichnet, dass** die Fragmentierung enzymatisch, physikalisch oder chemisch durchgeführt wird.

33. Markierte Nukleinsäure, die mit dem Verfahren gemäß einem der Ansprüche 27 bis 32 erhältlich ist.

34. Kit zum Nachweisen einer Ziel-Nukleinsäure, umfassend eine markierte Nukleinsäure gemäß Anspruch 33.

35. Fester Träger, auf welchem ein Reagens gemäß einem der Ansprüche 1 bis 19 fixiert ist.

36. Verfahren zum Abfangen von Nukleinsäuren, umfassend die folgenden Schritte:
• Bereitstellen eines festen Trägers, auf dem direkt oder indirekt wenigstens ein Molekül mit der Formel (0) gemäß Anspruch 1 fixiert ist,
• In-Kontakt-Bringen einer biologischen Probe, die wahrscheinlich freie Nukleinsäuren enthält, und
• Waschen des festen Trägers, an dem das (oder die) Molekül(e) kovalent an wenigstens einer Nukleinsäure fixiert ist (sind).
